# EUROPEAN PATENT APPLICATION

(11) **EP 2 524 912 A1**
(43) Date of publication of application: **21.11.2012**
(21) Application number: 11166243.3
(22) Date of filing: 16.05.2011
(51) Int. Cl.: C07D 211/38, C07D 213/30, C07D 213/40, C07D 241/18, C07D 401/12, C07D 405/12, C07D 411/12, C07D 413/12, A61K 31/4402, A61P 37/00

(54) **Amine derivatives**

(71) Applicant: Bionomics Limited, Thebarton, S.A. 5031 (AU); Merck Patent GmbH, 64293 Darmstadt (DE)
(72) Inventor: Harvey, Andrew, Goodwood, South Australia 5034 (AU); Bombrun, Agnes, 1292, Chambesy (CH); Cooke, Rachel, Woodville West, South Australia 5011 (AU); Jeanclaude-Etter, Isabelle, 1293, Bellevue (CH); Kuchel, Nathan, Clarence Park, South Australia 5034 (AU); Molette, Jerome, 01210, Versonnex (FR); Mould, Jorgen, Semaphore South, South Australia 5019 (AU); Paul, Dharam, Flinders Park, South Australia 5025 (AU); Singh, Rajinder, Torrensville, South Australia 5031 (AU)
(74) Representative: Kling, Simone

(57) **Abstract**

The present invention relates to Kv1.3 potassium channel blockers of Formula (I) and their use in the treatment of autoimmune and inflammatory diseases.

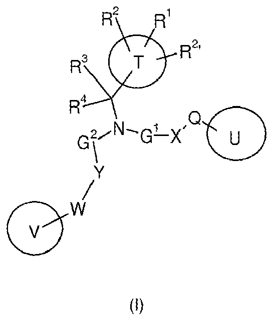

## Description

The present invention relates to compounds of Formula (I) as Kv1.3 channel blockers.

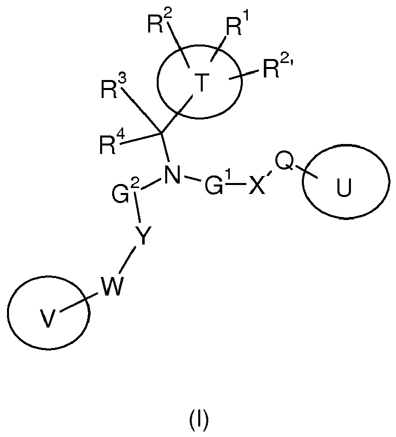

Wherein
- G¹: denotes a CO group or a single bond, preferably a single bond,
- G²: denotes a CO group, a CH₂ group, or a single bond, preferably a CO group,
- X,: Y are independently from one another selected from a single bond, a linear or branched alkylene group having 1 to 6 carbon atoms, or a saturated or unsaturated 3-8-membered cycloalkylene group,
- Q,: W are independently from one another selected from O, NH, SO, SO₂, CO, or a single bond,
- U: is a 3-8-membered saturated or unsaturated cycloalkyl group, a 3-8-membered saturated or unsaturated heterocyclic group, a 7-12 bridged or fused bicyclic carbocyclic or heterocyclic ring or a 5-6-membered heteroaromatic group, each of the above group being optionally substituted with 1 to 3 substitutents selected from Hal, NO₂ CN, SO₂, NMe₂, linear or branched alkyl group having 1 to 6 carbon atoms, O-C₁-C₆-alkyl, -(CH₂)ₘ-O-C₁-C₆-alkyl, CF₃, or a 5-6-membered heteroaromatic group
- V: is an aryl group optionally substituted with 1 to 3 substitutents selected from Hal, NO₂, CN, SO₂-C₁-C₆ alkyl, NMe₂, linear or branched alkyl having 1 to 6 carbon atoms, O-C₁-C₆-alkyl, -(CH₂)ₘ-O-C₁-C₆-alkyl, CF₃, or a 5-6-membered heteroaromatic group,
- T: denotes a phenyl, a triazole, a thiazole, an oxazole, an oxadiazole or an imidazol group,
- R¹, R², R^{2'}: are independently from one another H, Hal, O-C₁-C₆-alkyl, -(CH₂)ₘ-O-C₁-C₆-alkyl, O-C₁-C₆-halo-alkyl, -(CH₂)ₘ-O-C₁-C₆-halo-alkyl, -SO₂-C₁-C₆-alkyl, -(CH₂)ₘ-SO₂-C₁-C₆-alkyl, -SO₂-C₁-C₆-halo-alkyl, -(CH₂)ₘ-SO₂-C₁-C₆-halo-alkyl, -SO₂-3-8-cycloalkyl, -(CH₂)ₘ-SO₂-3-8-cycloalkyl, CF₃, or two of R¹,R² and R^{2'} are linked to form with the ring T to which they are attached a 7-12 fused bicyclic carbocyclic ring, optionally containing 1 to 3 groups independently selected from an oxygen atom, a sulphur atom, a SO₂ group, or a SO group, and optionally substituted with 1 to 3 Hal, CF₃, NO₂ CN, a linear or branched alkyl group having 1 to 6 carbon atoms, -(CH₂)ₘ-O-C₁-C₆-alkyl, -O-C₁-C₆-alkyl,
- R³: is a linear or branched C₁-C₆-alkyl group, a 3-8-membered cycloalkyl group, - (CH₂)ₘ-O-C₁-C₆-alkyl, -O-C₁-C₆-alkyl , or a 3-8-membered heterocyclic group, each of the above group being optionally substituted with 1 to 3 substitutents independently selected from Hal, CF₃, NO₂, CN, a linear or branched alkyl group having 1 to 6 carbon atoms, -(CH₂)ₘ-O-C₁-C₆-alkyl, -(CH₂)ₘ-SO₂-C₁-C₆-alkyl, -SO₂-C₁-C₆-alkyl, -O-C₁-C₆-alkyl,-(CH₂)ₘ-O-C₁-C₆-halo-alkyl, -(CH₂)ₘ-SO₂-C₁-C₆-halo-alkyl, -SO₂-C₁-C₆-halo-alkyl, or -O-C₁-C₆-halo-alkyl,
- R⁴: denotes H, a linear or branched C₁-C₆-alkyl group, or form together with R³ a 3-8-membered cycloalkyl, optionally substituted with Hal, CF₃, NO₂, CN, a linear or branched alkyl group having 1 to 6 carbon atoms, -(CH₂)ₘ-O-C₁-C₆-alkyl, -O-C₁-C₆-alkyl,
- m: is selected from 1, 2, 3 or 4, preferably 1 or 2,
- Hal: is F, Cl, Br, I, As well as pharmaceutically acceptable derivatives, solvates, tautomers, salts, hydrates and stereoisomers thereof, including mixtures thereof in all ratios.

When two or more adjacent groups selected from G¹, X, Q are a single bond, they denote together one single bond. The same applies for other groups of substituents such as G², Y, W.

Therapeutic strategies for the effective management of autoimmune disorders and immune-mediated disorders are sought.

Potassium channels represent a complex class of voltage-gated ion channels from both functional and structural standpoints. Their functions include regulating neurotransmitter release, heart rate, insulin secretion, neuronal excitability, epithelial electrolyte transport, smooth muscle contraction, and cell volume. In general, four sequence-related potassium channel genes - shaker, shaw, shab, and shal - have been identified in Drosophila, and each has been shown to have human homolog(s). KCNA3 encodes the voltage-gated Kᵥ1.3 potassium channel, which is shaker-related and is expressed in lymphocytes (T and B lymphocytes), the central nervous system, fat and other tissues. The functional channel is composed of four identical Kᵥ1.3 α-sub units. The Kᵥ1.3 potassium channel regulates membrane potential and thereby indirectly influences calcium signaling in human effector- memory T cells (Grissmer S. et al, Proc. Natl. Acad. Sci. U.S.A. 87(23): 9411-5; DeCoursey T.E. et al, Nature 307 (5950): 465-8; Chandy K.G. et al, Trends Pharmacol. Sci. 25(5): 280-9; Wulff H. et al, J. Clin. Invest. 111 (11): 1703-13). Effector memory T cells are important mediators of multiple sclerosis, Type I diabetes mellitus, psoriasis, and rheumatoid arthritis.

The Kᵥ1.3 channel is expressed in T and B lymphocytes in a distinct pattern that depends on the state of lymphocyte activation and differentiation. Upon activation, naive and central memory T cells increase expression of the KCa3.1 channel per cell, while effector-memory T cells increase expression of the Kᵥ1.3 channel. Amongst human B cells, naive and early memory B cells express small numbers of Kᵥ1.3 and KCa3.1 channels when they are quiescent, and augment KCa3.1 expression after activation. In contrast, class-switched memory B cells express high numbers of Kᵥ1.3 channels per cell (about 1500/cell) and this number increases after activation (Chandy K. G. et al, Trends Pharmacol. Sci. 25(5): 280-9; Wulff H. et al, J. Clin. Invest. III (11): 1703-13; Wulff H. et al, J. Immunol. 173(2): 776- 86). The Kᵥ 1.3 channel promotes the calcium homeostasis required for T-cell receptor-mediated cell activation, gene transcription, and proliferation (Panyi, G et al (2004) Trends Immunol 25:565-569). Kv1.3 is physically coupled through a series of adaptor proteins to the T-cell receptor signaling complex and it traffics to the immunological synapse during antigen presentation. However, blockade of the channel does not prevent immune synapse formation (Panyi G. et al, Proc. Natl. Acad. Sci. U.S.A., 101 (5):1285-90; Beeton C. et al, Proc. Natl. Acad. Sci. U.S.A., 103(46): 17414-9).Kᵥ1.3 and KCa3.1 regulate membrane potential and calcium signaling of T cells. Calcium entry through the CRAC channel is promoted by potassium efflux through the Kv1.3 and KCa3.1 potassium channels. Blockade of Kᵥ1.3 channels in effector-memory T cells suppresses activities like calcium signaling, cytokine production (interferon-gamma, interleukin 2) and cell proliferation. Effector-memory T cells (TEM) were originally defined by their expression of cell surface markers, and can enter sites of inflammation in non-lymphoid tissues, while not participating in the process of lymphoid recirculation carried out by most other lymphocytes. TEMs have been shown to uniquely express high numbers of the Kᵥ1.3 potassium channel and depend on these channels for their function. In vivo, Kᵥ1.3 blockers paralyze effector-memory T cells at the sites of inflammation and prevent their reactivation in inflamed tissues. In contrast, Kᵥ1.3 blockers do not affect the homing to and motility within lymph nodes of naive and central memory T cells, most likely because these cells express the KCa3.1 channel and are therefore protected from the effect of Kᵥ1.3 blockade. Suppressing the function of these cells by selectively blocking the Kᵥ1.3 channel offers the potential for highly effective therapy of autoimmune diseases with minimal effects on either beneficial immune responses or other organs (Chandy K.G. et al, Trends Pharmacol. Sci. 25(5): 280-9; Wulff H. et al, J. Clin. Invest. III (11): 1703-13; Beeton C. et al, Proc. Natl. Acad. Sci. U.S.A., 103(46): 17414-9; Matheu M. P. et al, Immunity 29(4): 602-14). Kᵥ1.3 has been reported to be expressed in the inner mitochondrial membrane in lymphocytes. The apoptotic protein Bax has been suggested to insert into the outer membrane of the mitochondria and occlude the pore of Kᵥ1.3 via a lysine residue. Thus, Kᵥ1.3 blockade may contribute to apoptosis (Szabo I. et al, J. Biol. Chem. 280(13): 12790-8; Szabo I. et al., Proc. Natl. Acad. Sci. U.S.A. 105(39): 14861-6).

Autoimmune Disease is a family of disorders resulting from tissue damage caused by a malfunctioning immune system, affecting tens of millions of people worldwide. Such diseases may be restricted to a single organ, as e.g. in multiple sclerosis and Type I diabetes mellitus, or may involve multiple organs as in the case of rheumatoid arthritis and systemic lupus erythematosus. Treatment is generally palliative and typically includes anti-inflammatory and immunosuppressive drugs. The severe side effects of many of these therapies have fueled a continuing search for more effective and selective immunosuppressive drugs. Among these are those which can selectively inhibit the function of effector-memory T cells, known to be involved in the etiology of many of these autoimmune diseases and thereby ameliorate many autoimmune diseases without compromising the protective immune response. Multiple sclerosis is a disease caused by autoimmune damage to the central nervous system including the brain, which affects roughly two and a half million people worldwide. Symptoms include muscle weakness and paralysis, and the disease can progress rapidly and unpredictably and may eventually lead to death. Treatment usually includes the use of anti-inflammatory and immunosuppressive drugs which have potentially severe side effects. Kᵥ1.3 has been shown to be highly expressed in autoreactive effector memory T cells from MS patients (Wulff, H et al (2003) J Clin Invest 111 :1703-1713; Rus H et al (2005) PNAS 102:11094-11099). Animal models of multiple sclerosis have been successfully treated using blockers of the Kᵥ1.3 potassium channel. In patients with multiple sclerosis, disease-associated myelin-specific T cells from the blood are predominantly co-stimulation independent effector-memory T cells that express high numbers of Kᵥ1.3 channels. T cells in MS lesions in postmortem brain lesions are also predominantly effector-memory T cells that express high levels of the Kᵥ1.3 channel (Wulff H. et al, J. Clin. Invest. 111(11): 1703-13; Beeton C. et al, Proc. Natl. Acad. Sci. U.S.A. 103(46): 17414-9).

Type 1 diabetes mellitus is a disease caused by autoimmune destruction of insulin-producing cells in the pancreas, resulting in high blood sugar and other metabolic abnormalities. Type 1 diabetes mellitus affects close to four hundred thousand people in the US alone, and is usually diagnosed before age 20. Its long-term consequences may include blindness, nerve damage and kidney failure, and left untreated is rapidly fatal. Treatment involves life-long administration of insulin or pancreas transplantation, both of which may entail serious side effects (Beeton C. et al, Proc. Natl. Acad. Sci. U.S.A. 103(46): 17414-9).

Kᵥ1.3 is also considered a therapeutic target for the treatment of obesity, for enhancing peripheral insulin sensitivity in patients with type-2 diabetes mellitus, for preventing bone resorption in periodontal disease, for rheumatoid arthritis, for inflammatory skin conditions, such as psoriasis, and for asthma (Tucker K. et al, Int. J. Obes. (Lond) 32(8): 1222-32; Xu J. et al, Hum. Mol Genet. 12(5): 551-9; Xu J. et al, Proc. Natl. Acad. Sci. U.S.A. 101 (9): 3112-7; Valverde P. et al, J. Dent. Res 84(6): 488-99; Tschritter O. et al, J. Clin. Endocrinol. Metab. 91 (2): 654-8; Beeton, C. et al, Proc. Natl. Acad. Sci. U.S.A. 103(46): 17414-17419; Azam, P. et al, J. Invest. Derm. 127: 1419-1429; Bradding, P et al, Br. J. Pharmacol. 157: 1330-1339).

Compounds which are selective Kᵥ1.3 blockers are thus potential therapeutic agents as immunosuppressants or immune system modulators including for the prevention of graft rejection, and the treatment of autoimmune and inflammatory disorders. Kᵥ1.3 modulators may be used alone or in conjunction with other immunosuppressants, such as selective KCa3.1 blockers or cyclosporin, in order to achieve synergism and/or to reduce toxicity, especially of cyclosporin. At present there exist a number of non-selective K channels that will inhibit lymphocyte proliferation, but have adverse side effects. Other K channels exist in a wide range of tissues including the heart and brain, and generally blocking these channels is undesirable. U.S. Patent No. 5,494,895 discloses the use of a thirty-one amino acid peptide, scorpion peptide margatoxin, as a selective inhibitor and probe of Kᵥ1.3 channels present in human lymphocytes, and also as an immunosuppressant. However the use of this compound is limited by its potent toxicity.

International patent Application publications numbers WO 97/16438 and WO 09/716437, and US Patent No. 6,051,590 describe the use of the triterpene, correolide and related compounds as immunosuppressants in the treatment of conditions in mammals affected or facilitated by Kᵥ1.3 inhibition.

There is still a need for improved and specific therapies for immune diseases, including autoimmune diseases, and for immunosuppressive agents which lack problematic side effects and specifically target channels involved in immune cell mediated actions.

WO 2005/090315, WO01/10799, and WO 02/083143 provide various compounds useful in the treatment of inflammatory diseases.

### SUMMARY OF THE INVENTION

The present invention provides compounds of Formula (I) and related Formulae, and pharmaceutical compositions thereof. Compounds of Formula (I) have potency and selectivity in the prevention and treatment of conditions that have been associated with autoimmune disorders, immune-mediated disorders, inflammatory disorders, or other disorders, or conditions which benefit clinically from immunosuppressants, including multiple sclerosis, type-1 diabetes mellitus, type-2 diabetes mellitus, rheumatoid arthritis, systemic lupus erythematosus, psoriasis, contact dermatitis, obesity, graft-versus host disease, transplant rejection, and delayed type hypersensitivity. In particular, compounds, pharmaceutical compositions and methods provided are useful to treat, prevent or ameliorate a range of conditions in mammals such as, but not limited to, immune disorders and autoimmune diseases of various genesis or etiology, for example rheumatoid arthritis, multiple sclerosis, psoriasis, type 1 diabetes, graft-versus host disease, transplant rejection. In some embodiments, compounds, pharmaceutical compositions and methods provided are useful as antiinflammatory agents for the treatment of arthritis, and as agents to treat Parkinson's Disease, Alzheimer's Disease, asthma, myocardial infarction, neurodegenerative disorders, inflammatory bowel disease and autoimmune disorders, renal disorders, obesity, eating disorders, cancer, schizophrenia, epilepsy, sleeping disorders, cognitive disorders, depression, anxiety, blood pressure, and lipid disorders.

In a second aspect, the present invention provides a kit or a set comprising at least one compound of Formula (I), preferably in combination with immunomodulating agents. Preferably, the kit consists of separate packs of:
(a) an effective amount of a compound of the formula (I) and/or pharmaceutically usable derivatives, solvates, salts, hydrates and stereoisomers thereof, including mixtures thereof in all ratios, and
(b) an effective amount of a further medicament active ingredient.

In another aspect, the present invention relates to pharmaceutical compositions comprising a compound provided herein, and a pharmaceutical carrier, excipient or diluent. The pharmaceutical composition can comprise one or more of the compounds described herein. It will be understood that compounds provided herein useful in the pharmaceutical compositions and treatment methods disclosed herein, can be pharmaceutically acceptable as prepared and used.

In another aspect, the present invention relates to methods for preventing, treating or ameliorating a condition from among those listed herein. In particularly, conditions that are associated with immune-mediated reactions, autoimmune conditions, or other conditions which are modulated by immunosuppression. Examples of these conditions are multiple sclerosis, type-1 diabetes mellitus, rheumatoid arthritis, psoriasis, contact dermatitis, obesity, systemic lupus erythematosus, graft-versus host disease, and transplant rejection, which method comprises administering to a mammal in need thereof an amount of one or more of the compounds provided herein, or pharmaceutical composition thereof, effective to prevent, treat or ameliorate the condition.

In addition to the methods of treatment set forth above, the present invention extends to the use of any of the compounds of the invention for the preparation of medicaments that may be administered for such treatments, as well as to such compounds for the treatments disclosed and specified. In additional aspects, the present invention is directed to methods for synthesizing the compounds described herein, with representative synthetic protocols and pathways described below.

Accordingly, it is a principal object of this invention to provide a novel series of compounds which can modulate the activity of the voltage gated potassium channel Kᵥ 1.3, and thus avert or treat any maladies that may be causally related to aberrations in such activity.

It is further an object of this invention to provide a series of compounds that can treat or alleviate maladies or symptoms of same, such as immune-mediated disorders and autoimmune diseases , that may be causally related to the activation of the Kv1.3 channel.

It is further an object of this invention to provide a series of compounds that can treat a disease or condition, wherein the disease or condition is selected from: Acute disseminated encephalomyelitis (ADEM), Addison's disease, Allopecia areata, Alzheimers disease, Ankylosing spondylitis, Antiphospholipid antibody syndrome, Autoimmune hemolytic anemia, Autoimmune hepatitis, Autoimmune inner ear disease, Autoimmune Lymphoproliferative Syndrome (ALPS), Autoimmune polyendocrine/polyglandular syndrome, Autoimmune thrombocytoipenia purpura, Balo disease, Behcet disease, Bullous pemphigoid, Cardiomyopathy, Celiac sprue-dermatitis herpetiformis, Chronic fatigue immune dysfunction syndrome (CFIDS), Chronic inflammatory demyelinating neuropathy, Cicatrical pemphigoid, Coeliac disease, Cold agglutinin disease, CREST syndrome, Crohn's disease, Cystic fibrosis, Degos disease, Dermatomyositis, Diabetes (Type I or Juvenile onset), Early onset dementia, Eczema, Endotoxin shock, Essential mixed cryoglobulinemia, Familial Mediterranean fever, Fibromyalgia, Fibromyositis, Goodpasture's syndrome, Graves' disease, Guillain-Barre syndrome (GBS), Hashimoto's thyroidosis, Hidradenitis suppurativa, Idiopathic pulmonary fibrosis, Idiopathic thrombocytopenic purpura, IgA nephropathy, Lambert-Eaton Myasthenic Syndrome, Leukemia, Lichen planus, Meniere disease, Mixed connective tissue disease, Multiple sclerosis, Multiphasic disseminated encephalomyelitis, Myasthenia gravis, Neuromyelitis Optica, Paraneoplastic Syndromes, Pemphigus, Pemphigus vulgaris, Pernicious anaemia, Polyarteritis nodosum, Polychondritis, Polymyalgia rhematica, Polymyositis, Primary agammaglobulinemia, Primary biliary cirrhosis, Plaque Psoriasis, Psoriatic arthritis, Raynaud phenomenon, Reiter syndrome, Restenosis following angioplasty, Rheumatic fever, Rheumatoid arthritis, Rheumatoid psoriasis, Sarcoidosis, Scleroderma, Sepsis, Sezary's disease, Sjogren's syndrome, Stiff- person syndrome, Systemic lupus erythematosis (SLE), Takayasu arteritis, Temporal arteritis (also known as "giant cell arteritis"), Transplant or Allograft rejection, Ulcerative colitis, Uveitis, Vasculitis, Vitiligo, Graft vs Host disease, pustular psoriasis, and Wegener's granulomatosis.

It is further an object of this invention to provide a series of compounds that can treat a disease or condition, wherein the disease or condition is selected from: resistance by transplantation of organs or tissue, graft- versus-host diseases brought about by medulla ossium transplantation, rheumatoid arthritis, systemic lupus, erythematosus, Hashimoto's thyroiditis, multiple sclerosis, myasthenia gravis, type I diabetes uveitis, juvenile- onset or recent-onset diabetes mellitus, posterior uveitis, allergic encephalomyelitis, glomerulonephritis, infectious diseases caused by pathogenic microorganisms, inflammatory and hyperproliferative skin diseases, psoriasis, atopical dermatitis, contact dermatitis, eczematous dermatitises, seborrhoeis dermatitis, Lichen planus, Pemphigus, bullous pemphigoid, Epidermolysis bullosa, urticaria angioedemas, vasculitides, erythemas, cutaneous eosinophilias, Lupus erythematosus, acne, Alopecia areata, keratoconjunctivitis, vernal conjunctivitis, uveitis associated with Behcet's disease, keratitis, herpetic keratitis, conical cornea, dystrophia epithelialis corneae, corneal leukoma, ocular pemphigus, Mooren's ulcer, Scleritis, Graves' opthalmopathy, Vogt-Koyanagi-Harada syndrome, sarcoidosis, pollen allergies, reversible obstructive airway disease, bronchial asthma, allergic asthma, intrinsic asthma, extrinsic asthma, dust asthma, chronic or inveterate asthma, late asthma and airway hyper-responsiveness, bronchitis, gastric ulcers, vascular damage caused by ischemic diseases and thrombosis, ischemic bowel diseases, inflammatory bowel diseases, necrotizing enterocolitis, intestinal lesions associated with thermal bums and leukotriene B₄-mediated diseases, Coeliac diseases, proctitis, eosinophilic gastroenteritis, mastocytosis, Crohn's disease, ulcerative colitis, migraine, rhinitis, eczema, interstitial nephritis, Good-pasture's syndrome, hemolytic-uremic syndrome, diabetic nephropathy, multiple myositis, Guillain-Barre syndrome, Meniere's disease, polyneuritis, multiple neuritis, mononeuritis, radiculopathy, hyperthyroidism, Basedow's disease, pure red cell aplasia, aplastic anemia, hypoplastic anemia, idiopathic thrombocytopenic purpura, autoimmune hemolytic anemia, agranulocytosis, pernicious anemia, megaloblastic anemia, anerythroplasia, osteoporosis, sarcoidosis, fibroid lung, idiopathic interstitial pneumonia, dermatomyositis, leukoderma vulgaris, ichthyosis vulgaris, photoallergic sensitivity, cutaneous T cell lymphoma, arteriosclerosis, atherosclerosis, aortitis syndrome, polyarteritis nodosa, myocardosis, scleroderma, Wegener's granuloma, Sjogren's syndrome, adiposis, eosinophilic fascitis, lesions of gingiva, periodontium, alveolar bone, substantia ossea dentis, glomerulonenephritis, male pattern alopecia or alopecia senilis by preventing epilation or providing hair germination and/or promoting hair generation and hair growth, muscular dystrophy; Pyoderma and Sezary's syndrome, Addison's disease, ischemia-reperfusion injury of organs which occurs upon preservation, transplantation or ischemic disease, endotoxin-shock, pseudomembranous colitis, colitis caused by drug or radiation, ischemic acute renal insufficiency, chronic renal insufficiency, toxinosis caused by lung-oxygen or drugs, lung cancer, pulmonary emphysema, cataracta siderosis, retinitis pigmentosa, senile macular degeneration, vitreal scarring, corneal alkali burn, dermatitis erythema multiforme, linear IgA ballous dermatitis and cement dermatitis, gingivitis, periodontitis, sepsis, pancreatitis, diseases caused by environmental pollution, aging, carcinogenis, metastasis of carcinoma and hypobaropathy, disease caused by histamine or leukotriene-C₄ release, Behcet's disease, autoimmune hepatitis, primary biliary cirrhosis sclerosing cholangitis, partial liver resection, acute liver necrosis, necrosis caused by toxin, viral hepatitis, shock, or anoxia, B-virus hepatitis, non-A/non-B hepatitis, cirrhosis, alcoholic cirrhosis, hepatic failure, fulminant hepatic failure, late-onset hepatic failure, "acute- on-chronic" liver failure, augmentation of chemotherapeutic effect, cytomegalovirus infection, HCMV infection, AIDS, cancer, senile dementia, trauma, and chronic bacterial infection.

A still further object of this invention is to provide pharmaceutical compositions that are effective in the treatment or prevention of a variety of disease states, including the diseases associated with the central nervous system, cardiovascular conditions, chronic pulmonary obstructive disease (COPD), inflammatory bowel disease, rheumatoid arthritis, osteoarthritis, and other diseases where an immunological inflammatory component or autoimmune component is present.

In a preferred embodiment, compounds of the present invention are used in the treatment and prophylaxis of a condition selected from Multiple sclerosis, Rheumatoid arthritis, Psoriasis, Type 1 Diabetes, Systemic lupus nephritis, Oncology, Glomerulonephritis, Sjögrens's syndrome, Transplant rejection, Graft versus host disease, Allergic contact dermatitis, Neointimal hyperplasia/restenosis, Periodontal disease, Leprosy, and Obesity

In one embodiment, G¹ and G² in Formula (I) are not both a CO group. In other words, G¹ in the compounds of Formula (I) denotes a single bond or, when G² is a CH₂ group or a single bond, G¹ also denotes a CO group.

Alternatively, G² denotes a single bond or a CH₂ group or, when G¹ is a single bond, G² also denotes a CO group, preferably, G¹ is a single bond and G² is a CO group.

In a second embodiment, the present invention provides compounds of Formula (II):

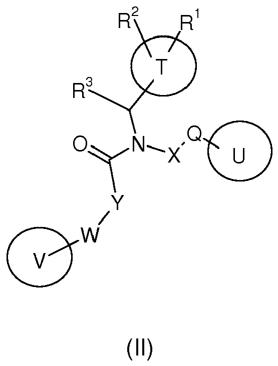

Wherein
- X: is a linear or branched alkylene group having 1 to 6 carbon atoms,
- Q: is O, NH or a single bond, preferably a single bond,
- Y: is a linear or branched alkylene group having 1 to 6 carbon atoms, or a saturated or unsaturated 3-8-membered cycloalkylene group.
- W: is SO, SO₂, CO, or a single bond, preferably a single bond,
- R³: is a linear or branched C₁-C₆-alkyl group, a 3-8-membered cycloalkyl group, - (CH₂)ₘ-O-C₁-C₆-alkyl,

And m, U, T, V, R¹, R² and R³ are as above defined,

As well as their pharmaceutically acceptable derivatives, solvates, tautomers, salts, hydrates and stereoisomers thereof, including mixtures thereof in all ratios.

In a third embodiment, the present invention provides compounds of Formula (III): 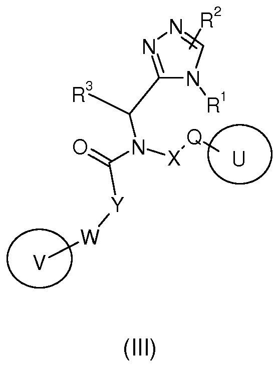 Wherein R², R³, X, Y, Q, U, W, m, and V are as above defined, and wherein R¹ is selected from H, -(CH₂)ₘ-O-C₁-C₆-alkyl, -(CH₂)ₘ-O-C₁-C₆-halo-alkyl, - SO₂-C₁-C₆-alkyl, -(CH₂)ₘ-SO₂-C₁-C₆-alkyl, -SO₂-C₁-C₆-halo-alkyl, -(CH₂)ₘ-SO₂-C₁-C₆-halo-alkyl, -SO₂-3-8-cycloalkyl, -(CH₂)ₘ-SO₂-3-8-cycloalkyl, CF₃, as well as their pharmaceutically acceptable derivatives, solvates, tautomers, salts, hydrates and stereoisomers thereof, including mixtures thereof in all ratios.

In a fourth embodiment, the present invention provides compounds of Formula (IV) 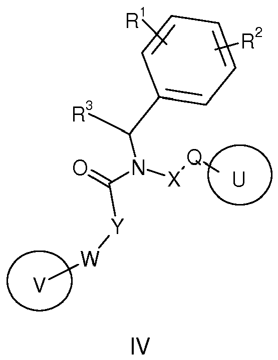 Wherein R¹, R², R³, X, Y, Q, U, W, m, and V are as above defined, as well as their pharmaceutically acceptable derivatives, solvates, tautomers, salts, hydrates and stereoisomers thereof, including mixtures thereof in all ratios.

In a fifth embodiment, the present invention provides compounds of Formula (I) and related Formulae wherein R⁴ denotes H and R³ is selected from the following groups:

| | | | |
|---|---|---|---|
| -CH₃ | -CH₂OCH₃ | -CH(CH₃)₂ | -CH₂CH₃ |
| 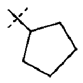 | 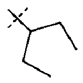 | 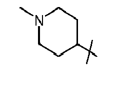 | 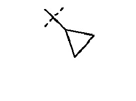 |
| -CH₂OCH₂CF₂H | -CH₂OCH₂CF₃ | 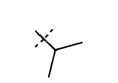 | 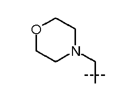 |

Wherein the above-mentioned groups may be further substituted by 1 to 3 substitutents independently selected from Hal, CF₃, NO₂, CN, a linear or branched alkyl group having 1 to 6 carbon atoms, -(CH₂)ₘ-O-C₁-C₆-alkyl, -(CH₂)ₘ-SO₂-C₁-C₆-alkyl, -SO₂-C₁-C₆-alkyl, -O-C₁-C₆-alkyl,-(CH₂)ₘ-O-C₁-C₆-halo-alkyl, -(CH₂)ₘ-SO₂-C₁-C₆-halo-alkyl, -SO₂-C₁-C₆-halo-alkyl, or -O-C₁-C₆-halo-alkyl.

In a sixth embodiment, the present invention provides compounds of Formula (I) and related Formulae wherein the group G¹-X-Q-U is selected from the following groups:

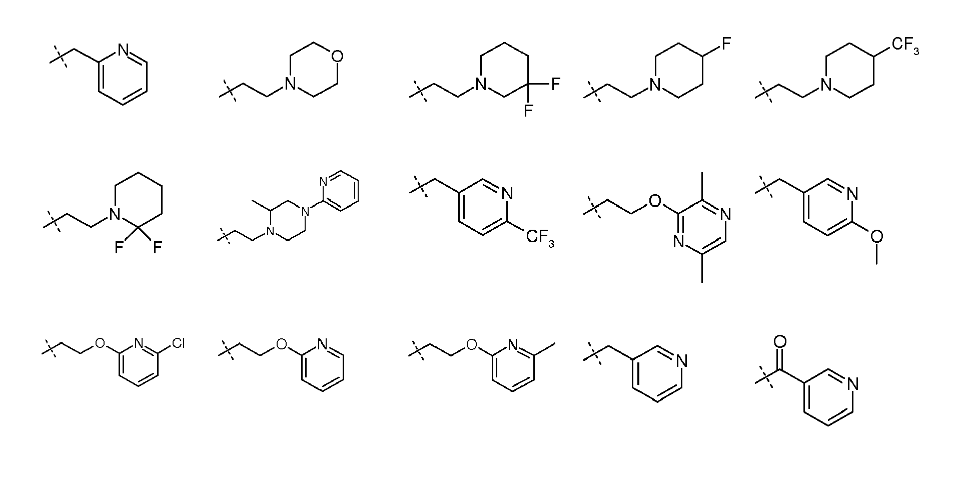

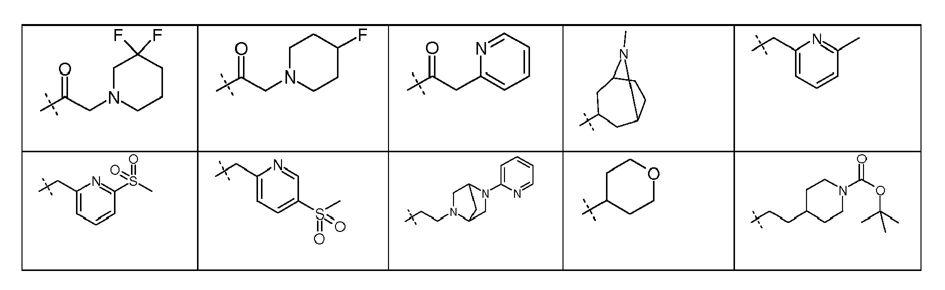

In a seventh embodiment, the group G²-Y-W-V in the compounds of Formula (I) and related Formulae is selected from the following groups:

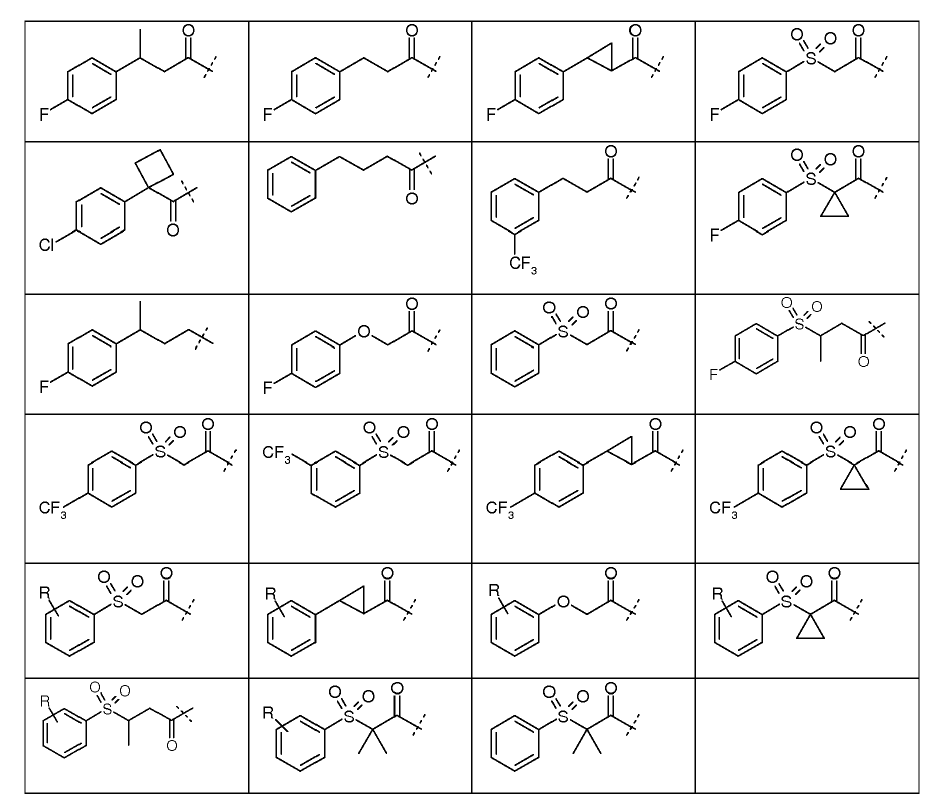

Wherein R denotes Hal, NO₂, CN, SO₂-C₁-C₆ alkyl, NMe₂, linear or branched alkyl having 1 to 6 carbon atoms, O-C₁-C₆-alkyl, -(CH₂)ₘ-O-C₁-C₆-alkyl, CF₃, or a 5-6-membered heteroaromatic group.

In a eighth embodiment, the group 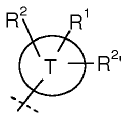 in the compounds of Formula (I) and related Formulae is selected from the following groups:

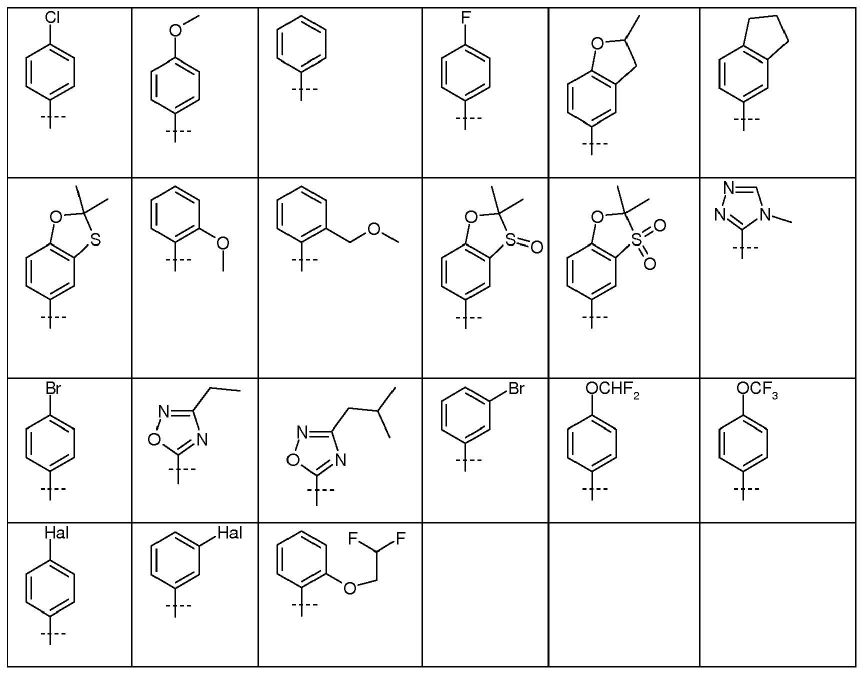

In a ninth embodiment, T is a phenyl ring wherein at least one of R¹, R² or R²' is in meta position with regard to the rest of the molecule.

In one aspect, the present invention provides compounds of Formula (I) and related Formulae wherein

Hal preferably denotes F, Cl or Br, most preferably F, and/or

A 3-8-membered cycloalkyl group denotes cycloalkyl having 3 to 8 carbon atoms, preferably a cyclopropyl, a cyclobutyl, or a cyclopentyl, and/or

A 3-8-membered cycloalkylene group denotes a divalent cycloalkyl having 3 to 8 carbon atoms, preferably cyclopropylene, a cyclobutylene, or a cyclopentylene, and/or

A 3-8-membered heterocyclic group denotes a 3-8-membered cycloalkyl group wherein 1 to 3 carbon atoms is replaced by a group selected from O, S, N, SO, SO₂, CO. A 3-8-membered heterocyclic group preferably denotes one of the following groups:

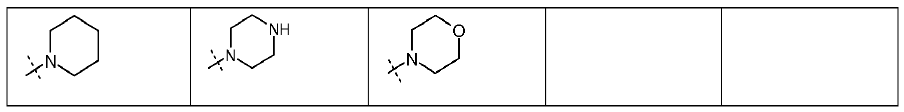

and/or
A 7-12 bridged or fused bicyclic carbocyclic or heterocyclic ring denotes a bicyclic ring having 7 to 12 carbon atoms wherein the 2 rings are fused or bridged, and wherein 1 to 3 carbon atoms may be replaced by a group selected from O, S, N, SO, SO₂, CO. A 7-12 bridged or fused bicyclic carbocyclic or heterocyclic ring preferably denotes one of the following groups:

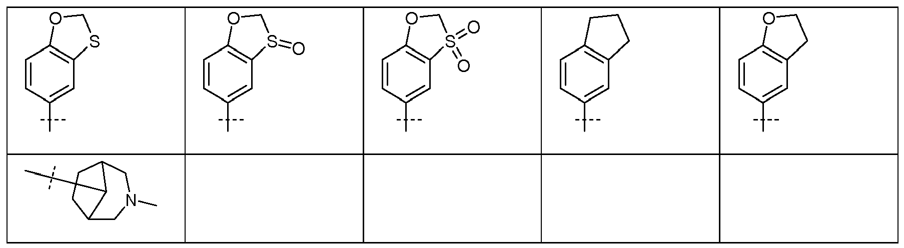

and/or
An aryl denotes a 5-6-membered aromatic group, preferably a phenyl ring, or 8-12-fused bicyclic system wherein at least one of the ring is aromatic, or two 5-6-membered aromatic groups linked through a single bond, such as a biphenyl group,
and/or
A 5-6-membered heteroaromatic group denotes an aromatic ring having 5 or 6 members and containing 1 to 3 heteroatoms selected from N, O or S. A 5-6-membered heteroaromatic group preferably denotes one of the following groups:
wherein these groups may be substituted according to the definitions provided above,
and/or
A C₁-C₆-halo-alkyl denotes a linear or branched alkyl having 1 to 6 carbon atom wherein 1 to 6 H atom is replaced by an halogen, preferably a F atom.

Preferred compounds of the present invention are the following:

| Ex | Structure | Ex | Structure |
|---|---|---|---|
| 1 | 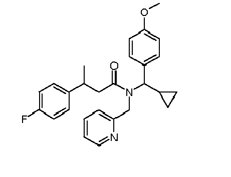 | 2 | 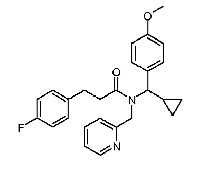 |
| 3 | 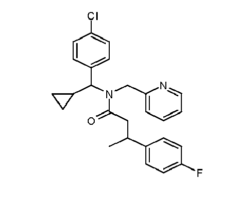 | 9 | 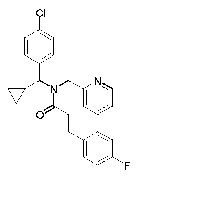 |
| 10 | 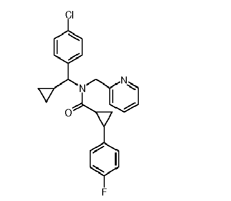 | 15 | 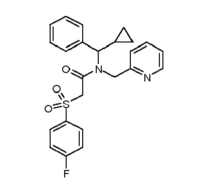 |
| 18 | 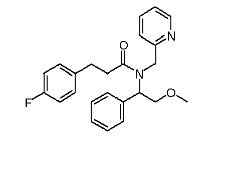 | 20 | 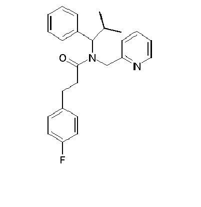 |
| 21 | 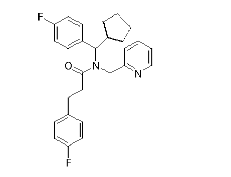 | 22 | 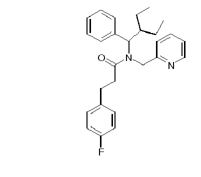 |
| 23 | 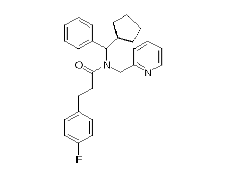 | 24 | 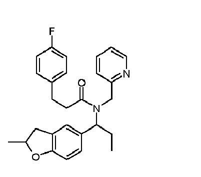 |
| 25 | 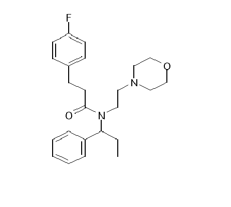 | 27 | 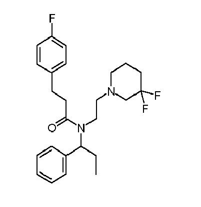 |
| 29 | 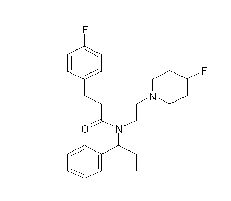 | 31 | 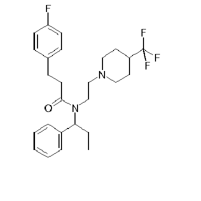 |
| 33 | 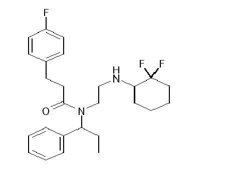 | 35 | 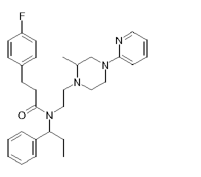 |
| 37 | 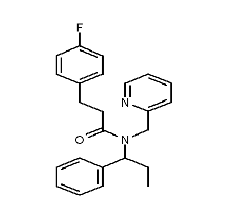 | 39 | 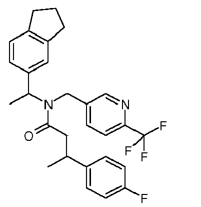 |
| 41 | 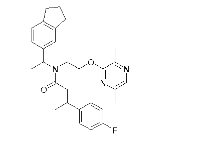 | 42 | 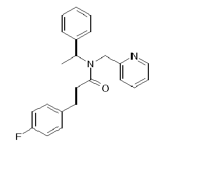 |
| 43 | 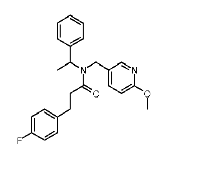 | 44 | 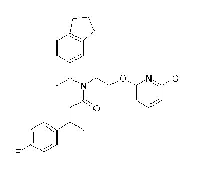 |
| 45 | 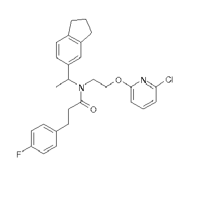 | 46 | 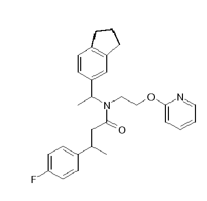 |
| 47 | 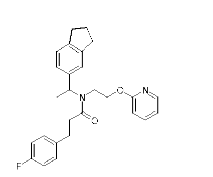 | 48 | 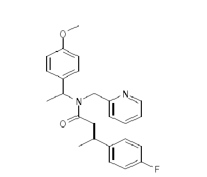 |
| 50 | 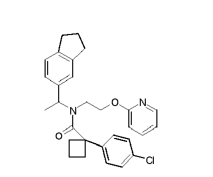 | 51 | 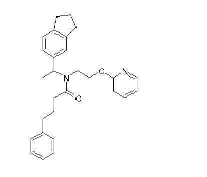 |
| 52 | 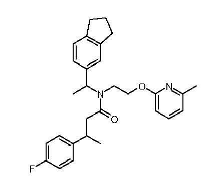 | 53 | 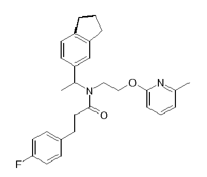 |
| 54 | 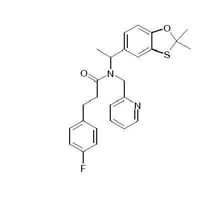 | 55 | 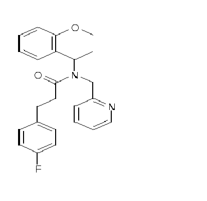 |
| 57 | 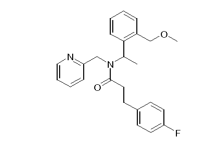 | 60 | 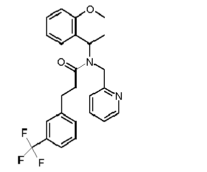 |
| 62 | 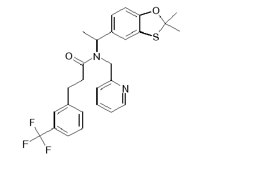 | 63 | 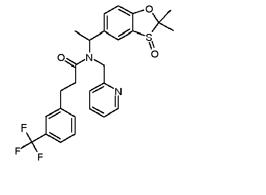 |
| 64 | 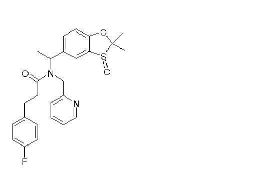 | 65 | 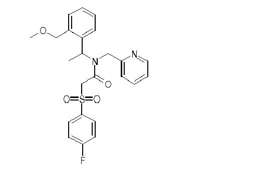 |
| 66 | 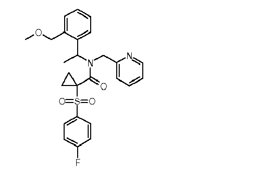 | 67 | 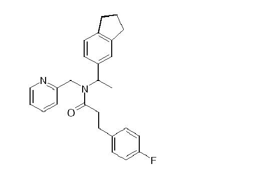 |
| 68 | 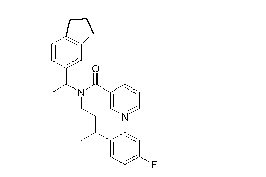 | 69 | 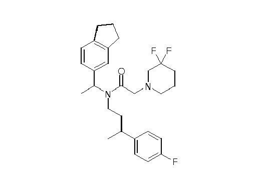 |
| 70 | 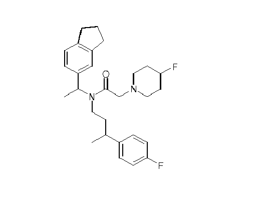 | 71 | 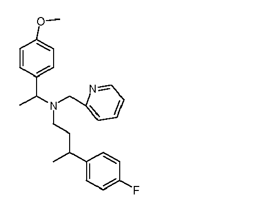 |
| 73 | 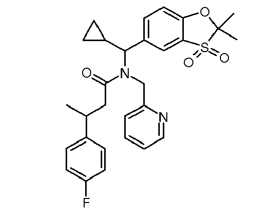 | 75 | 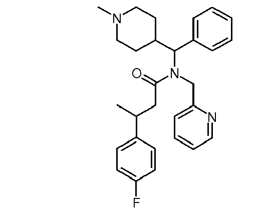 |
| 76 | 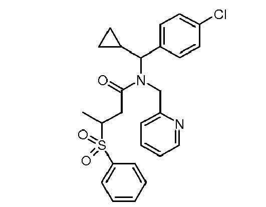 | 77 | 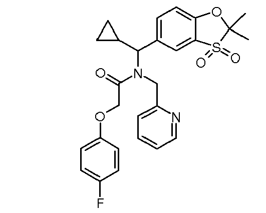 |
| 78 | 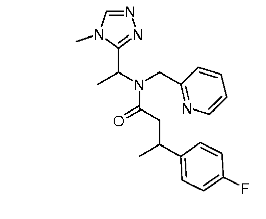 | 79 | 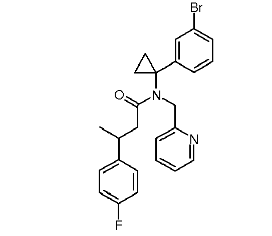 |
| 80 | 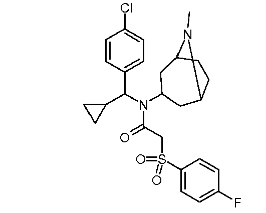 | 81 | 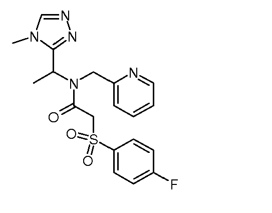 |
| 82 | 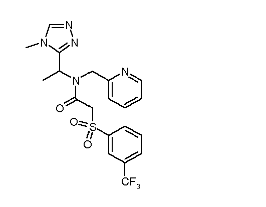 | 83 | 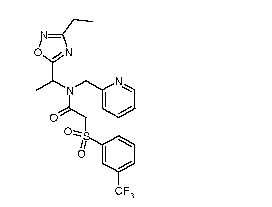 |
| 84 | 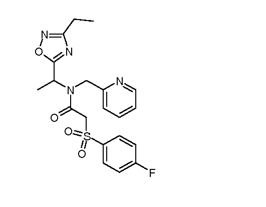 | 85 | 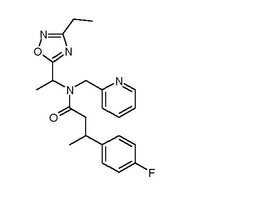 |
| 86 | 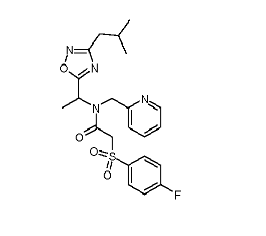 | 87 | 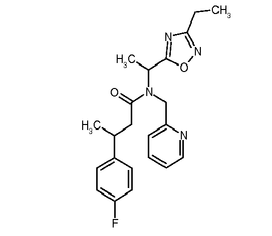 |
| 88 | 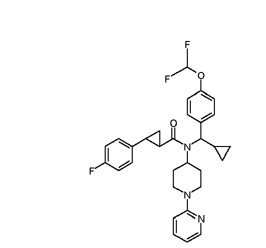 | 89 | 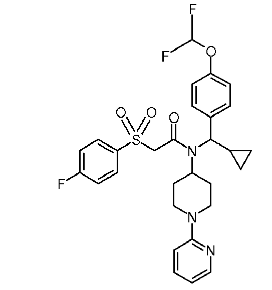 |
| 90 | 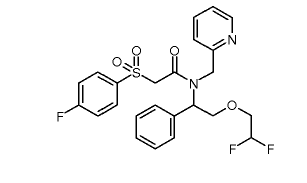 | 91 | 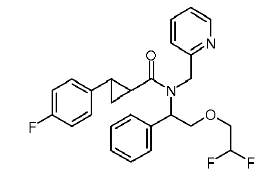 |
| 92 | 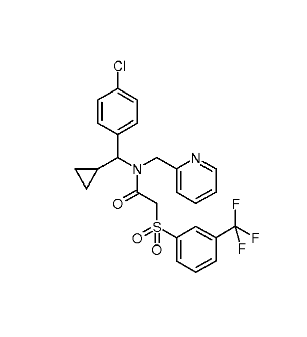 | 93 | 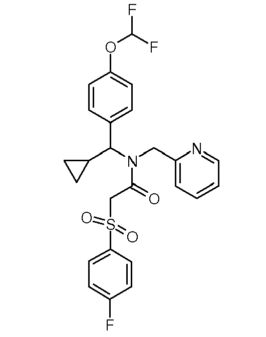 |
| 94 | 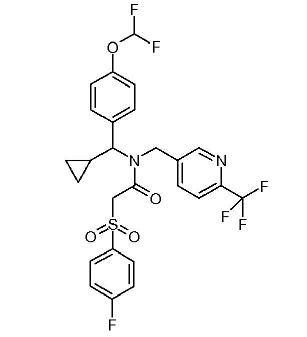 | 95 | 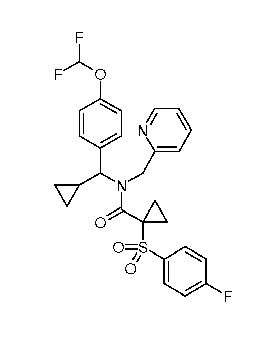 |
| 96 | 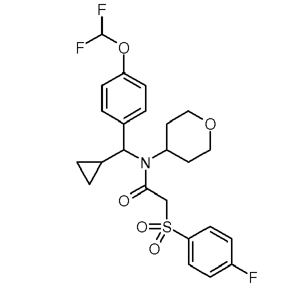 | 97 | 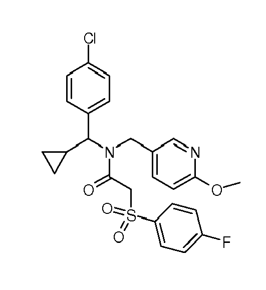 |
| 98 | 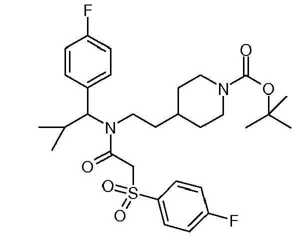 | 99 | 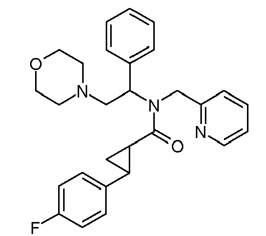 |
| 100 | 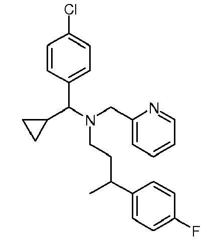 | 101 | 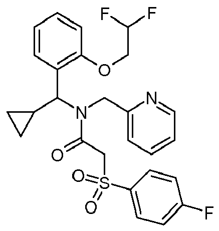 |

'Pharmaceutically acceptable' means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

'Pharmaceutically acceptable salt' refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non toxic organic or inorganic acids, such as hydrochloride, hydrobromide, tartrate, mesylate, acetate, maleate, oxalate and the like.

The term "pharmaceutically acceptable cation" refers to an acceptable cationic counter-ion of an acidic functional group. Such cations are exemplified by sodium, potassium, calcium, magnesium, ammonium, tetraalkylammonium cations, and the like.

'Pharmaceutically acceptable vehicle' refers to a diluent, adjuvant, excipient or carrier with which a compound of the invention is administered.

'Prodrugs' refers to compounds, including derivatives of the compounds of the invention, which have cleavable groups and become by solvolysis or under physiological conditions the compounds of the invention which are pharmaceutically active in vivo. Such examples include, but are not limited to, choline ester derivatives and the like, N-alkylmorpholine esters and the like.

'Solvate' refers to forms of the compound that are associated with a solvent, usually by a solvolysis reaction. This physical association includes hydrogen bonding. Conventional solvents include water, ethanol, acetic acid and the like. The compounds of the invention may be prepared e.g. in crystalline form and may be solvated or hydrated. Suitable solvates include pharmaceutically acceptable solvates, such as hydrates, and further include both stoichiometric solvates and non-stoichiometric solvates. In certain instances the solvate will be capable of isolation, for example when one or more solvent molecules are incorporated in the crystal lattice of the crystalline solid. 'Solvate' encompasses both solution-phase and isolable solvates. Representative solvates include hydrates, ethanolates and methanolates.

'Therapeutically effective amount' means the amount of a compound that, when administered to a subject for treating a disease, is sufficient to effect such treatment for the disease. The "therapeutically effective amount" includes that amount of a compound or composition that will elicit the biological or medical response of a subject that is being sought by a medical doctor or other clinician. The "therapeutically effective amount" can vary depending on the compound, the disease and its severity, and the age, weight, etc., of the subject to be treated.

'Preventing' or 'prevention' refers to a reduction in risk of acquiring or developing a disease or disorder (i.e., causing at least one of the clinical symptoms of the disease not to develop in a subject that may be exposed to a disease-causing agent, or predisposed to the disease in advance of disease onset.

The term 'prophylaxis' is related to 'prevention', and refers to a measure or procedure the purpose of which is to prevent, rather than to treat or cure a disease.

'Treating' or 'treatment' of any disease or disorder refers, in one embodiment, to ameliorating the disease or disorder (i.e., arresting the disease or reducing the manifestation, extent or severity of at least one of the clinical symptoms thereof). In another embodiment 'treating' or 'treatment' refers to ameliorating at least one physical parameter, which may not be discernible by the subject. In yet another embodiment, 'treating' or 'treatment' refers to modulating the disease or disorder, either physically, (e.g., stabilization of a discernible symptom), physiologically, (e.g., stabilization of a physical parameter), or both. In a further embodiment, "treating" or "treatment" relates to slowing the progression of the disease.

'Compounds of the present invention', and equivalent expressions, are meant to embrace compounds of the Formula(e) as hereinbefore described, which expression includes the prodrugs, the pharmaceutically acceptable salts, and the solvates, e.g., hydrates, where the context so permits. Similarly, reference to intermediates, whether or not they themselves are claimed, is meant to embrace their salts, and solvates, where the context so permits.

The inhibition data shown for the compounds of the list below was calculated using the steady state current amplitude at the end of the depolarising pulse in patch clamp evaluations (as described in the Pharmacology Example 2).

### GENERAL DESCRIPTION OF CHEMISTRY

Compounds of Formula (I) can be made by the reaction of the compounds of Formula 5, wherein R¹, R², R^{2'}, R³, R⁴, G¹, X, Q, T and U are as above defined, with a compound of Formula 8, wherein G¹, Y, W and V are as above defined, and wherein LG denotes a suitable leaving group, as depicted in scheme 1. LG preferably denotes an halogen, preferably chlorine or bromine, a sulfonate, or denotes an activated acid derivative obtained by the reaction of a carboxylic acid in the presence of an amide coupling agent. The amide coupling agents include EDCI, BOP, PyBOP, HOBt, HATU, T3P, DCC. They can be used in a suitable solvent, as for example dichloromethane or dimethylformamide at room temperature. Under preferred conditions, the secondary amines 5 is converted to compounds of Formula (I) by reaction with an activated acid such as an acid chloride, in dichloromethane at room temperature, or a mixed anhydride or a N-succinimide ester in ethanol at room temperature or by reaction with an acid in the presence of an amide coupling reagent selected from EDCI, BOP, PyBOP, HOBt, HATU, T3P, DCC in dichloromethane or dimethylformamide at room temperature.

Alternatively, compounds of Formula (I) can be synthesised by reacting a compound of Formula 7, wherein R¹, R², R²', R³, R⁴, G², Y, W, T and V are as above defined, with a compound of Formula 9, wherein G¹, X, Q, U are as above define and wherein LG denotes a suitable leaving group. Examples of such alkanes having LG groups are alkyl halides or alkyl sulfonates. The reaction of compounds of Formula 7 with compounds of Formula 9 is preferably performed in the presence of a base such as sodium hydride, potassium *tert*-butoxide, potassium carbonate preferably in dimethylformamide or acetonitrile. The temperature of the reaction is between room temperature and 100 °C, preferably between 20 and 60°C. Optionally, a phase transfer catalyst, such as tetra-n-butylammonium bromide can be used. Preferred conditions are the use of acetone or acetonitrile with heating at 45-100 °C.

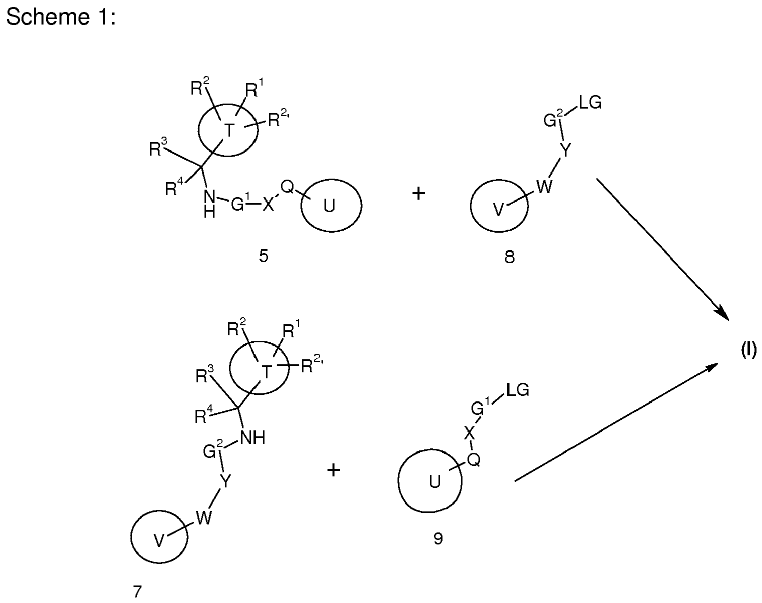

When R⁴ is H, the compounds of Formula 5 can be synthesised by reacting ketones 1, wherein R¹, R², R²', R³ and T are as above defined, with amines 2, wherein G¹, X, Q and U are as above defined, according to scheme 2. This reaction is preferably performed by reductive amination via an imine intermediate.

Alternatively, when G¹ is a single bond, compounds of Formula 5, wherein R¹, R², R²', R³, R⁴, X, Q, T and U are as defined above, may be synthesised by reacting a compound of Formula 3, wherein R¹, R², R²', R³, R⁴ and T are as above defined, with the aldehyde of Formula 4, wherein Q and U are as above defined, and wherein X' denotes a linear or branched alkyl having 1 to 6 carbon atom or a cyclic alkyl having 3 to 8 carbon atoms wherein 1 -CH₂- group, in this linear, branched or cyclic alkyl, is replaced by a -CO- group. Scheme 2.

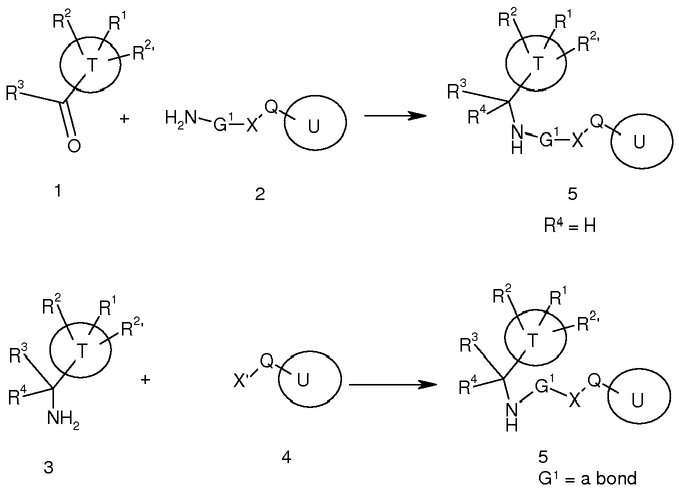

The reductive amination reaction described in scheme 2 can occur in a single pot reaction using borohydride reagants, including but not limited to sodium cyanoborohydride, sodium acetoxyboro-hydride and sodium borohydride in halogenated solvents such as dichloromethane or 1,2-dichloroethane, or alcohols such as methanol, typically at room temperature for 0.5-12 hours.

The reaction can also occur in two steps. Firstly by the formation of imine in the presence of an acid including but not limited to p-toluenesulfonic acid, or Amberlyst resin and also a dehydrating reagent, such as but not limited to magnesium sulphate, sodium sulphate, molecular sieves or TiCl₄ using for example dichloromethane, ethanol, ethyl acetate or dimethyl sulfoxide as solvent. This step may be performed in a Dean Stark apparatus using toluene at 90-110 °C. In a second step, the imine can be converted to the secondary amine using borohydride reagents as described above.

Secondary amines **5** may be prepared by the alkylation of primary amines **2** with a compound of Formula 6 or the amine **3** with a compound of Formula 10, wherein LG in Formulae 6 and 10 denotes a suitable leaving group. Such suitable leaving groups may be selected from halogen, preferably chlorine or bromine, or a sulfonate group, preferably selected from mesylate, tosylate, benzyl sulphonyl, a perfluoroalkyl sulfonate such as mono, di or trifluoromethyl sulfonate or triflate. The reaction is preferably performed in the presence of a base, such as potassium carbonate or triethylamine, in solvents preferably selected from dichloromethane, acetonitrile, dimethyl sulfoxide, at temperatures ranging from room temperature to 100 °C, As disclosed in scheme 3.

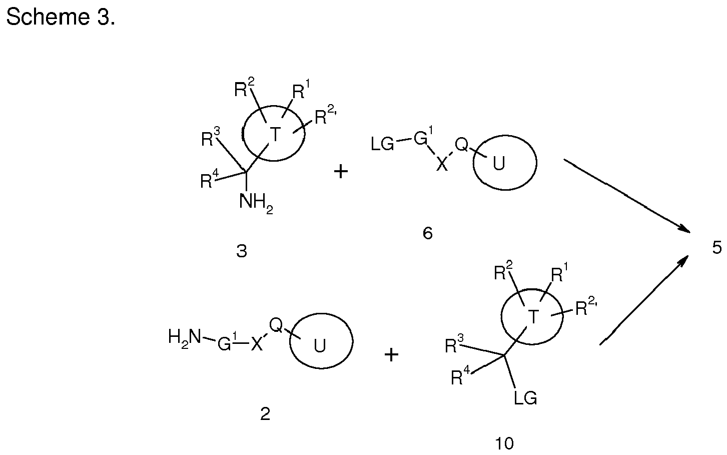

Compounds of Formula 7 may be prepared by reacting a compound of Formula 3, wherein R¹, R², R²', R³, R⁴ and T are as above defined, with a compound of Formula 8, wherein G², Y, W, and V are as above defined, and wherein LG is as defined above, as mentioned in scheme 4.

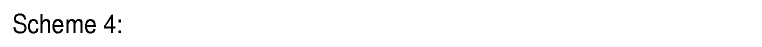

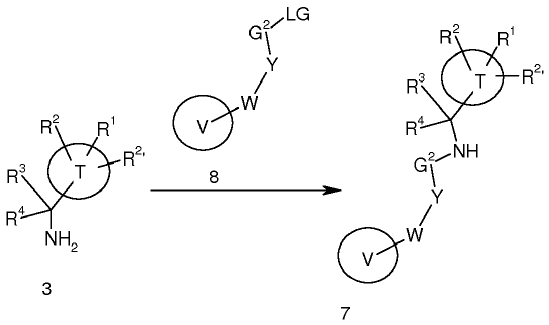

Amines of Formula **3** can be acylated with compounds of Formula 8, using techniques well known in the art. For this purpose, LG in the compounds of Formula 8 is preferably an activated acid obtained by reaction of a COOH group in the presence of an amide coupling reagent. The amide coupling reagents include EDCI, BOP, PyBOP, HOBt, HATU, T3P, DCC. They can be used in a suitable solvent, as for example dichloromethane or dimethylformamide at room temperature.

Amines **3** wherein R⁴ is H can be prepared by functional group transformations well known in the art. Non-limiting examples are provided in Scheme 5. Ketones **11** can be condensed with hydroxylamine, for instance in ethanol at 80-100 °C for 1-2 days to give **12,** which can in turn be reduced to amine **3** using for example Raney nickel in methanol at 80-100 °C for 2-6 hours, or by hydrogenation using palladium on charcoal for instance in ethanol at room temperature for 1-12 hours. Aldehydes **13** can be treated with metallated alkyl species such as Grignard reagents or alkyl lithiums to give secondary alcohols **14** for instance in diethylether or tetrahydrofuran at -78 °C for 1-4 hours, which can be converted to azides **15** using techniques known in the art, for instance by reaction with sodium azide in chloroform and sulfuric acid at 0 °C to room temperature. The azides can be reduced to the amines **3** for example by catalytic hydrogenation with palladium on charcoal in methanol at room temperature or by using PPh₃ / H₂O (Staudinger conditions). Reaction of benzonitriles **16** with Grignard reagents or alkyl lithiums for example diethyl ether or tetrahydrofuran at room temperature to 50 °C for 1-4 hours can give imines **17**, which may be reduced for instance with sodium borohydride in methanol at room temperature or lithium aluminium hydride in dimethyl formamide at room temperature or borane-THF complex in tetrahydrofuran at -20 °C to room temperature to give the amines **3.**

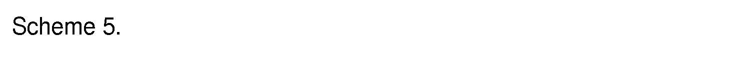

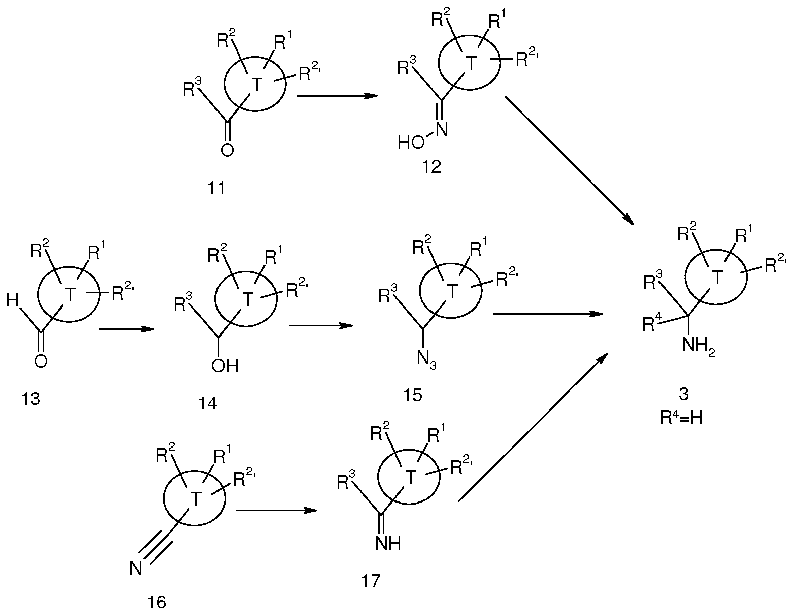

### CHEMISTRY PROTOCOLS

The following abbreviations refer to the abbreviations used below:
AcOH (acetic acid), CAN (acetonitrile) BINAP (2,2'-bis(disphenylphosphino)-1,1'-binaphthalene), dba (dibenzylidene acetone), bs (broad singlet) tBu (*tert*-Butyl), tBuOK (potassium *tert*-butoxide), CDI (1,1'-Carbonyldiimidazole), DBU (1,8-dizabicyclo[5.4.0]undec-7-ene), DCM (dichloromethane), DIAD (diisobutylazodicarboxylate), DIEA (di-isopropyl ethylamine), DMA (dimethyl acetamide), DMAP (4-dimethylaminopyridine), DMSO (dimethyl sulfoxide), DMF (N,N-dimethylformamide), d (doublet), EDC.HCI (1-ethyl-3-(3-dimethylaminopropyl)carbodiimide hydrochloride), equiv. (equivalents), EtOAc (ethyl acetate), EtOH (ethanol), g (gram), cHex (cyclohexane), HPLC (high performance liquid chromatography), hr (hour), LCMS (liquid chromatography - mass spectrometry), MHz (Megahertz), MeOH (methanol), min (minute), mL (milliliter), mmol (millimole), mM (millimolar), mp (melting point), MS (mass spectrometry), MW (microwave), NMM (N-methyl morpholine), m (multiplet), NMR (Nuclear Magnetic Resonance), NBS (N-bromo succinimide), PBS (phosphate buffered saline), PDA (photodiode array), PMB (para-methoxybenzyl), PTLC (preparative thin layer chromatography), Rt (tetention time), RT (room temperature), TBAF (tetrabutylammonium fluoride), TBTU (*N,N,N',N'*-tetramethyl-O-(benzotriazol-1-yl)uronium tetrafluoroborate), T3P (propane phosphonic acid anhydride), TEA (triethyl amine), TFA (trifluoroacetic acid), THF (tetrahydrofuran), t (triplet), PetEther (petroleum ether), TBME (*tert*-butyl methyl ether), TLC (thin layer chromatography), TMS (trimethylsilyl), TMSI (trimethylsilyl iodide), UV (ultraviolet).

The compounds of invention have been named according to the standards used in the program ACD/Name Batch from Advanced Chemistry Development Inc., ACD/Labs (7.00 Release). Product version: 7.10, build: 15 Sep 2003NMR, HPLC and MS data provided in the examples described below are registered on:

**NMR:** Bruker DPX-300 (300 MHz) or Varian Gemini 2000 (300 MHz) using residual signal of deuterated solvent as internal reference.

**HPLC:** Method 1 - Waters Alliance 2695, column Waters XBridge C8 3.5 µm 4.6x50 mm, conditions: solvent A (H₂O with 0.1% TFA), solvent B (ACN with 0.05% TFA), gradient 5% B to 100% B over 8 min, UV detection with PDA Water 996 (230-400 nm).

**LCMS: Method 2** - Agilent 1100 Series LC/MSD, column Phenomenex Gemini-NX C18 5 µm 150 x 4.6 mm, with mobile phase 80% ACN, 15% H₂O, 5% buffer (3:1 MeOH/H₂O, 315 mg HCO₂NH₄, 1 mL AcOH) and MS detection (ESI method).

**UPLC:** Method 3 - Waters Acquity, column Waters Acquity UPLC BEH C18 1.7 µm 2.1x50 mm, conditions: solvent A (10 mM ammonium acetate in water + 5% ACN), solvent B (ACN), UV detection (PDA, 230-400 nm) and MS detection (SQ detector, positive and negative ESI modes, cone voltage 30 V). Gradient 5% B to 100% B over 3 min or gradient 40% B to 100% B over 3 min.

**MD Autoprep:** preparative HPLC purifications are performed with a mass directed auto-purification Fractionlynx from Waters equipped with a Sunfire Prep C18 OBD column 19x100 mm or 30x100 mm 5 µm, unless otherwise reported. All HPLC purifications were performed with a gradient of ACN/H₂O or ACN/H₂O/HCOOH (0.1%).

### EXAMPLES AND GENERAL PROCEDURES

### General procedures

### General Procedure A: Reductive amination of aldehydes/ketones to sec-amines using triacetoxyborohydride

To a solution of aldehyde/ketone (1 equiv.) and amine (1-2.5 equiv.) in anhydrous DCE (~0.1M), sodium triacetoxyborohydride (1.5-3.0 equiv.) and with or without acetic acid (2-3 equiv.) were added sequentially and the resulting reaction mixture was stirred under a nitrogen atmosphere at RT until completion. The crude reaction mixture was diluted with EtOAc and the organics washed with a NaHCO₃ (aq.) solution or 1 M NaOH followed by distilled water. The organic layer was separated, dried (MgSO₄) and concentrated under reduced pressure to provide the crude product, which was used without purification to the next step or purified by MD Autoprep or by silica-gel column chromatography using increasing gradient of EtOAc or/and MeOH in hexane or DCM as eluant to afford the secondary amine.

### General Procedure B: Reductive amination of aldehydes/ketones to sec-amines using p-toluenesulfonic acid and Dean-Stark apparatus

To a solution of aldehyde/ketone (1 equiv.) and amine (1-4 equiv.) in toluene (2-5 M) was added p-toluenesulfonic acid (0.1 equiv.) and resulting mixture was heated to reflux under Dean-Stark conditions. After the formation of the imine intermediate (TLC/LCMS), solvent was removed under reduced pressure and the resulting residue was dissolved in MeOH or DCM. Sodium borohydride or sodium triacetoxyborohydride was added and reaction mixture was stirred at RT under a nitrogen atmosphere. Upon completion of the reaction (LCMS), the solvent was removed under reduced pressure and the resulting residue was diluted with EtOAc or DCM and the organics washed with saturated NH₄Cl (aq.) solution or 1 M NaOH followed by distilled water. The organic layer was separated, dried (MgSO₄) and concentrated under reduced pressure to give the crude product, which was used without purification to the next step or purified by MD Autoprep or by flash chromatography (silica-gel, EtOAc) to afford the secondary amine.

### General Procedure C: Preparation of t-amides from acid chlorides

Triethylamine or diisopropylethylamine (2 equiv.) was added to a solution of sec-amine (1 equiv.) in anhydrous DCM (0.1-0.3M) and the resulting solution was stirred at RT under a nitrogen atmosphere for 5 min. Acid chloride (1.5-2.5 equiv.) was added and the reaction mixture was stirred until completion (LCMS). The reaction mixture was diluted with EtOAc or diethyl ether and washed thoroughly with NaHCO₃ (aq.) solution followed by distilled water. The organic layer was separated, dried (MgSO₄) and concentrated under reduced pressure. The crude material was purified using either PTLC or silica-gel flash chromatography using increasing gradient of EtOAc or/and MeOH in hexane or DCM as eluant.

### General Procedure D: Preparation of tertiary-amides from acids

To a solution of *sec*-amine (1 equiv.) with or without triethylamine (1-1.5 equiv.) and acid (1-6 equiv.) in anhydrous DCE (0.22-0.28M) at 0°C or RT, was added 1-propylphosphonic acid cyclic anhydride (T3P) (1-4 equiv.) under a nitrogen atmosphere. The mixture was allowed to stir at RT or reflux until reaction completion (LCMS). The reaction was quenched by the addition of 1 M NaOH and extracted with EtOAc or DCM. Organic layer was washed sequentially with 1 M NaOH and then with 1 M HCl, filtered, dried (MgSO₄) and concentrated under reduced pressure, to obtain a yellow oil, which was purified using PTLC and/or silica-gel flash chromatography or was used without purification to the next step or by MD Autoprep.

### General procedure E: Formation of sulfonamides

To a mixture of potassium carbonate (2 equiv.) in anhydrous acetonitrile (0.1-0.4 M) were added the *sec*-amine (1 equiv.) and the sulfonyl chloride (1.5-2.5 equiv.) sequentially under nitrogen atmosphere. The resulting mixture was heated to reflux until reaction completion (LCMS). Solvent was removed under reduced pressure and the reaction mixture was diluted with EtOAc and washed thoroughly with NaHCO₃ (aq.) solution followed by distilled water. The organic layer was separated, dried (MgSO₄) and concentrated under reduced pressure. The crude material was purified using either PTLC or silica-gel flash chromatography using increasing gradient of EtOAc or/and MeOH in hexane or DCM as eluant.

### General Procedure F: Deprotection of acetal

A solution of acetal (1 equiv.) in a mixture of CH₃CN/H₂O/TFA (1/1/0.04) (0.1-0.5 M) was stirred at RT for 18 h. The reaction was diluted with DCM and was then washed sequentially with 1 M HCl, 1 M NaOH and brine. The organic was dried (MgSO₄), filtered and concentrated under reduced pressure and the crude material purified by column chromatography or used directly to the next step without further purification.

### General Procedure G: Swern-oxidation of alcohols to aldehydes

Oxalyl chloride (1.5 equiv.) was added slowly to a solution of anhydrous dimethylsulfoxide (3.0 equiv.) in anhydrous DCM (0.17-2.0M) at -78 °C under a nitrogen atmosphere and stirred for 30 min. To the resulting mixture, was added the alcohol (1 equiv.) dissolved in anhydrous DCM (0.2-0.4M) and the reaction mixture was stirred at -78 °C for 45 min. Anhydrous triethylamine (6 equiv.) was added dropwise and reaction was stirred at -78 °C for 30 min and then at RT for 30 min. The DCM was removed under reduced pressure and residue was dissolved in diethyl ether and washed with NH₄Cl (aq.) solution. The organic layer was separated and the aqueous layer was again extracted with diethyl ether. The organic layers were combined, dried (MgSO₄), filtered and concentrated under reduced pressure to give the crude product, which was purified by column chromatography.

### General Procedure H: Alkylation of sec-amines

To the solution of *sec*-amine (1 equiv.) in anhydrous DCM (~0.14M) was added sequentially DIPEA (1.2-1.5 equiv.) and chloroacetylchloride (1.1-.1.4 equiv.) and the resulting mixture was stirred at RT under a nitrogen atmosphere until completion. The reaction was quenched with water and extracted with DCM. The combined organics were dried (MgSO₄) and concentrated under reduced pressure. Crude material was purified using PTLC and/or silica-gel column chromatography.

### General Procedure I: Preparation of t-amides

To a solution of amide (1 equiv.) and amine (2-2.5 equiv.) in anhydrous DMF (0.11-0.12M) was added Na₂CO₃ (2-2.5 equiv.) and sodium iodide (1-1.1 equiv.) sequentially and the resulting mixture was stirred at 65°C overnight. The reaction mixture was cooled to RT and diluted with diethyl ether and washed with water. Organic layer was separated, dried (MgSO₄) and concentrated under reduced pressure. Crude material was purified using silica-gel column chromatography.

### General Procedure J: Preparation of acid chlorides

A suspension of acid in thionyl chloride (0.4-0.6M) was stirred at RT overnight or refluxed for 1 h. Excess thionyl chloride was removed under reduced pressure at RT and traces were removed under high vacuum. A clear oil of acid chloride was obtained, which was used without further purification.

### Intermediate a: 1-cyclopropyl-1-(4-methoxyphenyl)-N-(pyridin-2-ylmethyl)methanamine

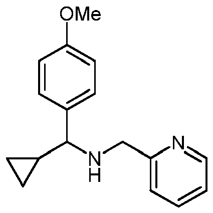

Cyclopropyl-4-methoxyphenyl ketone (1 g, 5.67 mmol) and 2-(aminomethyl) pyridine (1.18 ml, 11.35 mmol), were reacted according to General Procedure A to afford the title compound as a pale yellow oil. ¹H NMR (CDCl₃) δ 8.56-8.55 (m, 1 H), 7.63-7.57 (dt, *J* = 1.5, 7.8 Hz, 1 H), 7.33-7.29 (m, 2H), 7.19-7.12 (m, 2H), 6.91-6.87 (m, 2H), 3.81 (s, 3H), 3.81- 3.68 (m, 2H), 2.77 (d, *J* = 9.0 Hz, 1 H), 1.65 (bs, 1 H), 1.19-1.16 (m, 1 H), 0.66-0.56 (m, 1 H), 0.43-0.27 (m, 2H), 0.21-0.14 (m, 1 H). MS (ES⁺) m/z 269.2 (M + H)⁺.

### Intermediate b:

### 1-cyclopropyl-1-(4-chlorophenyl)-N-(pyridin-2-ylmethyl)methanamine

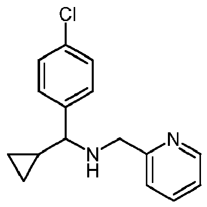

4-Chlorophenyl cyclopropylketone (500 mg, 2.77 mmol) and 2-(aminomethyl) pyridine (1.151 ml, 11.17 mmol) were reacted according to General Procedure B to afford title compound as a pale yellow oil. ¹H NMR (CDCl₃) δ 8.57 (m, 1 H), 7.63-7.57 (m, 1 H), 7.35-7.29 (m, 4H), 7.17-7.12 (m, 2H), 3.79-3.66 (m, 2H), 2.79 (d, *J* = 8.91 Hz, 1 H), 1.14-1.05 (m, 1 H), 0.67-0.59 (m, 1 H), 0.43-0.29 (m, 2H), 0.22-0.15 (m, 1 H). LCMS (Method 2) m/z 273.2 (M + H)⁺.

### Intermediate e: 1-cyclopropyl-1-phenyl-N-(pyridin-2-ylmethyl)methanamine

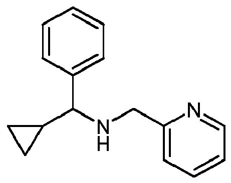

1-Cyclopropyl-1-phenylmethanamine hydrochloride (300 mg, 1.63 mmol) and 2-pyridinecarboxaldehyde (175 mg, 1.63 mmol) were reacted according to General Procedure A (however triethylamine (0.23 ml, 1.63 mmol) was stirred for 10 min with the amine hydrochloride prior to addition of aldehyde) to give the title compound as a yellow oil (445.2 mg, quantitative). HPLC (Method 1) Rt 1.90 min (Purity: 87.8%). UPLC/MS (Method 3) 239.1 (M+H)⁺.

### Intermediate h: 2-methyl-1-phenyl-N-(pyridin-2-ylmethyl)propan-1-amine

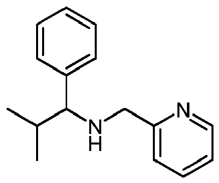

2-Methyl-1-phenylpropan-1-amine (150 mg, 1.01 mmol) and 2-pyridinecarboxaldehyde (108 mg, 1.01 mmol), were reacted according to General Procedure A to give the title compound as a yellow oil (249 mg, quantitative). ¹H NMR (CDCl₃) δ 8.57-8.56 (m, 1 H), 7.61 (td, *J* = 7.7, 1.8 Hz, 1 H), 7.37-7.14 (m, 8H), 3.75 (d, *J* = 14.2 Hz, 1 H), 3.64 (d, *J* = 14.2 Hz, 1 H), 3.60 (d, *J* = 7.1 Hz, 1 H), 2.02-1.89 (m, 1 H), 1.03 (d, *J* = 6.7 Hz, 3H), 0.77 (d, *J* = 6.7 Hz, 3H). HPLC (Method 1) Rt 2.10 min (Purity: 92.6%). UPLC/MS (Method 3) 241.1 (M+H)⁺.

### Intermediate i:

### 1-cyclopentyl-1-(4-fluorophenyl)-N-(pyridin-2-ylmethyl)methanamine

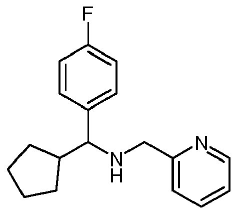

Cyclopentyl(4-fluorophenyl)methanamine hydrochloride (200 mg, 0.87 mmol) and 2-pyridinecarboxaldehyde (93 mg, 0.87 mmol) were reacted according to General Procedure A (however triethylamine (0.12 ml, 0.87 mmol) was stirred for 10 min with the amine hydrochloride prior to addition of aldehyde) to give the title compound as a yellow oil (251 mg, quantitative). ¹H NMR (CDCl₃) δ 8.58-8.56 (m, 1 H), 7.61 (td, *J* = 7.7, 1.8 Hz, 1 H), 7.33-7.25 (m, 2H), 7.17-6.99 (m, 4H), 3.69 (d, *J* = 14.2 Hz, 1 H), 3.59 (d, *J* = 14.2 Hz, 1 H), 3.32 (d, *J* = 8.3 Hz, 1 H), 2.18-1.22 (m, 8H), 1.10-1.01 (m, 1 H). HPLC (Method 1) Rt 2.72 min (Purity: 95.1%). UPLC/MS (Method 3) 285.2 (M+H)⁺.

### Intermediate j: 2-ethyl-1-phenyl-N-(pyridin-2-ylmethyl)butan-1-amine

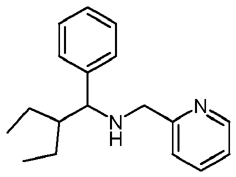

2-Ethyl-1-phenylbutan-1-amine (150 mg, 0.85 mmol) and 2-pyridinecarboxaldehyde (91 mg, 0.85 mmol) were reacted according to General Procedure A to give the title compound as a yellow oil (223 mg, 98%). HPLC (Method 1) Rt 2.84 min (Purity: 94.1 %). UPLC/MS (Method 3) 269.2 (M+H)⁺.

### Intermediate k: 1-cyclopentyl-1-phenyl-N-(pyridin-2-ylmethyl)methanamine

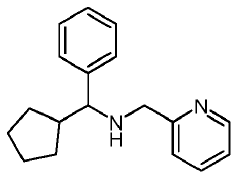

Cyclopentyl(phenyl)methanamine hydrochloride (200 mg, 0.94 mmol) and 2-pyridinecarboxaldehyde (101 mg, 0.94 mmol) were reacted according to General Procedure (however triethylamine (0.13 ml, 0.94 mmol) was stirred for 10 min with the amine hydrochloride prior to addition of aldehyde) to give the title compound as a yellow oil (258 mg, quantitative). HPLC (Method 1) Rt 2.60 min (Purity: 94.0%). UPLC/MS (Method 3) 267.2 (M+H)⁺.

### Intermediate I:

### 1-(2-methyl-2,3-dihydro-1-benzofuran-5-yl)-N-(pyridin-2-ylmethyl)propan-1-amine

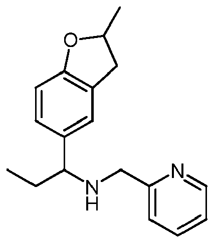

1-(2-Methyl-2,3-dihydro-benzofuran-5-yl)-propylamine (178 mg, 0.93 mmol) and 2-pyridinecarboxaldehyde (100 mg, 0.93 mmol), were reacted according to General Procedure A to give the title compound as a yellow oil (190 mg, 72%). ¹H NMR (d₆-DMSO) δ 8.46-8.44 (m, 1 H), 7.75-7.69 (m, 1 H), 7.40-7.38 (m, 1 H), 7.23-7.19 (m, 1 H), 7.13 (s, 1 H), 6.98-6.96 (m, 1 H), 6.65-6.62 (m, 1 H), 4.93-4.82 (m, 1 H), 3.63-3.51 (m, 2H), 3.30-3.24 (m, 1 H), 2.80-2.71 (m, 1 H), 2.64-2.52 (m, 1 H), 1.74-1.65 (m, 1 H), 1.55-1.46 (m, 1 H), 1.39-1.37 (d, *J* = 6.2 Hz, 3H), 0.77-0.72 (t, *J* = 7.4 Hz, 3H). HPLC (Method 1) Rt 7.76 min (Purity: 99.4%). UPLC/MS (Method 3) 283.1 (M+H)⁺.

### Intermediate v:

### 1-(2,3-dihydro-1H-inden-5-yl)-N-[2-(3,6-dimethylpyrazin-2-yloxy)ethyl]ethanamine

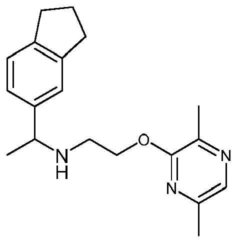

1-Indan-5-yl-ethylamine (107 mg, 0.66 mmol) and intermediate **mm** (110 mg, 0.73 mmol), were reacted according to General Procedure A to give the title compound as clear oil. ¹H NMR (CDCl₃) δ 7.83 (s, 1 H), 7.20-7.07 (m, 3H), 4.44-7.32 (m, 2H), 3.82 (q, *J* = 6.6 Hz, 1 H), 2.96- 2.82 (m, 6H), 2.42 (s, 3H), 2.36 (s, 3H), 2.12-2.02 (m, 2H), 1.37 (d, *J* = 6.6 Hz, 3H). -LCMS (Method 2) m/z 312.5 (M + H)⁺.

### Intermediate w: (N-[(6-methoxypyridin-3-yl)methyl]-1-phenylethanamine)

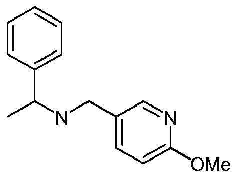

1-Phenylethanamine (242 mg, 2.0 mmol) and 6-methoxypyridine-3-carbaldehyde (242 mg, 1.76 mmol), were reacted according to General Procedure A to give the title compound as pale yellow oil. ¹H NMR (CDCl₃) δ 8.01 (d, *J* =2.1 Hz , 1 H), 7.60 (dd, *J* = 2.4, 8.4 Hz, 1 H), 7.35-7.22 (m, 5H), 6.70 (d, *J* = 8.7 Hz, 1 H), 3.92 (s, 3H), 3.79 (q, *J* = 6.6 Hz, 1 H), 3.54 (d, *J* = 3.9 Hz, 2H), 1.36 (d, *J* = 6.6 Hz, 3H). LCMS (Method 2) m/z 243.3 (M+H)⁺.

### Intermediate x:

### 1-(2,3-dihydro-1H-inden-5-yl)-N-[2-(6-chloropyridin-2-yloxy)ethyl]ethanamine

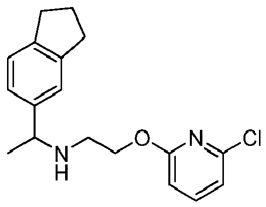

5-Acetylindane (106 mg, 0.66 mmol) and intermediate **kk** were reacted as described according to General Procedure A to afford the titled compound as a yellow oil. ¹HNMR (CDCl₃) δ 7.51 (t, *J* = 7.8 Hz, 1 H), 7.21-7.08 (m, 3H), 6.88 (d, *J* = 7.8 Hz, 1 H), 6.65 (d, *J* = 7.8 Hz, 1 H), 4.43-4.31 (m, 2H), 3.82 (q, *J* = 6.6 Hz, 1 H), 2.94-2.79 (m, 6H), 2.12-2.02 (m, 2H), 1.76 (s, 1 H), 1.37 (d, *J* = 6.6 Hz, 3H). LCMS (Method 2) m/z 317.3(M+H)⁺.

### Intermediate y:

### 1-(2,3-dihydro-1H-inden-5-yl)-N-[2-(pyridin-2-yloxy)ethyl]ethanamine

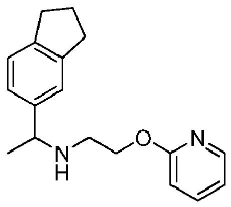

5-Acetylindane (214 mg, 1.34 mmol) and 2-(pyridin-2-yloxy)ethanamine (prepared according to the procedure outlined in Tetrahedron 1988, 44(1), 91-100) (448 mg, 3.24 mmol), were reacted as described according to General Procedure A to afford the titled compound (275 mg, 73%) as a yellow oil. ¹HNMR (CDCl₃) δ 8.14-8.12 (m, 1 H), 7.58-7.53 (m, 1 H), 7.22-7.08 (m, 3H), 6.87-6.83 (m, 1 H), 6.75-6.72 (m, 1 H), 4.43-4.30 (m, 2H), 3.82 (q, *J* = 6.6 Hz, 1 H), 2.95-2.80 (m, 6H), 2.12-2.02 (m, 2H), 1.76 (s, 1 H), 1.37 (d, *J* = 6.6 Hz, 3H). LCMS (Method 2) m/z 283.3 (M + H)⁺.

### Intermediate bb: 1-(2,3-dihydro-1H-inden-5-yl)-N-[2-(6-methylpyridin-2-yloxy)ethyl]ethanamine

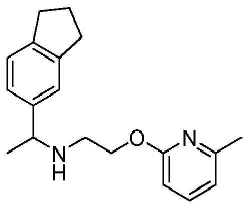

5-Acetylindane (214 mg, 1.34 mmol) and 2-[(6-methylpyridin-2-yl)oxy]ethanamine (prepared according to the procedure outlined in US3535328 (A), 761 mg, 5.0 mmol), were reacted as described according to General Procedure A to afford the titled compound as a yellow oil. ¹HNMR(CDCl₃)δ 7.44 (dd, *J* = 8.1, 7.2 Hz, 1 H), 7.22-7.08 (m, 3H), 6.70 (d, *J* = 7.2 Hz, 1 H), 6.52 (d, *J* = 8.1 Hz, 1 H), 4.41-4.28 (m, 2H), 3.82 (q, *J* = 6.6 Hz, 1 H), 2.94-2.79 (m, 6H), 2.42 (s, 3H), 2.12-2.02 (m, 2H), 1.88 (s, 1 H), 1.37 (d, *J* = 6.6 Hz, 3H). LCMS (Method 2) m/z 297.2 (M + H)⁺.

### Intermediate cc: 1-(2,2-dimethyl-1,3-benzoxathiol-5-yl)-N-(pyridin-2-ylmethyl)ethanamine

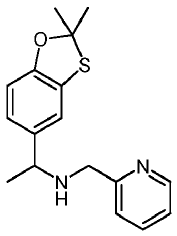

1-(2,2-Dimethyl-1,3-benzoxathiol-5-yl)ethanone (prepared according to procedure outlined in the Journal of Heterocyclic Chemistry, 1984, 21(2), 573-6 and 1982, 19(1), 135-9) (126 mg; 0.60 mmol), 2-(aminomethyl)pyridine (262 mg, 2.42 mmol) and p-toluenesulfonic acid monohydrate (12 mg, 0.06 mmol) were reacted according to General Procedure B, to give the title compound as a pale yellow oil (156 mg, 86%). ¹H NMR (CDCl₃) δ 8.54 (ddd, *J* = 4.9, 1.7, 0.9 Hz, 1 H), 7.60 (td, *J* = 7.7, 1.7

Hz, 1 H), 7.21-7.13 (m, 3H), 6.95 (dd, *J* = 8.2, 1.9 Hz, 1 H), 6.69 (d, *J* = 8.2 Hz, 1 H), 3.82-3.71 (m, 3H), 2.47 (brs, 1H), 1.84 (s, 6H), 1.39 (d, *J* = 6.6 Hz, 3H). HPLC (Method 1) Rt 2.84 min (Purity: 98.6%). UPLC/MS (Method 3) 301.1 (M+H)⁺.

### Intermediate ff: 1-[2-(methoxymethyl)phenyl]-N-(pyridin-2-ylmethyl)ethanamine

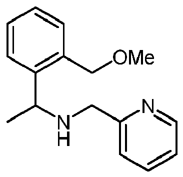

1-[2-(Methoxymethyl)phenyl]ethanone (prepared according to procedure outlined in the Journal of Organic Chemistry, 1970, 35(8), 2532-8) (205 mg, 1.25 mmol), 2-(aminomethyl)pyridine (135 mg, 1.25 mmol) and *p*-toluenesulfonic acid monohydrate (24 mg, 0.12 mmol), were reacted according to General Procedure B to give the title compound as a yellow oil (276 mg, 86%). ¹H NMR (CDCl₃) δ 8.58-8.52 (m, 1 H), 7.66 (d, *J* = 7.8, 1 H), 7.61 (td, *J* = 7.7, 1.8, 1 H), 7.39-7.28 (m, 2H), 7.19 (ddd, *J* = 12.4, 8.2, 3.1, 3H), 4.45 (s, 2H), 4.24 (q, *J* = 6.5, 1 H), 3.78 (s, 2H), 3.33 (s, 3H), 1.44 (d, *J* = 6.5, 3H). HPLC (Method 1) Rt 2.34 min (Purity: 96.2%). UPLC/MS (Method 3) 257.1 (M+H)⁺.

### Intermediate gg: 1-(2,3-dihydro-1H-inden-5-yl)-N-(pyridin-2-ylmethyl)ethanamine

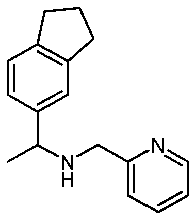

5-Acetylindane (1.0 g, 6.24 mmol) and 2-(aminomethyl)pyridine (639 µl, 6.24 mmol) were reacted according to General Procedure B to give the title compound as a yellow oil. ¹H NMR (d₆-DMSO) δ 8.47-8.45 (m, 1 H), 7.76-7.70 (m, 1 H), 7.41-7.39 (m, 1 H), 7.24-7.06 (m, 4H), 3.71-3.65 (m, 1 H), 3.59 (s, 2H), 2.85-2.79 (m, 4H), 2.02-1.98 (m, 2H), 1.26 (d, *J* = 6.6Hz, 3H). HPLC (Method 1) Rt 2.39 min (Purity: 91.7%). UPLC/MS (Method 3) 253.1 (M+H)⁺.

### Intermediate hh: 3-(4-Fluorophenyl)butanal

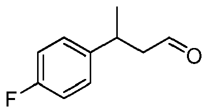

3-(4-Fluorophenyl)butan-1-ol (2.01 g, 11.94 mmol), oxalyl chloride (1.60 mL, 18.34 mmol), DMSO (2.60 ml, 36.61 mmol) and TEA (10.00 ml, 74.46 mmol), were reacted according to General Procedure G to afford the titled compound (1.69 g, 85%) as a yellow oil. ¹HNMR (CDCl₃) δ 9.69 (t, *J* = 1.8Hz, 1H), 7.21-7.14 (m, 2H), 7.02-6.95 (m, 2H), 3.36 (sextet, *J* = 7.2 Hz, 1 H), 2.77-2.60 (m, 2H), 1.29 (d, *J* = 7.2 Hz, 3H).

### Intermediate ii: 3-(4-fluorophenyl)-N-[1-(2,3-dihydro-1H-inden-5-yl)ethyl]butanamine

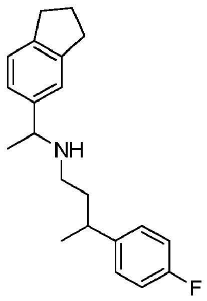

1-Indan-5-yl-ethylamine (269 mg, 1.67 mmol) and intermediate **hh** (280 mg, 1.69 mmol), were reacted according to General Procedure A to afford the titled compound as a yellow oil. ¹HNMR (CDCl₃) δ 7.17-6.89 (m, 7H), 3.70-3.60 (m, 1 H), 2.89 (t, *J* = 7.5Hz, 4H), 2.81-2.69 (m, 1 H), 2.50-2.15 (m, 3H), 2.13-2.03 (m, 2H), 1.80-1.62 (m, 2H), 1.35-1.15 (m, 6H). LCMS (Method 2) m/z 312.3 (M + H)⁺.

### Intermediate ii: N-[1-(2,3-dihydro-1H-inden-5-yl)ethyl]-3-(4-fluorophenyl)-N-(chloroacetyl)butanamine

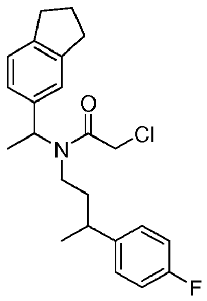

Intermediate ii (215 mg, 0.69 mmol), chloroacetyl chloride (66 µl, 0.83 mmol) and DIPEA (142 µl, 0.83 mmol) were reacted according to General Procedure H to afford the titled compound (223 mg, 83%) as a yellow oil. ¹H NMR (CDCl₃) δ 7.20-6.89 (m, 7H), 5.95-4.97 (m, 1 H), 4.21-4.09 (m, 1 H), 4.00-3.80 (m, 1 H), 3.30-2.18 (m, 7H), 2.15-2.02 (m, 2H), 1.85-1.60 (m, 2H), 1.58-1.41 (m, 3H), 1.15-0.98 (m, 3H). LCMS (Method 2) m/z 388.3 (M + H)⁺.

### Intermediate kk: 2-[(6-chloropyridin-2-yl)oxy]ethanamine

To a solution of ethanolamine (1.00 g, 16.4 mmol) in anhydrous 1,4-dioxane (20 ml) at RT was added NaH (60% in oil) (655 mg, 16.4 mmol) portion-wise. The reaction mixture was heated to reflux (100 °C) for 30 mins, then cooled to RT and 2,6-dichloropyridine (2.423 g, 16.4 mmol) was then added. The reaction mixture was again heated to reflux (100 °C) for 3 h, then cooled to RT, quenched with water and extracted with diethyl ether. The combined organic extracts were dried (MgSO₄), filtered and concentrated under reduced pressure to afford crude 2-[(6-chloropyridin-2-yl)oxy]ethanamine which was purified by flash chromatography eluting initially with1:1 EtOAc:hexane to remove any remaining 2,6-dichloropyridine then eluted with 1:9 MeOH:DCM to obtain pure 2-[(6-chloropyridin-2-yl)oxy]ethanamine as a pale yellow oil (1.846 g, 65% yield). ¹H NMR (CDCl₃) δ 7.50 (t, *J* = 7.8 Hz, 1 H), 6.88 (d, *J* = 7.8 Hz, 1H),6.66(d, *J* = 7.8 Hz, 1H), 4.31 (t, *J* = 5.4 Hz, 2H), 3.05 (t, *J* = 5.4 Hz, 2H), 1.35 (br s, 2H).

### Intermediate II: 2-[(3,6-dimethylpyrazin-2-yl)oxylethanol-

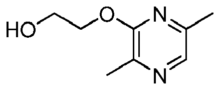

To a solution of ethylene glycol (1.00 g, 16.11 mmol) in anhydrous DMF (10 ml) at RT was added NaH (60% in oil) (222 mg, 6.04 mmol) portion-wise. The reaction mixture was stirred for 5 min before addition of 3-chloro-2,5-dimethylpyrazine (574 mg, 4.03 mmol). The mixture was heated to 50 °C for 20 h, then cooled to RT, quenched with water and extracted with. The combined organic extracts were dried (MgSO₄), filtered and concentrated under reduced pressure. The crude material was purified by flash chromatography eluting with 1:4 EtOAc:hexane to afford the titled compound as a pale yellow solid. ¹H NMR (CDCl₃) δ 7.87 (s, 1 H), 4.50-4.47 (m, 2H), 3.98-3.93 (m, 2H), 3.44 (t, *J* = 5.7 Hz, 1 H), 2.42 (s, 3H), 2.38 (s, 3H). LCMS (Method 2) m/z 169.3 (M+H)⁺.

### Intermediate mm: [(3,6-dimethylpyrazin-2-yl)oxy]acetaldehyde-

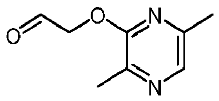

Intermediate II (150 mg, 0.89 mmol) was reacted according to General Procedure G and the crude aldehyde was used as such without further purification. LCMS (Method 2) m/z 167.3 (M + H)⁺.

### Intermediate nn:3-(4-fluorophenyl)butanoyl chloride

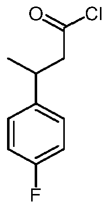

3-(4-Fluorophenyl)butanoic acid (2 g, 9.97 mmol) and thionyl chloride (20 ml) were reacted according to General Procedure J to afford the titled compound (quantitative conversion) as a clear oil, which was used without further purification.

### Intermediate qq: 2-(4-fluorophenyl)cyclopropanecarbonyl chloride

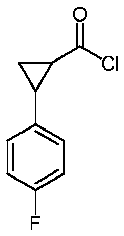

2-(4-fluorophenyl)cyclopropanecarboxylic acid (2 g, 10.07 mmol) and thionyl chloride (20 ml) were reacted according to General Procedure J to afford the titled compound as a clear oil, which was used without further purification.

### Intermediate tt: 1-(4-chlorophenyl)cyclobutanecarbonyl chloride

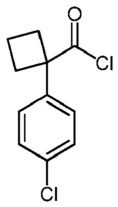

1-(4-chlorophenyl)cyclobutanecarboxylic acid (500 mg, 2.18mmol) and thionyl chloride (5 ml) were reacted according to General Procedure J to afford the titled compound as a clear oil, which was used without further purification.

### Intermediate uu: cyclopropyl(2,2-dimethyl-1,3-benzoxathiol-5-yl)methanone

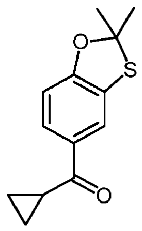

To a solution of cyclopropancarbonyl chloride (2.6 g, 25.26 mmol) in anhydrous DCM (20 ml) at -10 °C under a nitrogen atmosphere was added AlCl₃ (1.7 g, 12.63 mmol) portion-wise and the mixture was stirred until homogeneous. The solution was then added to a solution of 2,2-dimethyl-1,3-benzoxathiole (prepared according to procedure outlined in the Journal of Heterocyclic Chemistry, 1984, 21 (2), 573-6 and 1982, 19(1), 135-9) (2.1 g, 12.63 mmol) in anhydrous DCM (20 ml) at -10 °C. After stirring for 30 min at -10 °C, the mixture was poured into 5M NaOH. The aqueous phase was extracted twice with DCM. The combined organics were dried (MgSO₄) filtered and concentrated under reduced pressure to give an oil. The oil was purified by column chromatography using 5% to 20% EtOAc in *n*-heptane to give title compound as a white solid (542mg, 18%). ¹H NMR (CDCl₃) δ 7.80 (d, *J* = 1.9 Hz, 1 H), 7.74 (dd, *J* = 8.3, 1.9 Hz, 1 H), 6.82 (d, *J* = 8.3 Hz, 1 H), 2.60-2.53 (m, 1 H), 1.86 (s, 6H), 1.22-1.17 (m, 2H), 1.02-0.96 (m, 2H). HPLC (Method 1) Rt 4.15 min (Purity: 86.9%).

### Intermediate vv: cyclopropyl(2,2-dimethyl-3,3-dioxido-1,3-benzoxathiol-5-yl)methanone

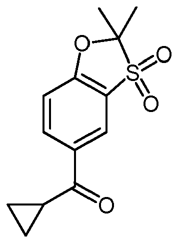

To a solution of Intermediate uu (542 mg, 2.31 mmol) in glacial AcOH (10 ml) at 0 °C was added H₂O₂ (2.62 ml, 23.13 mmol). After stirring for 30 min at RT, H₂O₂ (5.2 ml, 46.26 mmol) was added and the reaction was stirred at RT for 18 h. DCM was added and the organic phase was washed with water, 1 N NaOH, saturated solution of sodium thiosulfate, dried (MgSO₄), filtered and concentrated under reduced pressure to give the title compound as a white solid (625 mg, quantitative). ¹H NMR (CDCl₃) δ 8.04 (d, *J* = 1.9 Hz, 1 H), 7.89 (dd, *J* = 8.7, 1.9 Hz, 1 H), 6.76 (d, *J* = 8.7 Hz, 1 H), 2.30-2.22 (m, 1 H), 1.44 (s, 6H), 0.95-0.90 (m, 2H), 0.78-0.72 (m, 2H). HPLC (max plot) 93.7%; Rt 3.40min. UPLC/MS (Method 3) 267.0 (M+H)⁺.

### Intermediate ww: (E)-cyclopropyl(2,2-dimethyl-3,3-dioxido-1,3-benzoxathiol-5-yl)methanone oxime

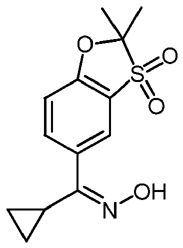

A solution of Intermediate vv (625 mg, 2.35 mmol) in hydroxylamine (10 ml) and EtOH (10 ml) was heated at 90 °C for 2 days. The mixture was then cooled to RT, water was added and the mixture was extracted four times with DCM. The combined organics were washed with water, dried (MgSO₄), filtered and concentrated under reduced pressure. *n*-Heptane was added and the solid was triturated, sonicated and filtered to give the title compound as a white solid (578 mg, 88%). UPLC/MS (Method 3) 282.0 (M+H)⁺.

### Intermediate xx: 1-cyclopropyl-1-(2,2-dimethyl-3,3-dioxido-1,3-benzoxathiol-5-yl)methanamine

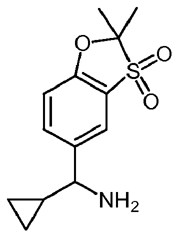

A solution of Intermediate ww (578 mg, 2.05 mmol) in MeOH (20 ml) was hydrogenated in H-Cube® (thalesnano)with Raney nickel 1ml/min 60 bars at 90 °C under recycling condition for 3 h. The mixture was then concentrated under reduced pressure and the titled compound was isolated as HCl salt (white solid) after exposure to HCl (1.25N in diethyl ether) (380 mg, 61%). HPLC (max plot) 90.5%; Rt 3.40min. UPLC/MS (Method 3) 251.0 (M+H)⁺.

### Intermediate yy: 1-cyclopropyl-1-(2,2-dimethyl-3,3-dioxido-1,3-benzoxathiol-5-yl)-N(pyridin-2-ylmethyl)methanamine

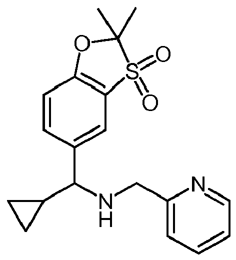

Intermediate xx (195 mg, 0.64 mmol) and 2-pyridinecarboxaldehyde (69 mg; 0.64 mmol) were reacted according to General Procedure A (however triethylamine (0.089 ml, 0.64 mmol) was stirred for 10 min with the amine hydrochloride prior to addition of aldehyde) to give the title compound as a yellow oil (180 mg, 78%). ¹H NMR (CDCl₃) δ 8.57-8.55 (m, 1 H), 7.79-7.62 (m, 3H), 7.19-6.99 (m, 3H), 3.89-3.70 (m, 2H), 2.88 (d, *J* = 9.0 Hz, 1 H), 1.75 (s, 6H), 1.19 (brs, 1 H), 0.75-0.17 (m, 4H). HPLC (Method 1) Rt 2.26 min (Purity: 86.3%). UPLC/MS (Method 3) 359.1 (M+H)⁺.

### Intermediate zz: 1-(4-methyl-4H-1,2,4-triazol-3-yl-N-(pyridin-2-ylmethyl)ethanamine

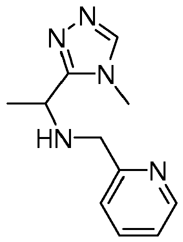

1-(4-Methyl-4H-[1,2,4]triazol-3-yl)-ethylamine dihydrochloride (140 mg; 0.70 mmol) and 2-pyridinecarboxaldehyde (83 mg, 0.77 mmol) were reacted according to General Procedure A to give the title compound as an oil. UPLC/MS (Method 3) 218.1 (M+H)⁺.

### Intermediate aaa: 1-(3-ethyl-1,2,4-oxadiazol-5-yl)-N-(pyridin-2-ylmethyl)ethanamine

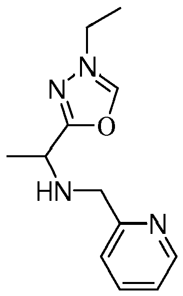

1-(3-ethyl-1,2,4-oxadiazol-5-yl)ethanamine (180 mg, 0.71 mmol) and 2-pyridinecarboxaldehyde (83 mg, 0.78 mmol), were reacted according to General Procedure A to give the title compound as an oil (87 mg, 53%). ¹H NMR (d₆-DMSO) δ 8.56-8.54 (m, 1 H), 7.64 (dt, *J*= 1.8, 7.7Hz, 1 H), 7.30-7.28 (m, 1 H), 7.19-7.15 (m, 1 H), 4.19 (q, *J* = 6.9Hz, 1 H), 4.00-3.88 (m, 2H), 3.10-2.70 (br s, 1 H) 2.75 (q, *J* = 7.6Hz, 2H), 1.58 (d, *J* = 6.9Hz, 3H), 1.32 (t, *J* = 7.6Hz, 3H). UPLC/MS (Method 3) 233.1 (M+H)⁺.

### Intermediate bbb : N-(4-chlorophenyl)(cyclopropyl)methyl]-8-methyl-8-azabicyclo[3.2.1]octan-3-amine

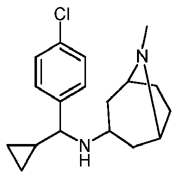

To a solution of 4-chlorobenzyldehyde (400 mg, 2.84 mmol) and 8-methyl-8-azabicyclo[3.2.1]octan-3-amine (413 mg, 2.98 mmol) in anhydrous THF (6 ml) was added magnesium sulphate (685 mg, 5.69 mmol) and the resulting reaction mixture was stirred at RT for 4 hours. Reaction mixture was filtered and filtrate was concentrated under reduced pressure to obtain the imine intermediate as light brown oil (748 mg). In a second reaction vessel a mixture of cyclopropyl bromide (5.69 mmol) was dissolved in dry diethyl ether (6 ml) under nitrogen atmosphere at -78 °C and treated with *tert-*BuLi. After 10 minutes, cooling was removed and the mixture was stirred at room temperature for 1 hr. The reaction mixture was again cooled to - 78°C and a solution of the imine intermediate in dry diethyl ether (4 ml) was added slowly. Cooling was removed and the reaction was stirred at room temperature for 24h. The crude reaction mixture was diluted with EtOAc and the organics washed with ammonium chloride (aq.) solution, followed by brine. The organic layer was separated, dried (MgSO₄) and concentrated under reduced pressure to provide the title compound (800 mg), which was used without purification to the next step. ¹H NMR (CDCl₃) δ 7.29-7.21 (m, 4H), 3.08-3.0 (m, 2H), 2.87 (d, *J* = 8.4 Hz, 1 H), 2.67-2.61 (m, 1 H), 2.23 (s, 3H), 2.03-1.38 (m, 9H), 1.02-0.88 (m, 1 H), 0.63-0.53 (m, 1 H), 0.43-0.33 (m, 1 H), 0.32-0.18 (m, 1 H). LCMS (Method 2) m/z 305.2 (M + H)⁺.

### Example 1: N-[cyclopropyl(4-methoxyphenyl)methyl]-3-(4-fluorophenyl)-N-(pyridin-2-ylmethyl)butanamide

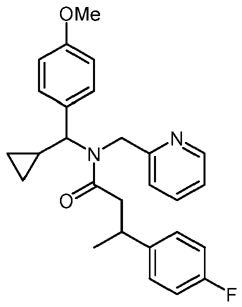

Intermediate a (25 mg, 0.09 mmol) was reacted with 3-(4-fluorophenyl)butanoyl chloride (46.7 mg, 0.23 mmol) in presence of triethylamine (26 µl, 0.19 mmol) according to General Procedure C to afford the title compound (34 mg, 84 %) as clear oil. ¹H NMR (CDCl₃) δ 8.52-8.37 (m, 1 H), 7.65-7.43 (m, 1 H), 7.30-6.70 (m, 10H), 5.19-4.96 (m, 1H), 4.55-4.12 (m, 2H), 3.81-3.77 (m, 3H), 3.57- 3.47 (m, 1H), 2.79-2.35 (m, 2H), 1.33-1.23 (m, 4H), 1.20-0.15 (m, 4H). ¹H NMR (d₆-DMSO, 110°C) δ 8.42 (br s, 1 H), 7.61-7.56 (m, 1 H), 7.27-6.99 (m, 8H), 6.84-6.81 (m, 2H), 4.72-4.67 (m, 2H), 4.34 (t, *J* = 17.7 Hz, 1 H), 3.75 (s, 3H), 3.42-3.32 (m, 1 H), 2.80-2.43 (m, 2H), 1.28-1.12 (m, 4H), 0.68-0.61 (m, 1 H), 0.32-0.12 (m, 3H). LCMS (Method 2) Rt 2.346 min (98.2% purity), m/z 433.3 (M + H)⁺.

### Example 2: N-[cyclopropyl(4-methoxyphenyl)methyl]-3-(4-fluorophenyl)-N-(pyridin-2-ylmethyl)propanamide

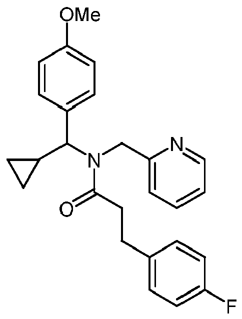

Intermediate **a** (30 mg, 0.11 mmol) was reacted with 3-(4-fluorophenyl)propanoyl chloride ( 41.7 mg, 0.22 mmol) in presence of triethylamine (31.2 µl, 0.22 mmol) according to General Procedure C to afford the title compound (32 mg, 68 %) as clear oil. ¹H NMR (CDCl₃) δ 8.49-8.41 (m, 1 H), 7.56-7.50 (m, 1 H), 7.26-7.05 (m, 6H), 6.99-6.89 (m, 2H), 6.84-6.78 (m, 2H), 5.20-5.11 (m, 1 H), 4.51-4.09 (m, 2H), 3.79 (br s, 3H), 3.06- 2.90 (m, 2H), 2.75-2.47 (m, 2H), 1.04-0.95 (m, 1 H), 0.79-0.66 (m, 1 H), 0.39-0.28 (m, 2H), 0.12-0.04 (m, 1 H). LCMS (Method 2) Rt 2.192 min (96.6% purity), m/z 419.2 (M + H)⁺.

### Example 9: N-[cyclopropyl(4-chlorophenyl)methyl]-3-(4-fluorophenyl)-N-(pyridin-2-ylmethyl)propanamide

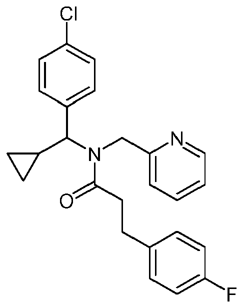

Intermediate **b** (30 mg, 0.11 mmol) was reacted with 3-(4-fluorophenyl)propanoyl chloride (41 mg, 0.22 mmol) in presence of triethylamine (31 µl, 0.22 mmol) according to General Procedure C to afford the title compound (40 mg, 86 %) as clear oil. ¹H NMR (CDCl₃) δ 8.49-8.38 (m, 1 H), 7.58-7.50 (m, 1 H), 7.28-6.90 (m, 10H) 5.19-5.11 (m, 1 H), 4.53-4.07 (m, 2H), 3.05-2.95 (m, 2H), 2.70- 2.51 (m, 2H), 1.18-0.92 (m, 1H), 0.86-0.68 (m, 1H), 0.58-0.49 (m, 1H), 0.40-0.27 (m, 2H). LCMS (Method 2) Rt 2.605 min (100% purity), m/z 423.3 (M + H)⁺.

### Example 10 N-[(4-chlorophenyl)(cyclopropyl)methyl]-2-(4-fluorophenyl)-N-(pyridin-2-ylmethyl)cyclopropanecarboxamide

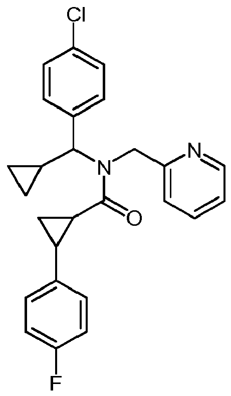

Intermediate **b** (23 mg, 0.08 mmol) was reacted with 2-(4-fluorophenyl)cyclopropanecarbonyl chloride (33.5 mg, 0.17 mmol) in presence of triethylamine (23.5 µl, 0.17 mmol) according to General Procedure C to afford the title compound (28 mg, 76 %) as clear oil. ¹H NMR (CDCl₃) δ 8.44-8.35 (m, 1 H), 7.65-6.80 (m, 11 H), 5.22-5.07 (m, 1 H), 4.78-4.28 (m, 2H), 2.52-2.43 (m, 1 H), 1.88- 1.66

(m, 2H), 1.30-1.05 (m, 2H), 0.90-054 (m, 2H), 0.48-0.12 (m, 2H). LCMS (Method 2) Rt 2.652 min (98.4% purity), m/z 435.2 (M + H)⁺.

### Example 15: N-[cyclopropyl(phenyl)methyl]-2-[(4-fluorophenyl)sulfonyl]-N-(pyridin-2-ylmethyl)acetamide

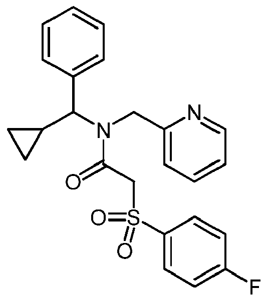

Intermediate **e** (111 mg, 0.47 mmol), [(4-fluorophenyl)sulfonyl]acetic acid (prepared according to the procedure outlined in Bioorganic & Medicinal Chemistry Letters 2009, 19(1), 31-35) (203 mg, 0.93 mmol), triethylamine (0.13 ml, 0.93 mmol) and T3P (0.89 ml, 1.40 mmol) were reacted according to General Procedure D to give the title compound as a vitreous solid. ¹H NMR (d₆-DMSO) δ 8.50-8.36 (m, 1 H), 8.01-7.97 (m, 1 H), 7.69-7.63 (m, 2H), 7.51-7.09 (m, 9H), 5.07-4.61 (m, 4H), 4.48-4.09 (m, 1H), 1.18-1.07 (m, 1H), 0.72-0.15 (m, 4H). HPLC (Method 1) Rt 2.99 min (Purity: 99.2%). UPLC/MS (Method 3) 439.2 (M+H)⁺.

### Example 20: 3-(4-fluorophenyl)-N-(2-methyl-1-phenylpropyl)-N-(pyridin-2-ylmethyl)propanamide

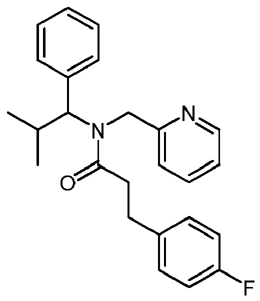

Intermediate h (124 mg, 0.52 mmol), 3-(4-fluorophenyl)propionic acid (174 mg, 1.03 mmol), triethylamine (0.14 ml, 1.03 mmol) and T3P (0.98 ml, 1.55 mmol) were reacted according to General Procedure D to give the title compound as a yellow oil. ¹H NMR (d₆-DMSO) δ 8.32-8.28 (m, 1H), 7.37-6.97 (m, 11H), 6.31-6.14 (m, 1H), 5.43-4.34 (m, 3H), 3.23-2.22 (m, 5H), 1.88-0.73 (m, 6H). HPLC (Method 1) Rt 3.43 min (Purity: 99.6%). UPLC/MS (Method 3) 391.2 (M+H)⁺.

### Example 21: N-[cyclopentyl(4-fluorophenyl)methyl]-3-(4-fluorophenyl)-N-(pyridin-2-ylmethyl)propanamide

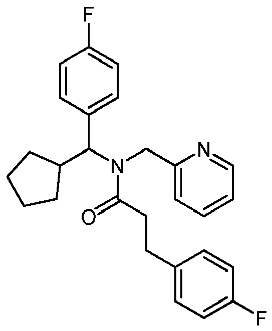

Intermediate i (125 mg, 0.44 mmol), 3-(4-fluorophenyl)propionic acid (148 mg, 0.88 mmol), triethylamine (0.12 ml, 0.88 mmol) and T3P (0.84 ml, 1.32 mmol) were reacted according to General Procedure D to give the title compound as a yellow oil. ¹H NMR (d₆-DMSO) δ 8.35-8.31 (m, 1H), 7.46-6.92 (m, 10H), 6.57-6.38 (m, 1H), 5.58-4.90 (m, 1H), 4.60-4.46 (m, 2H), 3.23-2.68 (m, 4H), 2.48-2.36 (m, 1H), 1.61-1.36 (m, 6H), 1.26-0.87 (m, 2H). HPLC (Method 1) Rt 3.80 min (Purity: 99.9%). UPLC/MS (Method 3) 435.2 (M+H)⁺.

### Example 22: N-(2-ethyl-1-phenylbutyl)-3-(4-fluorophenyl)-N-(pyridin-2-ylmethyl)propanamide

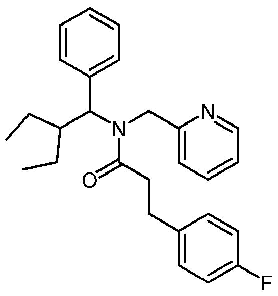

Intermediate j (111 mg, 0.41 mmol), 3-(4-fluorophenyl)propionic acid (139 mg, 0.83 mmol), triethylamine (0.12 ml, 0.83 mmol) and T3P (0.79 ml, 1.24 mmol) were reacted according to General Procedure D to give the title compound as a yellow oil. ¹H NMR (d₆-DMSO) δ 8.32-8.29 (m, 1H), 7.35-6.98 (m, 11H), 6.27-6.11 (m, 1H), 5.71-4.85 (m, 1H), 4.67-4.40 (m, 2H), 3.19-2.65 (m, 3H), 2.35-2.18 (m, 2H), 1.41-0.98 (m, 4H), 0.79-0.66 (m, 6H). HPLC (Method 1) Rt 3.86 min (Purity: 99.7%). UPLC/MS (Method 3) 419.3 (M+H)⁺.

### Example 23: N-[cyclopentyl(phenyl)methyl]-3-(4-fluorophenyl)-N-(pyridin-2-ylmethyl)propanamide

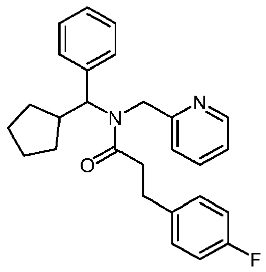

Intermediate k (128 mg, 0.48 mmol), 3-(4-fluorophenyl)propionic acid (162 mg, 0.96 mmol), triethylamine (0.13 ml, 0.96 mmol) and T3P (0.92 ml, 1.44 mmol) were reacted according to General Procedure D to give the title compound as a yellow oil. ¹H NMR (d₆-DMSO) δ 8.36-8.31 (m, 1H), 7.42-6.99 (m, 11H), 6.50-6.42 (m, 1H), 5.61-4.87 (m, 1H), 4.59-4.39 (m, 2H), 3.03-2.67 (m, 4H), 2.43-2.31 (m, 1H), 1.62-1.28 (m, 6H), 1.26-0.86 (m, 2H). HPLC (Method 1) Rt 3.74 min (Purity: 99.2%). UPLC/MS (Method 3) 417.2 (M+H)⁺.

### Example 24: 3-(4-fluorophenyl)-N-[1-(2-methyl-2,3-dihydro-1-benzofuran-5-yl)propyl]-N-(pyridin-2-ylmethyl)propanamide

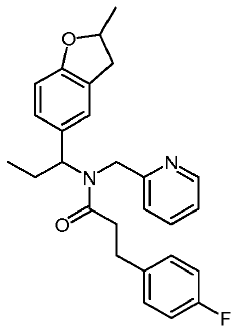

Intermediate I (60 mg, 0.21 mmol), 3-(4-fluorophenyl)propionic acid (54 mg, 0.32 mmol), T3P (178 µl, 0.32 mmol) and triethylamine (29 µl, 0.21 mmol) were reacted according to General Procedure D to give the title compound as a colourless oil (51 mg). ¹H NMR (d₆-DMSO) δ 8.42-8.33(m, 1H), 7.56-7.46 (m, 1H), 7.38-6.94 (m, 7H), 6.81-6.76 (m, 1H), 6.58-6.52 (m, 1H), 5.65-4.96 (m, 1H), 4.89-4.77 (m, 1H), 4.48-4.22 (m, 2H), 3.24-2.81 (m, 4H), 2.70-2.52 (m, 2H), 1.93-1.66 (m, 2H), 1.33-1.31 (m, 3H), 0.77-0.70 (m, 3H). HPLC (Method 1) Rt 3.36 min (Purity: 100.0%). UPLC/MS (Method 3) 433.4 (M+H)⁺.

### Example 41: N-[1-(2,3-dihydro-1H-inden-5-yl)ethyl]-3-(4-fluorophenyl)-N-[2-(3,6-dimethylpyrazin-2-yloxy)ethyl]butanamide

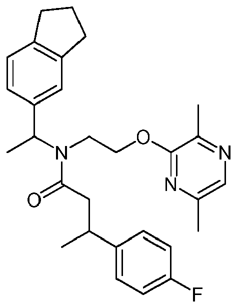

Intermediate v (70 mg, 0.23 mmol) was reacted with Intermediate mm (113 mg, 0.56 mmol) in presence of triethylamine (63 µl, 0.45 mmol) according to General Procedure C to afford the title compound (75 mg, 70 %) as clear oil. ¹H NMR (CDCl₃) δ 7.86-7.80 (m, 1H), 7.25-6.76 (m, 7H), 6.05-4.97 (m, 1H), 4.42- 3.12 (m, 5H), 2.91 - 2.58 (m, 5H), 2.36-2.28 (m, 6H), 2.12-1.98 (m, 2H), 1.68-1.26 (m, 7H). LCMS (Method 2) Rt 4.153 min (95% purity), m/z 476.5 (M + H)⁺.

### Example 42: 3-(4-fluorophenyl)-N-(1-phenylethyl)-N-(pyridin-2-ylmethyl)propanamide

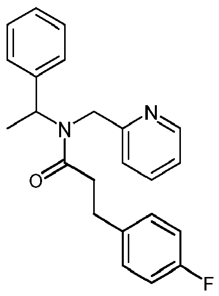

A solution of 1-phenyl-*N*-(pyridin-2-ylmethyl)ethanamine (prepared according to the procedure outlined in Tetrahedron Letters 2006, 62(18) 4506-4518) (92 mg, 0.43 mmol) was reacted with 3-(4-fluorophenyl)propanoyl chloride (260 mg, 1.39 mmol) in the presence of triethylamine (206 µl, 1.48 mmol) according to General Procedure C to give the title compound (42 mg, 62%) as colourless oil. ¹H NMR (CDCl₃)δ 8.48-8.40 (m, 1H), 7.58-7.30 (m, 1H), 7.31-6.80 (m, 11H), 6.24-5.20 (m, 1H), 4.98-4.16 (m, 2H), 3.06-2.53 (m, 4H), 1.49-1.38 (m, 3H). LCMS (Method 2) Rt 2.145 min (Purity: 98.4%), m/z 363.3(M+H)⁺.

### Example 43: 3-(4-fluorophenyl)-N-(1-phenylethyl)-N-(6-methoxypyridin-3-ylmethyl)propanamide

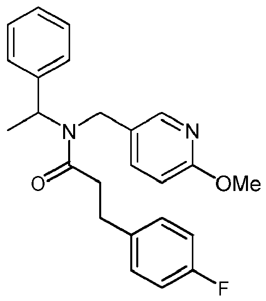

A solution of 1-phenyl-*N*-(pyridin-2-ylmethyl)ethanamine (prepared according to the procedure outlined in Tetrahedron Letters 2006, 62(18) 4506-4518) (92 mg, 0.38 mmol) was reacted with 3-(4-fluorophenyl)propanoyl chloride (226 mg, 1.21 mmol) in the presence of triethylamine (189 µl, 1.36 mmol) according to General Procedure C to give the title compound as colourless oil. ¹H NMR (CDCl₃) δ 7.80 (br s, 1H), 7.37-6.9 0 (m, 10H), 6.58 (d, *J* = 8.7 Hz, 1H), 6.19-5.13 (m, 1H), 4.69-3.89 (m, 5H), 3.06-2.45 (m, 4H), 1.56-1.40 (m, 3H). LCMS (Method 2) Rt 2.351 min (Purity: 100.0%), m/z 393.2(M+H)⁺.

### Example 44: N-[1-(2,3-dihydro-1H-inden-5-yl)ethyl]-3-(4-fluorophenyl)-N-[2-(6-chloropyridin-2-yloxy)ethyl]butanamide

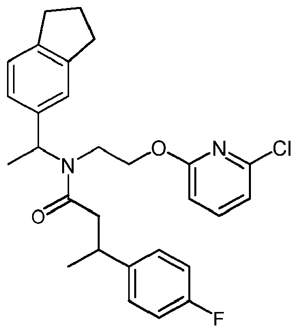

Intermediate **x** (67 mg, 0.21 mmol), 3-(4-fluorophenyl)butanoyl chloride and (75 mg, 0.38 mmol), DIPEA (60 µl, 0.35 mmol) were reacted according to General Procedure C to afford the titled compound (93 mg, 91%) as a yellow oil. ¹H NMR (CDCl₃) δ 7.53-7.44 (m, 1H), 7.30-7.04 (m, 4H), 6.99-6.72 (m, 4H), 6.55-6.50 (m, 1H), 6.07-4.97 (m, 1H), 4.38-4.19 (m, 1H), 4.12-3.84 (m, 1H), 3.80-3.09 (m, 3H), 2.95-2.74 (m, 5H), 2.70-2.59 (m, 1H), 2.12-1.98 (m, 2H), 1.67-1.29 (m, 6H). LCMS (Method 2) Rt 5.132 min (98% purity), m/z 481.2 (M + H)⁺.

### Example 45: N-[1-(2,3-dihydro-1H-inden-5-yl)ethyl]-3-(4-fluorophenyl)-N-[2-(6-chloropyridin-2-yloxy)ethyl]propanamide

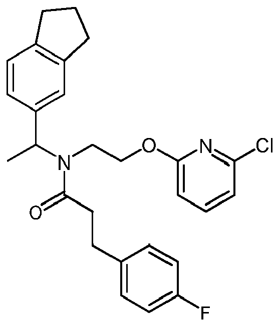

Intermediate **x** (66 mg, 0.21 mmol), 3-(4-fluorophenyl)propanoyl chloride, (58 mg, 0.31 mmol) and DIPEA (50 µl, 0.29 mmol) were reacted according to General Procedure C to afford the titled compound (83 mg, 86%) as a yellow oil. ¹H NMR (CDCl₃) δ 7.51-7.44 (m, 1H), 7.27-7.12 (m, 4H), 7.03-6.85 (m, 4H), 6.56-6.48 (m, 1H), 6.10-5.02 (m, 1H), 4.43-4.24 (m, 1H), 4.09-3.20 (m, 3H), 3.09-2.98 (m, 2H), 2.94-2.72 (m, 6H), 2.11-2.00 (m, 2H), 1.62-1.51 (m, 3H). LCMS (Method 2) Rt 4.592 min (98% purity), m/z 467.3 (M + H)⁺.

### Example 46: N-[1-(2,3-dihydro-1H-inden-5-yl)ethyl]-3-(4-fluorophenyl)-N-[2-(pyridin-2-yloxy)ethyl]butanamide

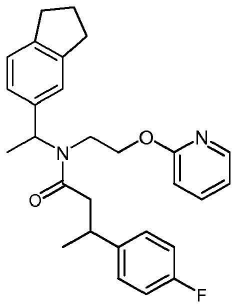

Intermediate y (69 mg, 0.24 mmol), 3-(4-fluorophenyl)butanoyl chloride (71 mg, 0.35 mmol) and DIPEA (60 µl, 0.35 mmol) were reacted according to General Procedure C to afford the titled compound (98 mg, 90%) as a pale yellow oil. ¹H NMR (CDCl₃) δ 8.10-8.04 (m, 1H), 7.58-7.49 (m, 1H), 7.28-7.04 (m, 4H), 6.99-6.76 (m, 4H), 6.64-6.59 (m, 1H), 6.04-4.96 (m, 1H), 4.38-4.23 (m, 1H), 4.18-4.02 (m, 1H), 3.87-3.14 (m, 3H), 2.98-2.58 (m, 6H), 2.11-1.98 (m, 2H), 1.64-1.29 (m, 6H). LCMS (Method 2) Rt 3.979 min (100% purity), m/z 447.3 (M + H)⁺.

### Example 47: N-[1-(2,3-dihydro-1H-inden-5-yl)ethyl]-3-(4-fluorophenyl)-N-[2-(pyridin-2-yloxy)ethyl]propanamide

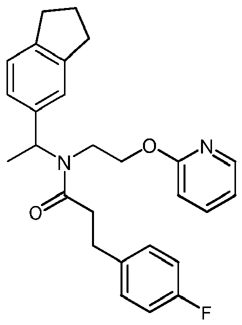

Intermediate **y** (71 mg, 0.25 mmol), 3-(4-fluorophenyl)propanoyl chloride (72 mg, 0.39 mmol) and DIPEA (60 µl, 0.35 mmol) were reacted according to General Procedure C to afford the titled compound (99 mg, 91%) as a yellow oil. ¹H NMR (CDCl₃) δ 8.11-7.97 (m, 1H), 7.56-7.50 (m, 1H), 7.24-7.13 (m, 4H), 7.04-6.92 (m, 3H), 6.91-6.81 (m, 1H), 6.65-6.57 (m, 1H), 6.07-5.03 (m, 1H), 4.45-4.29 (m, 1H), 4.16-3.22 (m, 3H), 3.07-2.97 (m, 2H), 2.94-2.71 (m, 6H), 2.10-1.99 (m, 2H), 1.60-1.52 (m, 3H). LCMS (Method 2) Rt 3.568 min (100% purity), m/z 433.3 (M + H)⁺.

### Example 50 : 1-(4-chlorophenyl)-N-[1-(2,3-dihydro-1H-inden-5-yl)ethyl]-N-[2-(pyridin-2-yloxy)ethyl]cyclobutanecarboxamide

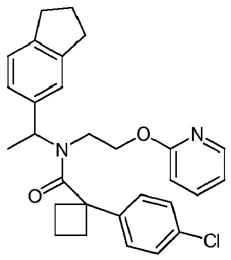

Intermediate v (59 mg, 0.21 mmol) was reacted with 1-(4-chlorophenyl)cyclobutanecarbonyl chloride (120 mg, 0.52 mmol) in presence of triethylamine (58 µl, 0.42 mmol) according to General Procedure C to afford title compound (65 mg, 65.5%) as clear oil. ¹H NMR (CDCl₃) δ 8.11-8.8.03 (m, 1H), 7.55-7.49 (m, 1H), 7.40-7.23 (m, 5H), 7.11-7.0 (m, 1H), 6.84-6.80 (m, 1H), 6.67-6.48 (m, 2H), 4.-64-4.59 (m, 1H), 4.47-4.40 (m, 1H), 4.32-2.25 (m, 1H), 3.67-3.48 (m, 1H), 3.07-2.70 (m, 7H), 2.40-2.25 (m, 2H), 2.06-1.80 (m, 4H), 1.18 (d, J = 6.9 Hz, 3H). LCMS (Method 2) Rt 5.920 min (100% purity), m/z 475.2 (M + H)⁺.

### Example 51: N-[1-(2,3-dihydro-1H-inden-5-yl)ethyl]-4-phenyl-N-[2-(pyridin-2-yloxy)ethyl]butanamide

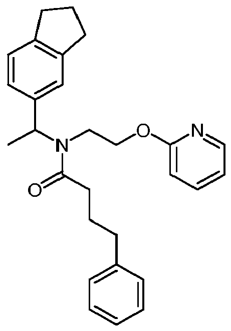

Intermediate **y** (72 mg, 0.25 mmol), 3-phenylpropanoyl chloride (76 mg, 0.41 mmol) and DIPEA (65 µl, 0.38 mmol) were reacted according to General Procedure C to afford the titled compound (96 mg, 88%) as a yellow oil. ¹H NMR (CDCl₃) δ 8.11-8.08 (m, 1H), 7.57-7.49 (m, 1H), 7.30-6.95 (m, 8H), 6.88-6.80 (m, 1H), 6.67-6.59 (m, 1H), 6.11-4.99 (m, 1H), 4.48-4.28 (m, 1H), 4.20-3.98 (m, 1H), 3.81-3.23 (m, 2H), 2.89-2.84 (m, 4H), 2.74-2.67 (m, 2H), 2.59-2.45 (m, 2H), 2.13-1.99 (m, 4H), 1.63-1.54 (m, 3H). LCMS (Method 2) Rt 4.078 min (100% purity), m/z 429.2 (M + H)⁺.

### Example 52: N-[1-(2,3-dihydro-1H-inden-5-yl)ethyl]-3-(4-fluorophenyl)-N-[2-(6-methylpyridin-2-yloxv)ethyl]butanamide

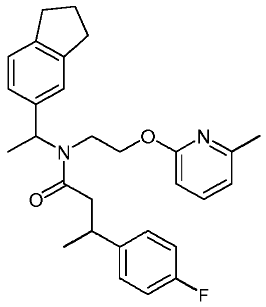

Intermediate **bb** (73 mg, 0.25 mmol), 3-(4-fluorophenyl)butanoyl chloride (71 mg, 0.35 mmol) and DIPEA (60 µl, 0.35 mmol) were reacted according to General Procedure C to afford the titled compound (103 mg, 91%) as a pale yellow oil. ¹H NMR (CDCl₃) δ 7.45-7.38 (m, 1H), 7.25-7.05 (m, 4H), 6.98-6.90 (m, 2H), 6.83-6.65 (m, 2H), 6.44-6.37 (m, 1H), 6.07-4.94 (m, 1H), 4.40-3.98 (m, 2H), 3.88-3.10 (m, 3H), 2.93-2.56 (m, 6H), 2.40-2.38 (m, 3H), 2.12-1.98 (m, 2H), 1.69-1.43 (m, 3H), 1.37-1.26 (m, 3H). LCMS (Method 2) Rt 5.038 min (100% purity), m/z 461.2 (M + H)⁺.

### Example 53: N-[1-(2,3-dihydro-1H-inden-5-yl)ethyl]-3-(4-fluorophenyl)-N-[2-(6-methylpyridin-2-yloxy)ethyl]propanamide

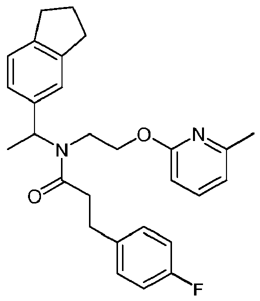

Intermediate **bb** (71 mg, 0.24 mmol), 3-(4-fluorophenyl)propanoyl chloride (66 mg, 0.35 mmol) and DIPEA (60 µl, 0.35 mmol) were reacted according to General Procedure C to afford the titled compound (74 mg, 70%) as a pale yellow oil. ¹H NMR (CDCl₃) δ 7.44-7.38 (m, 1H), 7.22-7.13 (m, 4H), 7.04-6.90 (m, 3H), 6.69-6.67 (m, 1H), 6.46-6.36 (m, 1H), 6.07-5.00 (m, 1H), 4.45-4.25 (m, 1H), 4.16-3.20 (m, 3H), 3.13-2.92 (m, 2H), 2.89-2.71 (m, 6H), 2.40-2.30 (m, 3H), 2.11-2.00 (m, 2H), 1.68-1.53 (m, 3H). LCMS (Method 2) Rt 4.44 min (98% purity), m/z 447.3 (M + H)⁺.

### Example 54: N-[1-(2,2-dimethyl-1,3-benzoxathiol-5-yl)ethyl]-3-(4-fluorophenyl)-N-(pyridin-2-ylmethyl)propanamide

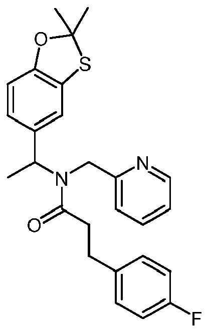

Intermediate cc (83 mg, 0.28 mmol), 3-(4-fluorophenyl)propionic acid (46 mg, 0.28 mmol), triethylamine (0.04 ml, 0.28 mmol) and T3P (0.18 ml, 0.28 mmol) were reacted according to General Procedure D to give the title compound as a colorless oil (75 mg, 61%). ¹H NMR (CDCl₃) δ 8.40 (d, *J* = 4.0 Hz, 0.7H), 8.35 (d, *J* = 4.9 Hz, 0.3H), 7.43 (td, *J* = 7.7, 1.8 Hz, 1H), 7.17 - 6.62 (m, 7H), 6.56 (t, *J* = 8.7 Hz, 1H), 6.04 (q, *J* = 7.1 Hz, 0.6H), 5.07 (d, *J*= 6.6 Hz, 0.4H), 4.78 (d, *J*= 16.0 Hz, 0.4H), 4.25 (m, 1.6H), 3.64 (t, *J* = 5.7 Hz, 0.2H), 2.95 (dt, *J* = 19.7, 7.2 Hz, 1.8H), 2.77 (t, *J* = 7.3 Hz, 0.6H), 2.59 - 2.38 (m, 1.4H), 1.74 (s, 5H), 1.49 (s, 1H), 1.37 (d, *J* = 7.0 Hz, 1H), 1.26 (d, *J* = 7.2 Hz, 2H). HPLC (Method 1) Rt 3.61 min (Purity: 97.8%). UPLC/MS (Method 3) 451.3 (M+H)⁺.

### Example 57: 3-(4-fluorophenyl)-N-{1-[2-(methoxymethyl)phenyl]ethyl}-N-(pyridin-2-ylmethyl)propanamide

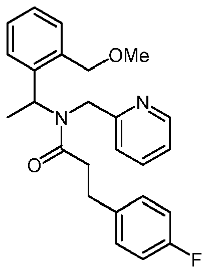

Intermediate ff (80 mg; 0.31 mmol), 3-(4-fluorophenyl)propionic acid (58 mg, 0.34 mmol), T3P (348 µl, 0.62 mmol) and triethylamine (42 µl, 0.31 mmol) were reacted according to General Procedure D to give the title compound as a yellow oil. ¹H NMR (d₆-DMSO) δ 8.38-8.33 (m, 1H), 7.58-7.35 (m, 2H), 7.28-7.01 (m, 8H), 6.94-6.65 (m, 1H), 6.05-5.53 (m, 1H), 4.70-4.06 (m, 4H), 3.24-3.20 (m, 3H), 2.94-2.53 (m, 4H), 1.45-1.31 (m, 3H). HPLC (Method 1) Rt 3.30 min (Purity: 100.0%). UPLC/MS (Method 3) 407.3 (M+H)⁺.

### Example 62: N-[1-(2,2-dimethyl-1,3-benzoxathiol-5-yl)ethyl]-N-(pyridin-2-ylmethyl)-3-[3-(trifluoromethyl)phenyl]propanamide

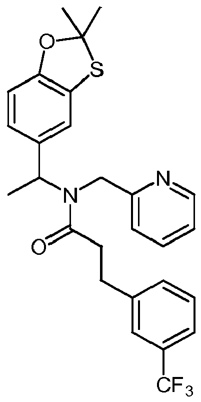

Intermediate cc (83 mg, 0.28 mmol), 3-(3-trifluoromethylphenyl)propionic acid (60 mg, 0.28 mmol), T3P (0.18 ml, 0.28 mmol and triethylamine (0.04 ml, 0.28 mmol) were reacted according to General Procedure D to give the title compound as a yellow oil. ¹H NMR (CDCl₃) δ 8.47-8.41 (m, 1H), 7.61-7.35 (m, 5H), 7.13-6.60 (m, 5H), 6.14-5.11 (m, 1H), 4.89-4.29 (m, 2H), 3.16-3.01 (m, 2H), 2.91-2.57 (m, 2H), 1.80 (s, 6H), 1.45-1.32 (m, 3H). HPLC (Method 1) Rt 3.99 min (Purity: 96.8%). UPLC/MS (Method 3) 501.4 (M+H)⁺.

### Example 63: N-[1-(2,2-dimethyl-3-oxido-1,3-benzoxathiol-5-yl)ethyl]-N-(pyridin-2-ylmethyl)-3-[3-(trifluoromethyl)phenyl]propanamide

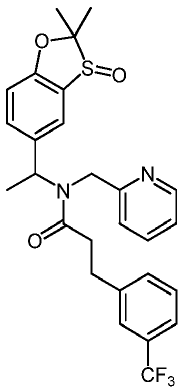

To a solution of Example 62 (70 mg, 0.14 mmol) in glacial AcOH (5 ml) at 0 °C was added H₂O₂ (0.03 ml, 0.28 mmol). After stirring for 2 h, H₂O₂ (0.12 ml, 1.12 mmol) was added and the stirring continued at RT overnight. Water was added and the aqueous phase was extracted with DCM. The combined organics were then extracted with 1 M NaOH, dired (MgSO₄), filtered and concentrated under reduced pressure. The crude mixture was purified by MD Autoprep to give the title compound as a vitreous solid. ¹H NMR (CDCl₃) δ 8.41-8.29 (m, 1H), 7.79-7.30 (m, 7H), 7.22-6.79 (m, 3H), 6.23-5.18 (m, 1H), 4.87-4.29 (m, 2H), 3.26-2.61 (m, 4H), 1.81 (br s, 3H), 1.50-1.39 (m, 6H). HPLC (Method 1) Rt 3.42 min (Purity: 85.9%). UPLC/MS (Method 3) 517.3 (M+H)⁺.

### Example 64: N-[1-(2,2-dimethyl-3-oxido-1,3-benzoxathiol-5-yl)ethyl]-3-(4-fluorophenyl)-N-(pyridin-2-ylmethyl)propanamide

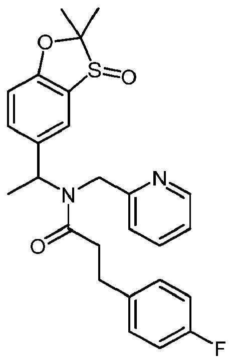

To a solution of Example 54 (70 mg, 0.16 mmol) in glacial AcOH (5 ml) at 0 °C was added H₂O₂ (35 µl, 0.31 mmol). After stirring for 2 h, H₂O₂ (0.14 ml, 1.24 mmol) was added and the stirring continued at RT overnight. Water was added and the aqueous phase was extracted with DCM. The combined organics were then extracted with 1 M NaOH, dired (MgSO₄), filtered and concentrated under reduced pressure. The crude was purified by MD Autoprep to give the title compound as a vitreous solid. ¹H NMR (CDCl₃) δ 8.50-8.37 (m, 1H), 7.72-6.82 (m, 10H), 6.22-5.19 (m, 1H), 4.85-4.21 (m, 2H), 3.10-2.53 (m, 4H), 1.81 (s, 3H), 1.51-1.37 (m, 6H). HPLC (Method 1) Rt 2.81 min (Purity: 96.0%). UPLC/MS (Method 3) 467.3 (M+H)⁺.

### Example 65: 2-[(4-fluorophenyl)sulfonyl]-N-{1-[2-(methoxymethyl)phenyl]ethyl}-N-(pyridin-2-ylmethyl)acetamide and Example 66: 1-[(4-fluorophenyl)sulfonyl]-N-{1-[2-(methoxymethyl)phenyl]ethyl}-N-(pyridin-2-ylmethyl)cyclopropanecarboxamide

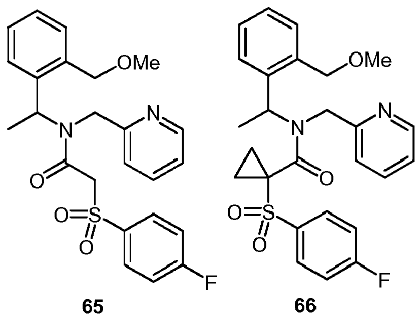

Intermediate ff (120 mg, 0.47 mmol), 1-[(4-fluorophenyl)sulfonyl]cyclopropanecarboxylic acid (prepared according to the procedure outlined in the Bulletin of the Chemical Society of Japan 1985, 58(2), 765-6) (172 mg, 0.70 mmol) containing 10% of [(4-fluorophenyl)sulfonyl]acetic acid, T3P (392 µl, 0.70 mmol) and triethylamine (65 µl, 0.47 mmol) were reacted according to General Procedure D to give Example 65 and Example 66 as brown solids. Example 65: HPLC (Method 1) Rt 2.78 min (Purity: 93.7%). UPLC/MS (Method 3) 457.1 (M+H)⁺. Example 66: ¹H NMR (CDCl₃) δ 8.44-8.43 (m, 1H), 7.69-7.64 (m, 2H), 7.48-7.38 (m, 2H), 7.30-7.07 (m, 6H), 6.75-6.72 (m, 1H), 5.80-5.70 (m, 1H), 5.12-5.00 (m, 2H), 4.66-4.62 (m, 1H), 4.27-4.23 (m, 1H), 3.36 (s, 3H), 1.95 (br s, 1H), 1.77-1.54 (m, 4H), 1.40 (d, *J* = 7.0 Hz, 3H). HPLC (Method 1) Rt 3.11 min (Purity: 97.6%). UPLC/MS (Method 3) 483.1 (M+H)⁺.

### Example 67: N-[1-(2,3-dihydro-1H-inden-5-yl)ethyl]-3-(4-fluorophenyl)-N-(pyridin-2-ylmethyl)propanamide

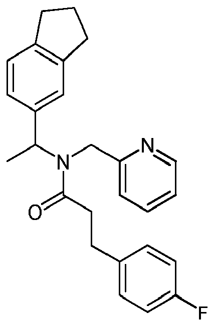

Intermediate gg (100 mg, 0.40 mmol), 3-(4-fluorophenyl)propionic acid (73 mg, 0.44 mmol), T3P (442 µl, 0.79 mmol) and triethylamine (53 µl, 0.40 mmol) were reacted according to General Procedure D to give the title compound as a yellow oil. ¹H NMR (d₆-DMSO) δ 8.48-8.46 (m, 1H), 7.67-7.58 (m, 1H), 7.32-6.94 (m, 9H), 5.94-5.31 (m, 1H), 4.69-4.01 (m, 2H), 2.99-2.65 (m, 8H), 2.02-1.91 (m, 2H), 1.39-1.24 (m, 3H). HPLC (Method 1) Rt 3.68 min (Purity: 100.0%). UPLC/MS (Method 3) 403.3 (M+H)⁺.

### Example 68 N-[1-(2,3-dihydro-1H-inden-5-yl)ethyl]-N-[3-(4-fluorophenyl)butyl]pyridine-3-carboxamide

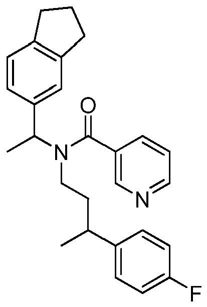

Intermediate ii (81 mg, 0.26 mmol), nicotinoyl chloride hydrochloride (56 mg, 0.32 mmol) and DIPEA (110 µl, 0.64 mmol) were reacted as described under General Procedure C to afford the titled compound (71 mg, 65%) as a yellow oil. ¹H NMR (d₆-DMSO, 100 °C) δ 8.63-8.50 (m, 2H), 7.79-7.70 (m, 1H), 7.45-7.41 (m, 1H), 7.19-7.16 (m, 1H), 7.11-6.97 (m, 6H), 5.16 (br s, 1H), 3.30-2.75 (m, 9H), 2.27-1.98 (m, 2H), 1.78-1.44 (m, 3H), 1.30-0.80 (m, 3H). ¹H NMR (CDCl₃) δ 8.70-8.55 (m, 2H), 7.84-7.50 (m, 1H), 7.40-7.28 (m, 1H), 7.24-6.46 (m 7H), 6.10-4.80 (m, 1H), 3.50-2.50 (m, 9H), 2.34-2.00 (m, 2H), 1.90-1.40 (m, 3H), 1.34-0.75 (m, 3H). LCMS (Method 2) Rt 3.161 min (95% purity), m/z 417.3 (M + H)⁺.

### Example 69 2-(3,3-difluoropiperidin-1-yl)-N-(2,3-dihydro-1H-inden-5-yl)ethyl]-N-[3-(4-fluorophenyl)butyl]acetamide

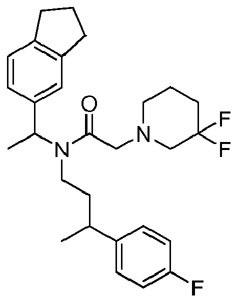

Intermediate **jj** (117 mg, 0.30 mmol), 3,3-difluoropiperidine hydrochloride (98 mg, 0.62 mmol), Na₂CO₃ (69 mg, 0.65 mmol) and Nal (151 mg, 1.00 mmol) were reacted as described under General Procedure I to afford the titled compound (106 mg, 75%) as a yellow oil. ¹H NMR (CDCl₃) δ 7.18-6.75 (m, 7H), 5.90-5.27 (m, 1H), 3.43-2.96 (m, 3H), 2.94-2.71 (m, 6H), 2.66-2.20 (m, 4H), 2.15-2.02 (m, 2H), 2.00-1.57 (m, 6H), 1.51-1.34 (m, 3H), 1.14-0.85 (m, 3H). LCMS (Method 2) Rt 4.475-4.619 min (100% purity), m/z 473.2 (M + H)⁺.

### Example 70 N-[1-(2,3-dihydro-1H-inden-5-yl)ethyl]-N-[3-(4-fluorophenyl)butyl]-2-(4-fluoropiperidin-1-yl)acetamide

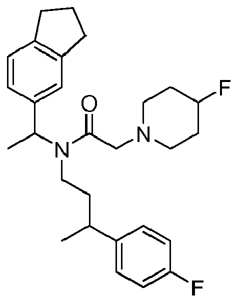

Intermediate **jj** (106 mg, 0.27 mmol), 4-fluoropiperidine hydrochloride (84 mg, 0.60 mmol), Na₂CO₃ (62 mg, 0.58 mmol) and Nal (140 mg, 0.93 mmol) were reacted as described under General Procedure I to afford the titled compound as a yellow oil. ¹H NMR (CDCl₃) δ 7.20-6.83 (m, 7H), 6.95-5.30 (m, 1H), 4.81-4.51 (m, 1H), 3.33-3.12 (m, 2H), 3.10-2.78 (m, 6H), 2.75-2.20 (m, 7H), 2.15-2.02 (m, 2H), 2.00-1.59 (m, 4H), 1.51-1.40 (m, 3H), 1.25-1.06 (m, 3H). LCMS (Method 2) Rt 4.820 min (100% purity), m/z 455.3 (M + H)⁺.

### Example 76: N-[(4-chlorophenyl)(cyclopropyl)methyl]-3-[(4-fluorophenyl)sulfonyl]-N-(pyridin-2-ylmethyl)butanamide

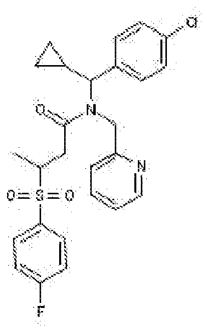

Intermediate b (100 mg, 0.37 mmol), 3-[(4-fluorobenzene)sulfonyl]butanoic acid (135 mg; 0.55 mmol), T3P (409 µl, 0.73 mmol) and triethylamine (77 µl, 0.55 mmol) were reacted according to General Procedure D to give the title compound as a pale pink solid. ¹H NMR (d₆-DMSO) δ 8.51-8.43 (m, 1H), 7.93-7.83 (m, 2H), 7.64-7.54 (m, 1H), 7.34-7.08 (m, 8H), 5.12-5.07 (m, 1H), 4.65-4.17 (m, 2H), 3.94-3.84 (m, 1H), 3.19-3.07 (m, 1H), 2.68-2.60 (m, 1H), 1.34-0.15 (m, 8H). HPLC (Method 1) Rt 3.55 min (Purity: 94.7%). UPLC/MS (Method 3) 501.1 (M+H)⁺.

### Example 77: N-[cyclopropyl(2,2-dimethyl-3,3-dioxido-1,3-benzoxathiol-5-yl)methyl]-2-(4-fluorophenoxy)-N-(pyridin-2-ylmethyl)acetamide

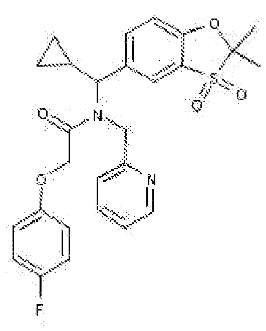

Intermediate yy (30 mg, 0.08 mmol), 4-fluorophenoxyacetic acid (29 mg, 0.17 mmol), T3P (0.11 ml, 0.17 mmol) and triethylamine (0.03 ml, 0.25 mmol) were reacted according to General Procedure D to give the title compound as a white solid. ¹H NMR (CDCl₃) δ 8.54-8.38 (m, 1H), 7.80-7.69 (m, 1H), 7.50 (td, *J* = 7.7, 1.8Hz, 1H), 7.41 (dd, *J* = 8.6, 1.9Hz, 1H), 7.18-6.78 (m, 7H), 5.10-4.44 (m, 5H), 1.73-1.71 (m, 6H), 1.28-0.22 (m, 5H). HPLC (Method 1) Rt 3.44 min (Purity: 96.5%). UPLC/MS (Method 3) 511.0 (M+H)⁺.

### Example 78: 3-(4-fluorophenyl)-N-[1-(4-methyl-4H-1,2,4-triazol-3-yl)ethyl]-N-(pyridin-2-ylmethyl)butanamide

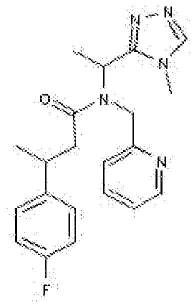

Intermediate zz (44 mg, 0.2 mmol), ,3-(4-fluorophenyl)butanoic acid (55 mg; 0.30 mmol), T3P (226 µl, 0.41 mmol) and triethylamine (42 µl, 0.3 mmol) were reacted according to General Procedure D to give the title compound as a brown solid. ¹H NMR (d₆-DMSO) δ 8.38-8.30 (m, 1H), 7.85-7.80 (m, 1H), 7.56-7.49 (m, 1H), 7.22-7.07 (m, 3H), 6.29-6.17 (m, 1H), 4.68-4.36 (m, 2H), 3.53 (s, 1.5H), 3.51-3.41 (m, 1H), 3.14 (s, 1.5H), 2.99-2.57 (m, 2H), 1.67-1.55 (m, 3H), 1.29-1.26 (m, 3H). HPLC (Method 1) Rt 2.21 min (Purity: 97.7%). UPLC/MS (Method 3) 382.2 (M+H)⁺.

### Example 80: Preparation of N-[(4-chlorophenyl)(cyclopropyl)methyl]-2-[(4-fluorophenyl)sulfonyl]-N-(8-methyl-8-azabicyclo[3.2.1]octan-3-yl)acetamide

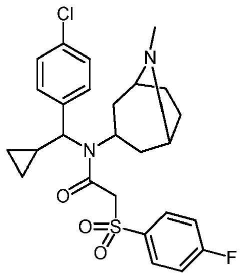

Intermediate bbb (430 mg, 1.41 mmol), (4-fluoro-benzenesulfonyl)-acetic acid (339 mg, 1.55 mmol), T3P (1.678 ml, 2.82 mmol) and triethylamine (0.393 ml, 2.82 mmol) were reacted according to General Procedure D to give the title compound as a white solid (380 mg, 53%). ¹H NMR (CDCl₃) δ 7.99-7.92 (m, 2H), 7.49-7.47 (m, 2H), 7.38-7.35 (m, 2H), 7.30-7.23 (m, 2H), 4.69-4.56 (m, 1H), 4.26-4.04 (m, 3H), 3.65-3.44 (m, 2H), 3.03-2.92 (m, 1H), 2.61 (d, *J* = 3.9 Hz, 3H), 2.26-1.84 (m, 7H), 1.86-1.75 (m, 1H), 0.99-0.90 (m, 1H), 0.81-0.71 (m, 1H), 0.63-0.55 (m, 1H), 0.49-0.41 (m, 1H). LCMS (Method 2) Rt 2.59 min (Purity: 99.4%), m/z 505.2 (M + H)⁺.

### Example 87: 3-(4-fluorophenyl)-N-[1-(4-methyl-4H-1,2,4-triazol-3-yl)ethyl]-N-(pyridin-2-ylmethyl)butanamide

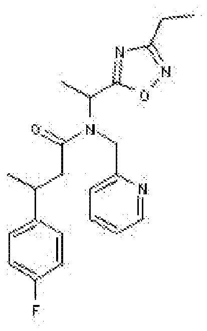

Intermediate aaa (87 mg, 0.37 mmol), ,3-(4-fluorophenyl)butanoic acid (102 mg; 0.56 mmol), T3P (418 µl, 0.75 mmol) and triethylamine (78 µl, 0.56 mmol) were reacted according to General Procedure D to give the title compound as a brown oil. ¹H NMR (d₆-DMSO) δ 8.59-8.52 (m, 1H), 7.78-7.59 (m, 1H), 7.24-7.18 (m, 1H), 7.15-7.09 (m, 2H), 7.03-6.90 (m, 3H), 5.94-5.30 (m, 1H), 4.96-4.46 (m, 2H), 3.50-3.40 (m, 1H), 2.94-2.46 (m, 4H), 1.76-1.56 (m, 1H), 1.54-1.47 (m, 2H), 1.35-1.31 (m, 1H), 1.27-1.20 (m, 5H). HPLC (Method 1) Rt 3.03 min (Purity: 100.0%). UPLC/MS (Method 3) 397.2 (M+H)⁺.

### D BIOLOGY PROTOCOLS

### 1. STABLE CELL LINE GENERATION AND PROPAGATION

### CHO/Kv1.3

The human Kv1.3 construct (Kv1.3/pENTR, clone IOH61428) was purchased from Invitrogen and subcloned into pcDNA 3.2-DEST (Invitrogen). CHO-K1 cells obtained from ATCC (cat #CCL-61) were transfected with the Kv1.3/pcDNA3.2DEST construct and a stable cell line was developed through two rounds of limiting dilution under G418 selection. Positive (Kv1.3 expressing) clones were identified using electrophysiology.

### CHO/Kv1.1 and CHO/Kv1.5

The sequence verified (accession # NM_000217) human Kv1.1 construct was obtained from a hippocampus cDNA library (Clontech). The sequence verified (Accession # NM_002234) human Kv1.5 construct. Both constructs were subcloned into pcDNA3.1(Neo-) Invitrogen. CHO-K1 cells obtained from ATCC (cat #CCL-61) were transfected with the Kv1.1 or Kv1.5/ pcDNA3.1 (Neo-) construct and stable cells line were developed through two rounds of limiting dilution under G418 selection. Positive (Kv1.x expressing) clones were identified using electrophysiology.

### Propagation

CHO/Kv1.x stable cell lines were grown in a humidified atmosphere at 37 °C, 5% CO₂ in T-75 flasks containing F12 Kaighn's medium supplemented with 10% fetal calf serum, 2 mM L-glutamine, 100 units/ml penicillin, 100 µg/ml streptomycin and 500 µg/ml G418 (to maintain selection). CHO/Kv1.1 and CHO/Kv1.5 cells used for patching were transferred to 33 °C for 48 hours prior to use.

### 2. ELECTROPHYSIOLOGY

Kv1.x currents were measured using a planar electrode version (Nanion Technologies GMBH) of the patch-clamp technique. Whole-cell Kv1.x current transients were evoked by 500 ms depolarising voltage pulses to +40 mV from a holding potential of -80 mV applied at 10 s intervals for Kv1.1 and Kv1.5 and at 30 s intervals for Kv1.3 to allow adequate time for recovery from inactivation. Cells were continuously bathed in a buffered saline solution containing (mM): 160 NaCl, 4.5 KCI, 2 CaCl₂, 1 MgCl₂, 5 glucose, 10 HEPES, pH 7.4, 290-310 mOsm.Kg⁻¹. The internal (pipette) solution contained (mM): 10 NaCl, 70 KF, 75 KCI, 2 MgCl₂, 10 HEPES, 10 EGTA, pH 7.2, 290-310 mOsm.Kg⁻¹. Series resistance compensation (60-80%) was applied when the peak current amplitude exceeded 2 nA.

### Compound preparation and potency assessment

Compounds were initially dissolved in DMSO to 10 mM. After further dilution in DMSO, compounds were finally diluted in bath solution 1/200 (to give a final DMSO concentration of 0.5%) and applied directly to the recording chamber. Compounds were added at increasing concentrations allowing ample time for steady state block to be achieved between each concentration. Each compound was tested at 5-6 different concentrations on 2-3 cells. IC₅₀ values can be determined by fitting the average normalised reduction of either the current integral or the steady state current amplitude at the end of the depolarising pulse obtained at each compound concentration to the Hill equation. The IC₅₀ data reported herein is based on the steady state calculation.

### 3. EFFECTOR MEMORY T CELL PROLIFERATION ASSAY WITH CYTOKINE READOUT

### Inhibition of proliferation

Inhibition of T_{EM} function in vitro was based on methods published by (Hu et al., 2007, J. Immunol., 179, 4563-4570; Wulff et al., 2003, J. Clin. Invest., 111, 1703-1713; Beeton et al., 2005, Mol. Pharmacol., 67, 1369-1381). Peripheral blood mononuclear cells were purified from human whole blood preparations using Ficoll density centrifugation. T_{EM} cells were obtained by enrichment of the CD45RA-CCR7-population using monoclonal antibodies, labelled magnetic beads and magnetic separation (Miltenyi Biotec). Enriched T_{EM} cells were incubated at a concentration of 2x10⁵ cells per well in 96-well plates in RPMI medium supplemented with 5% human serum, glutamine (Gibco) and penicillin/streptomycin (Gibco). Once plated, cells were incubated with compound dilutions for 2 hours at 37°C before being stimulated. Compound dilutions were made up in T cell medium + DMSO (to keep the concentration of DMSO constant within the dilutions) and 75 µl/well were added. After two hours, 150 µl of well contents were transferred to another 96 well plate coated with anti-human CD3 antibody (2 µg/ml overnight and then extensively washed with PBS). 72 hours later tritiated thymidine was added and proliferation of Tem cells measured by scintillation counting of thymidine incorporation. All incubations took place in an incubator at 37°C and 5% CO₂.

### Inhibition of interferon gamma (IFNγ) secretion

Peripheral blood mononuclear cells were purified from human whole blood preparations using Ficoll density centrifugation. T_{EM} cells were obtained by enrichment of the CD45RA-CCR7- population using monoclonal antibodies, labelled magnetic beads and magnetic separation (Miltenyi Biotec). Enriched T_{EM} cells were incubated at a concentration of 2x10⁵ cells per well in 96-well plates in RPMI medium supplemented with 5% human serum, glutamine (Gibco) and penicillin/streptomycin (Gibco). Once plated, cells were incubated with compound dilutions for 2 hours at 37°C before being stimulated. Compound dilutions were made up in T cell medium + DMSO (to keep the concentration of DMSO constant within the dilutions) and 75 µl/well were added. After two hours, 150 µl of well contents were transferred to another 96 well plate coated with anti-human CD3 antibody (2 µg/ml overnight and then extensively washed with PBS). 72 hours later supernatant was removed and analysed for presence of human IFNγ using an ELISA kit (R&D Systems) and a Fluostar optical density reader (450 nm wavelength filter). All incubations took place in an incubator at 37°C and 5% CO₂.

### In vitro inhibition of proliferation and cytokine secretion by antigen-specific rat T cells

Lewis rats were immunised subcutaneously with 200 µl OVA protein (Sigma) emulsion in CFA (DIFCO). 7 days later rats were challenged with OVA solution intradermally into the middle of the right ear. 24 hours later the rats were killed and inguinal lymph nodes removed. Following homogenisation (gentleMACS Dissociator (MACS Miltenyi Biotec)) and passage through a filter cell suspensions were prepared in RPMI (supplemented with 10% FBS (heat-inactivated, Invitrogen) 1% Pen-Strep (Invitrogen), 1% Hepes 1M (Invitrogen), 1% Glutamax (Invitrogen), 1% MEM (SIGMA), 2.5µM B-mercaptoethanol (Invitrogen), 1µM sodium pyruvate (Invitrogen) and plated in 96 well plates at a concentration of 5x10⁵ cells per well. Cells were left unstimulated or stimulated with a titration of compound or with ConA (Sigma) as a positive control and incubated at 37°C, 5% CO₂ for 48 hours. After this time 10 µl tritiated thymidine (1µCi per well) was added to cell proliferation plates and incubated overnight for a further 16 hours at 37°C and 5% CO2. Plates were frozen

(-20°C) until transfer to filters (Filtermat A Perkin Elmer) and tritium count (Microbeta counter).

Duplicate cultures were set up with the same stimulation conditions, but after 72 hours incubation, supernatants were removed and stored at -80°C until cytokine analysis (IL17A & IFNg, custom Rat 2 Plex Cytokine Panel, IL17-A & IFNgamma, Kit LEGENDplex, or Kit Milliplex, MerckMillipore).

### Results:

| Ex. | Structure | IC₅₀ (Kv1.3 ephys steady state, nM) |
|---|---|---|
| 1 | 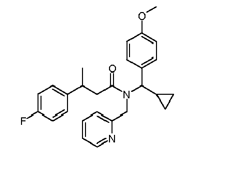 | <50 |
| 2 | 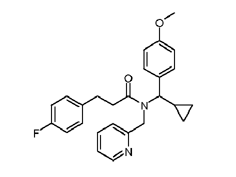 | 50-200 |
| 9 | 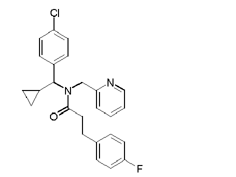 | 50-200 |
| 10 | 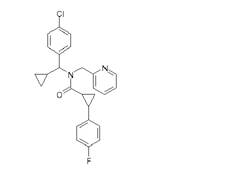 | <50 |
| 15 | 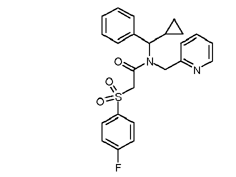 | 200-1000 |
| 20 | 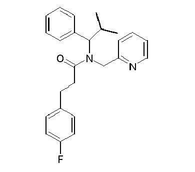 | 50-200 |
| 21 | 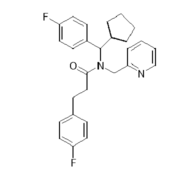 | 50-200 |
| 22 | 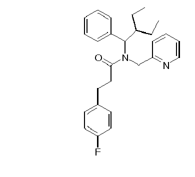 | 200-1000 |
| 23 | 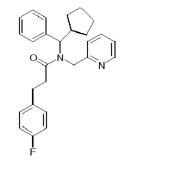 | 50-200 |
| 24 | 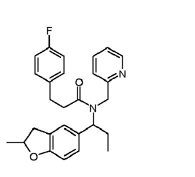 | 50-200 |
| 41 | 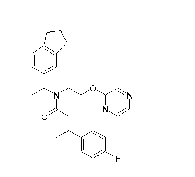 | 50-200 |
| 42 | 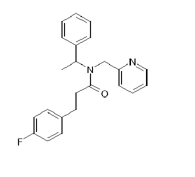 | 200-1000 |
| 43 | 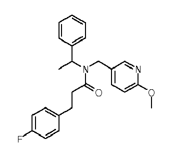 | 200-1000 |
| 44 | 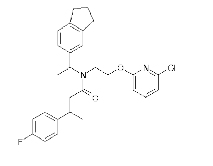 | 200-1000 |
| 45 | 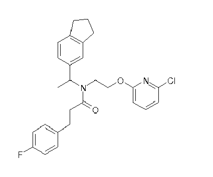 | 200-1000 |
| 46 | 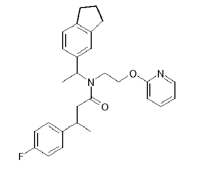 | 50-200 |
| 47 | 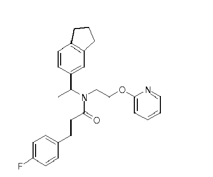 | 50-200 |
| 50 | 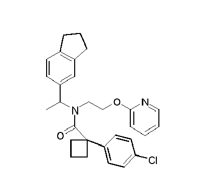 | 200-1000 |
| 51 | 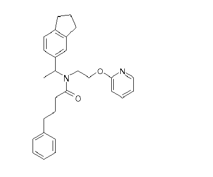 | 50-200 |
| 52 | 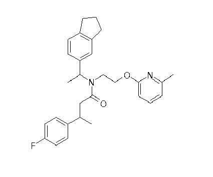 | 200-1000 |
| 53 | 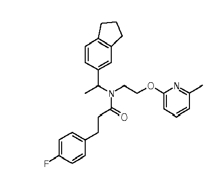 | 50-200 |
| 54 | 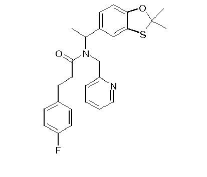 | 200-1000 |
| 57 | 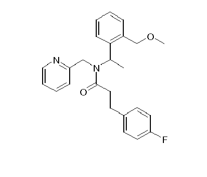 | 200-1000 |
| 62 | 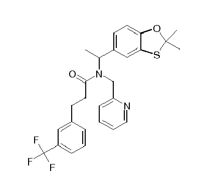 | 200-1000 |
| 63 | 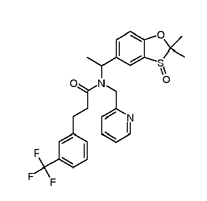 | >1000 |
| 65 | 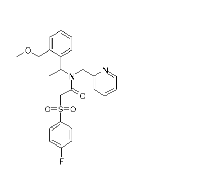 | >1000 |
| 66 | 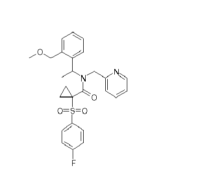 | 50-200 |
| 67 | 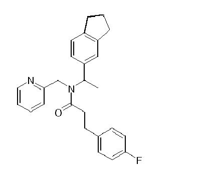 | 200-1000 |
| 68 | 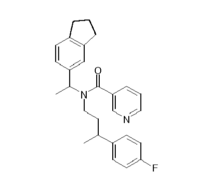 | 200-1000 |
| 69 | 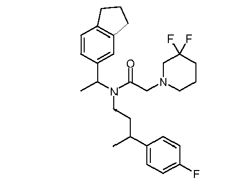 | 50-200 |
| 70 | 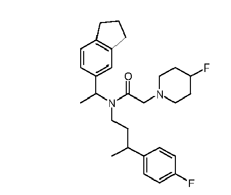 | 50-200 |
| 76 | 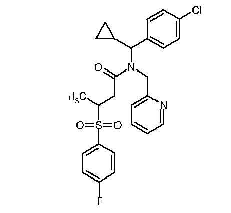 | 50-200 |
| 77 | 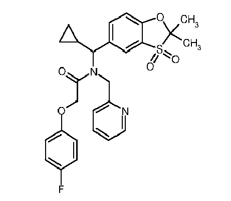 | 50-200 |
| 78 | 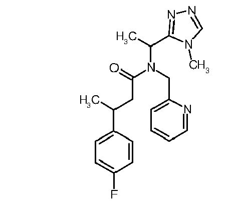 | 200-1000 |
| 87 | 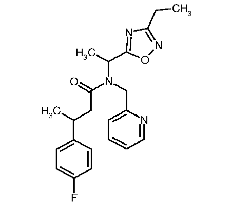 | 200-1000 |

### PHARMACEUTICAL COMPOSITIONS

When employed as pharmaceuticals, the compounds provided herein are typically administered in the form of a pharmaceutical composition. Such compositions can be prepared in a manner well known in the pharmaceutical art and comprise at least one active compound. Generally, the compounds provided herein are administered in a therapeutically effective amount. The amount of the compound actually administered will typically be determined by a physician, in the light of the relevant circumstances, including the condition to be treated, the chosen route of administration, the actual compound -administered, the age, weight, and response of the individual patient, the severity of the patient's symptoms, and the like. The pharmaceutical compositions provided herein can be administered by a variety of routes including oral, rectal, transdermal, subcutaneous, intravenous, intramuscular, and intranasal. Depending on the intended route of delivery, the compounds provided herein are preferably formulated as either injectable or oral compositions or as salves, as lotions or as patches all for transdermal administration.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the furansulfonic acid compound is usually a minor component (from about 0.1 to about 50% by weight or preferably from about 1 to about 40% by weight) with the remainder being various vehicles or carriers and processing aids helpful for forming the desired dosing form. Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable carriers known in the art. As before, the active compound in such compositions is typically a minor component, often being from about 0.05 to 10% by weight with the remainder being the injectable carrier and the like.

Transdermal compositions are typically formulated as a topical ointment or cream containing the active ingredient(s), generally in an amount ranging from about 0.01 to about 20% by weight, preferably from about 0.1 to about 20% by weight, preferably from about 0.1 to about 10% by weight, and more preferably from about 0.5 to about 15% by weight. When formulated as a ointment, the active ingredients will typically be combined with either a paraffmic or a water-miscible ointment base. Alternatively, the active ingredients may be formulated in a cream with, for example an oil- in-water cream base. Such transdermal formulations are well-known in the art and generally include additional ingredients to enhance the dermal penetration of stability of the active ingredients or the formulation. All such known transdermal formulations and ingredients are included within the scope provided herein. The compounds provided herein can also be administered by a transdermal device.

Accordingly, transdermal administration can be accomplished using a patch either of the reservoir or porous membrane type, or of a solid matrix variety. The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference. The above-described components for orally administrable, injectable or topically administrable compositions are merely representative. Other materials as well as processing techniques and the like are set forth in Part 8 of Remington's The Science and Practice of Pharmacy, 21 st edition, 2005, Publisher: Lippincott Williams & Wilkins, which is incorporated herein by reference. The compounds of this invention can also be administered in sustained release forms or from sustained release drug delivery systems. A description of representative sustained release materials can be found in Remington's Pharmaceutical Sciences.

The following formulation examples illustrate representative pharmaceutical compositions that may be prepared in accordance with this invention. The present invention, however, is not limited to the following pharmaceutical compositions.

### Formulation 1 - Tablets

A compound of the invention may be admixed as a dry powder with a dry gelatin binder in an approximate 1 :2 weight ratio. A minor amount of magnesium stearate may be added as a lubricant. The mixture is formed into 240-270 mg tablets (80-90 mg of active amide compound per tablet) in a tablet press.

### Formulation 2 - Capsules

A compound of the invention may be admixed as a dry powder with a starch diluent in an approximate 1 :1 weight ratio. The mixture may be filled into 250 mg capsules (125 mg of active amide compound per capsule).

### Formulation 3 - Liquid

A compound of the invention (125 mg), sucrose (1.75 g) and xanthan gum (4 mg) may be admixed with sucrose (1.75 g) and xanthan gum (4 mg) and the resultant mixture may be blended, passed through a No. 10 mesh U.S. sieve, and then mixed with a previously made solution of microcrystalline cellulose and sodium carboxymethyl cellulose (11 :89, 50 mg) in water. Sodium benzoate (10 mg), flavor, and color are diluted with water and added with stirring. Sufficient water is then added to produce a total volume of 5 mL.

### Formulation 4 - Tablets

A compound of the invention may be admixed as a dry powder with a dry gelatin binder in an approximate 1 :2 weight ratio. A minor amount of magnesium stearate is added as a lubricant. The mixture is formed into 450-900 mg tablets (150-300 mg of active amide compound) in a tablet press.

### Formulation 5 - Injection

A compound of the invention may be dissolved or suspended in a buffered sterile saline injectable aqueous medium to a concentration of approximately 5 mg/ml.

Formulation 6 - Topical Stearyl alcohol (250 g) and a white petrolatum (250 g) may be melted at about 75°C and then a mixture of a compound of the invention (50 g) methylparaben (0.25 g), propylparaben (0.15 g), sodium lauryl sulfate (10 g), and propylene glycol (120 g) would be dissolved in water (about 370 g) is added and the resulting mixture is stirred until it congeals.

## Claims

1. A compound of Formula (I) 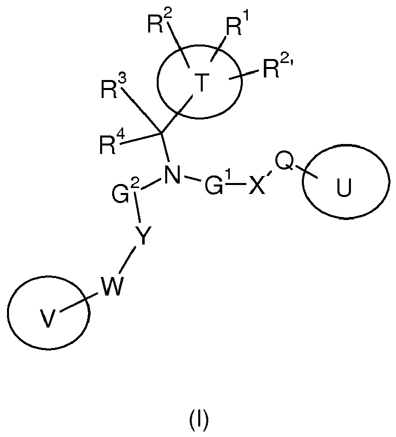 Wherein
G¹ denotes a CO group or a single bond, preferably a single bond,
G² denotes a CO group, a CH₂ group, or a single bond, preferably a CO group,
X, Y are independently from one another selected from a single bond, a linear or branched alkylene group having 1 to 6 carbon atoms, or a saturated or unsaturated 3-8-membered cycloalkylene group,
Q, W are independently from one another selected from O, NH, SO, SO₂, CO, or a single bond,
U is a 3-8-membered saturated or unsaturated cycloalkyl group, a 3-8-membered saturated or unsaturated heterocyclic group, a 7-12 bridged or fused bicyclic carbocyclic or heterocyclic ring or a 5-6-membered heteroaromatic group, each of the above group being optionally substituted with 1 to 3 substitutents selected from Hal, NO₂, CN, SO₂, NMe₂, linear or branched alkyl group having 1 to 6 carbon atoms, O-C₁-C₆-alkyl, -(CH₂)ₘ-O-C₁-C₆-alkyl, CF₃, or a 5-6-membered heteroaromatic group
V is an aryl group optionally substituted with 1 to 3 substitutents selected from Hal, NO₂, CN, SO₂-C₁₋C₆ alkyl, NMe₂, linear or branched alkyl having 1 to 6 carbon atoms, O-C₁-C₆-alkyl, -(CH₂)ₘ-O-C₁-C₆-alkyl, CF₃, or a 5-6-membered heteroaromatic group,
T denotes a phenyl, a triazole, a thiazole, an oxazole, an oxadiazole or an imidazol group,
R¹, R², R^{2'} are independently from one another H, Hal, O-C₁-C₆-alkyl, -(CH₂)ₘ-O-C₁-C₆-alkyl, O-C₁-C₆-halo-alkyl, -(CH₂)ₘ-O-C₁-C₆-halo-alkyl, -SO₂-C₁-C₆-alkyl, -(CH₂)ₘ-SO₂-C₁-C₆-alkyl, -SO₂-C₁-C₆-halo-alkyl, -(CH₂)ₘ-SO₂-C₁-C₆-halo-alkyl, -SO₂-3-8-cycloalkyl, -(CH₂)ₘ-SO₂-3-8-cycloalkyl, CF₃, or two of R¹,R² and R^{2'} are linked to form with the ring T to which they are attached a 7-12 fused bicyclic carbocyclic ring, optionally containing 1 to 3 groups independently selected from an oxygen atom, a sulphur atom, a SO₂ group, or a SO group, and optionally substituted with 1 to 3 Hal, CF₃, NO₂, CN, a linear or branched alkyl group having 1 to 6 carbon atoms, -(CH₂)ₘ-O-C₁-C₆-alkyl, -O-C₁-C₆-alkyl,
R³ is a linear or branched C₁-C₆-alkyl group, a 3-8-membered cycloalkyl group, - (CH₂)ₘ-O-C₁-C₆-alkyl, -O-C₁-C₆-alkyl, or a 3-8-membered heterocyclic group, each of the above group being optionally substituted with 1 to 3 substitutents independently selected from Hal, CF₃, NO₂, CN, a linear or branched alkyl group having 1 to 6 carbon atoms, -(CH₂)ₘ-O-C₁-C₆-alkyl, -(CH₂)ₘ-SO₂-C₁-C₆-alkyl, -SO₂-C₁-C₆-alkyl, -O-C₁-C₆-alkyl,-(CH₂)ₘ-O-C₁-C₆-halo-alkyl, -(CH₂)ₘ-SO₂-C₁-C₆-halo-alkyl, -SO₂-C₁-C₆-halo-alkyl, or -O-C₁-C₆-halo-alkyl,
R⁴ denotes H, a linear or branched C₁-C₆-alkyl group, or form together with R³ a 3-8-membered cycloalkyl, optionally substituted with Hal, CF₃, NO₂, CN, a linear or branched alkyl group having 1 to 6 carbon atoms, -(CH₂)ₘ-O-C₁-C₆-alkyl, -O-C₁-C₆-alkyl,
m is selected from 1, 2, 3 or 4, preferably 1 or 2,
Hal is F, Cl, Br, I,
As well as pharmaceutically acceptable derivatives, solvates, tautomers, salts, hydrates and stereoisomers thereof, including mixtures thereof in all ratios.

2. A compound of Formula (I) according to claim 1 wherein R⁴ denotes H and R³
is selected from the following groups:
| | | | |
|---|---|---|---|
| -CH₃ | -CH₂OCH₃ | -CH(CH₃)₂ | -CH₂CH₃ |
| 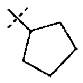 | 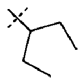 | 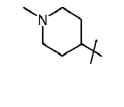 | 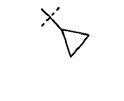 |
| -CH₂OCH₂CF₂H | -CH₂OCH₂CF₃ | 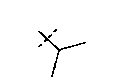 | 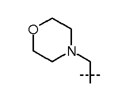 |
Wherein the above-mentioned groups may be further substituted by 1 to 3 substitutents independently selected from Hal, CF₃, NO₂, CN, a linear or branched alkyl group having 1 to 6 carbon atoms, -(CH₂)ₘ-O-C₁-C₆-alkyl, -(CH₂)ₘ-SO₂-C₁-C₆-alkyl, -SO₂-C₁-C₆-alkyl, -O-C₁-C₆-alkyl,-(CH₂)ₘ-O-C₁-C₆-halo-alkyl, - (CH₂)ₘ-SO₂-C₁-C₆-halo-alkyl, -SO₂-C₁-C₆-halo-alkyl, or -O-C₁-C₆-halo-alkyl

3. A compound according to claim 1 or 2 wherein the group G¹-X-Q-U is selected from the following groups:

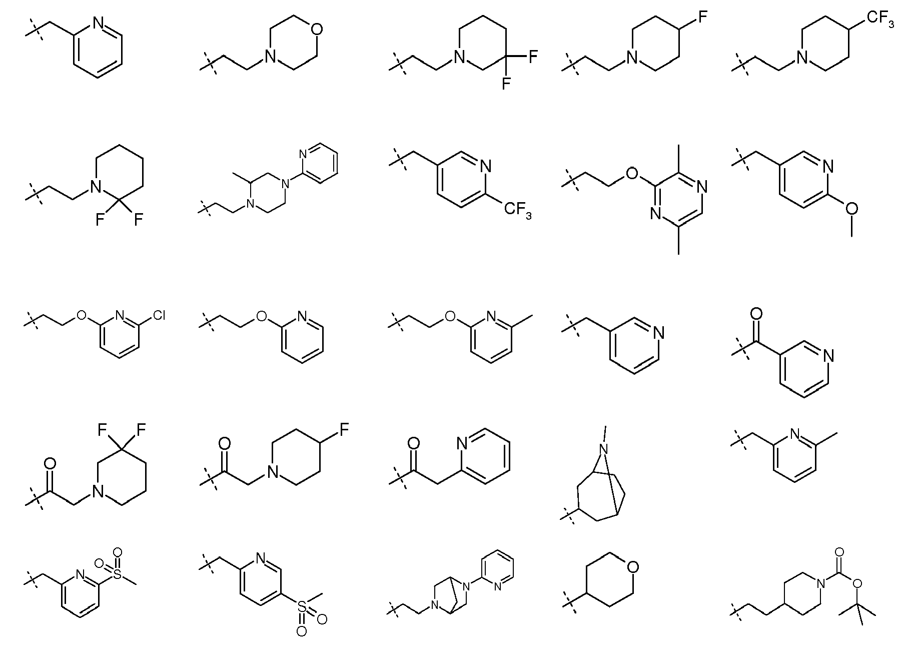

4. A compound according to claims 1 to 3 wherein the sequence G²-Y-W-V is
selected from the following groups: 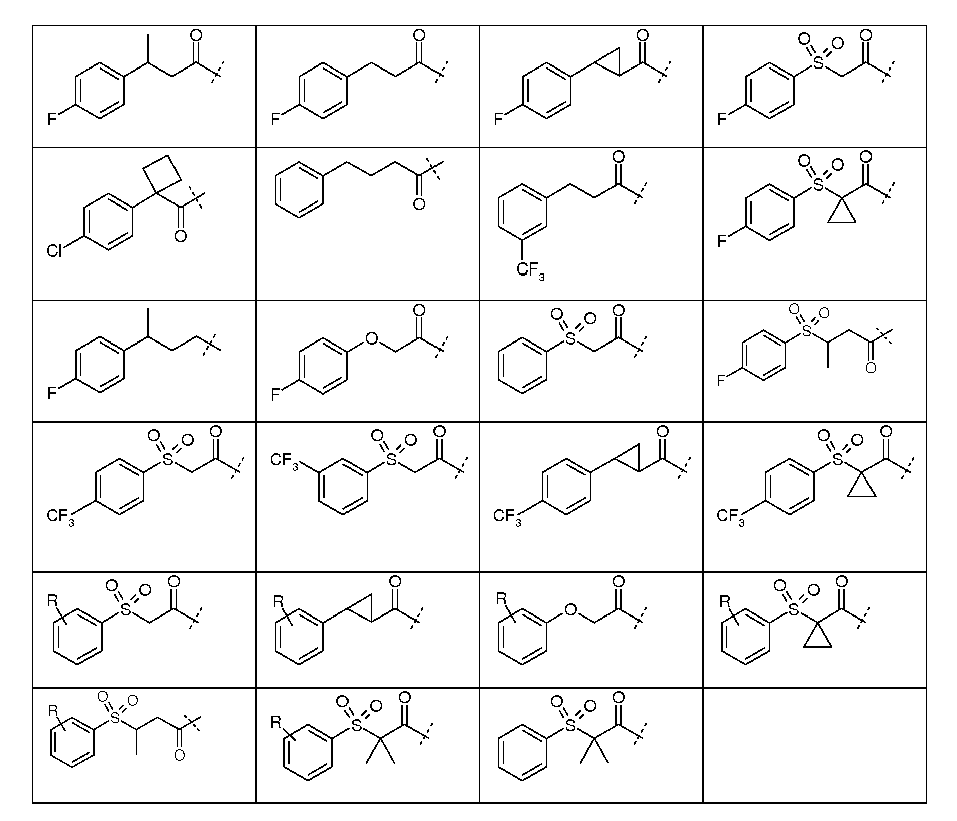 Wherein R denotes Hal, NO₂, CN, SO₂, NMe₂, linear or branched alkyl having 1 to 6 carbon atoms, O-C₁-C₆-alkyl, -(CH₂)ₘ-O-C₁-C₆-alkyl, CF₃, or a 5-6-membered heteroaromatic group.

5. A compound according to claims 1 to 4 wherein the group 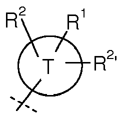 is selected from the following groups:

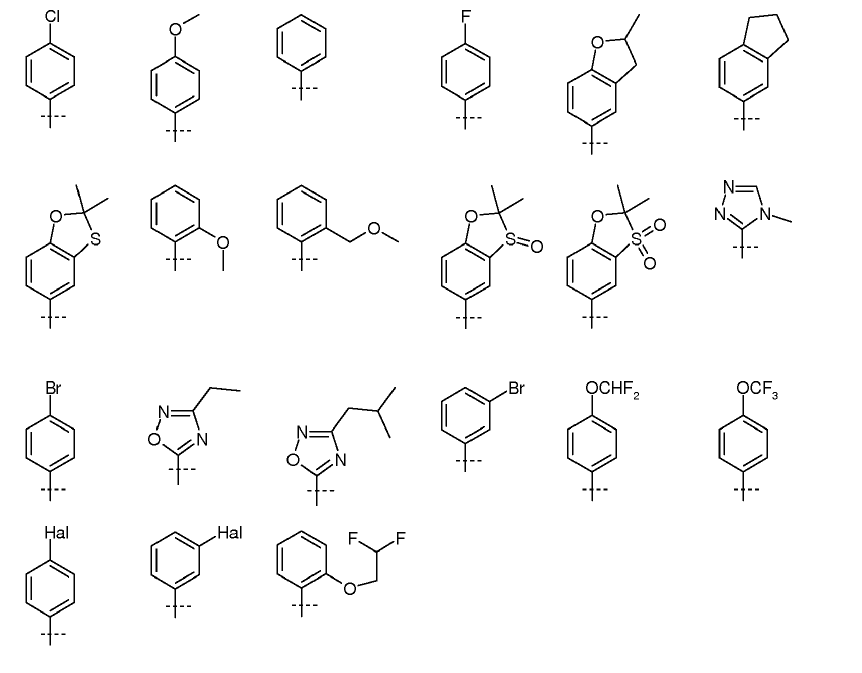

6. A compound according to claims 1 to 5 wherein the compound is selected from the following group:
| Ex | Structure | Ex | Structure |
|---|---|---|---|
| 1 | 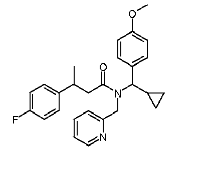 | 2 | 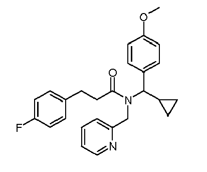 |
| 3 | 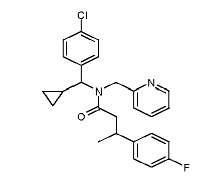 | 9 | 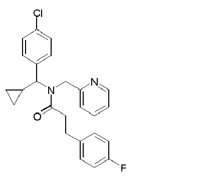 |
| 10 | 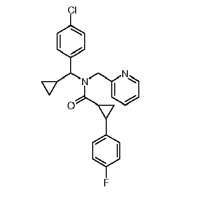 | 15 | 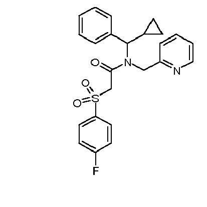 |
| 18 | 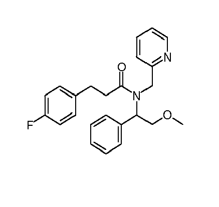 | 20 | 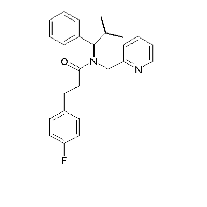 |
| 21 | 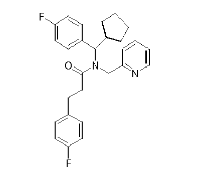 | 22 | 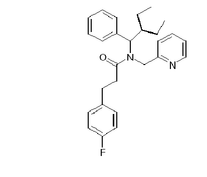 |
| 23 | 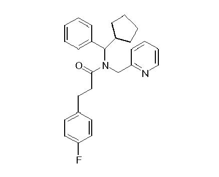 | 24 | 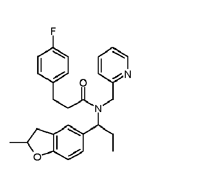 |
| 25 | 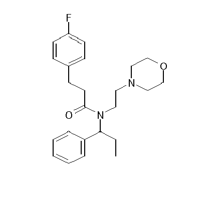 | 27 | 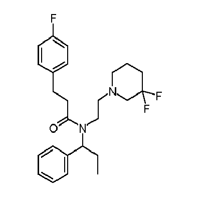 |
| 29 | 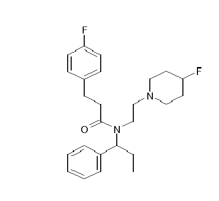 | 31 | 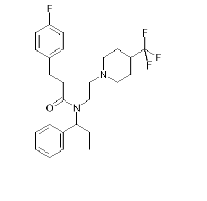 |
| 33 | 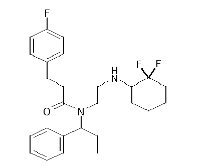 | 35 | 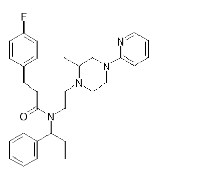 |
| 37 | 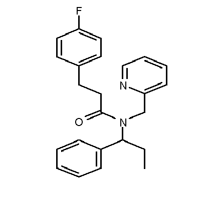 | 39 | 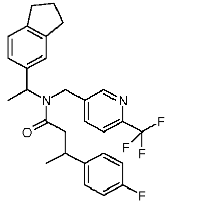 |
| 41 | 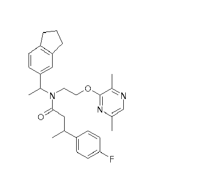 | 42 | 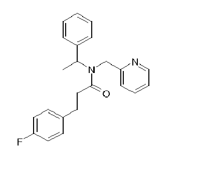 |
| 43 | 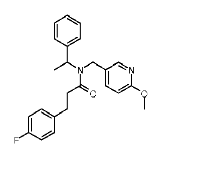 | 44 | 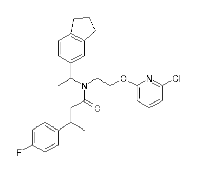 |
| 45 | 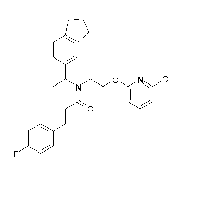 | 46 | 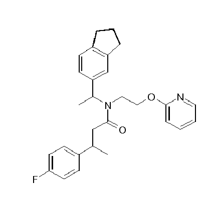 |
| 47 | 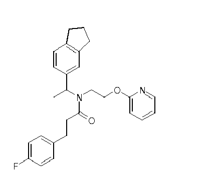 | 48 | 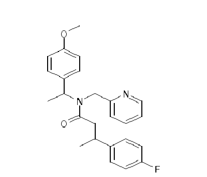 |
| 50 | 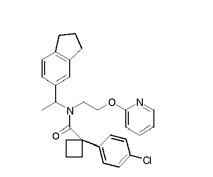 | 51 | 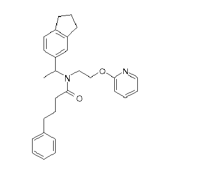 |
| 52 | 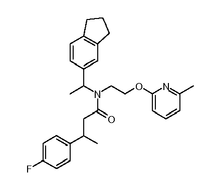 | 53 | 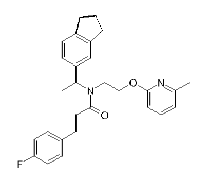 |
| 54 | 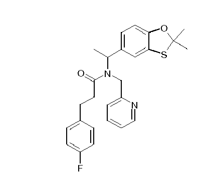 | 55 | 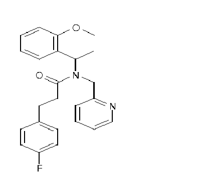 |
| 57 | 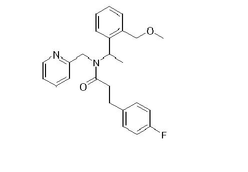 | 60 | 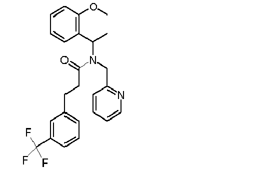 |
| 62 | 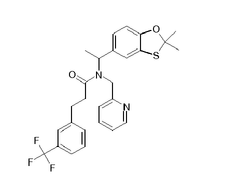 | 63 | 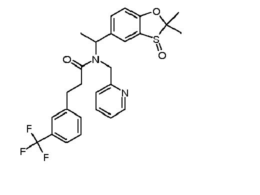 |
| 64 | 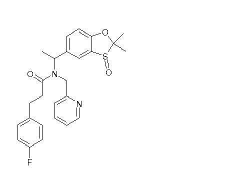 | 65 | 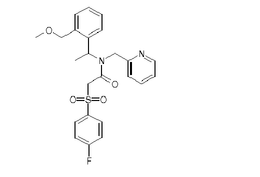 |
| 66 | 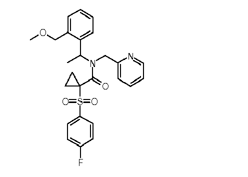 | 67 | 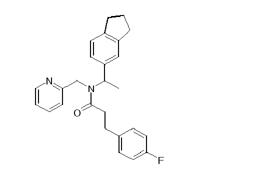 |
| 68 | 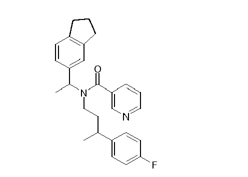 | 69 | 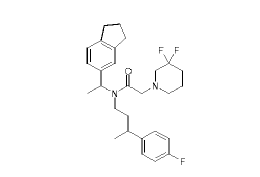 |
| 70 | 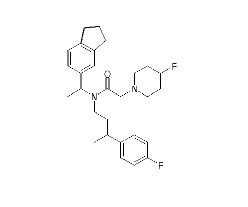 | 71 | 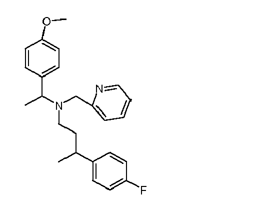 |
| 73 | 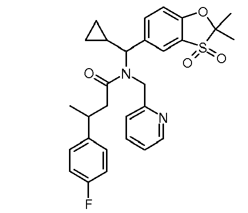 | 75 | 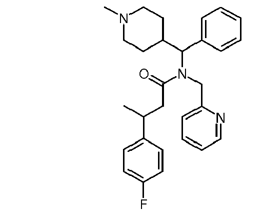 |
| 76 | 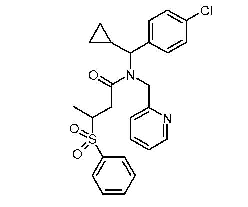 | 77 | 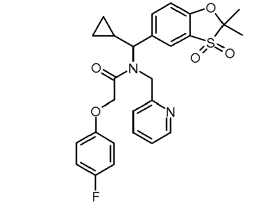 |
| 78 | 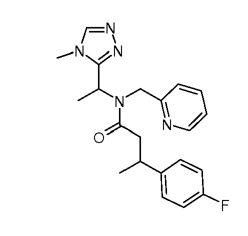 | 79 | 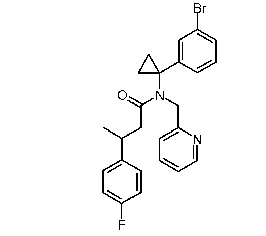 |
| 80 | 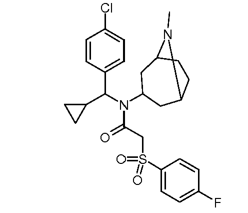 | 81 | 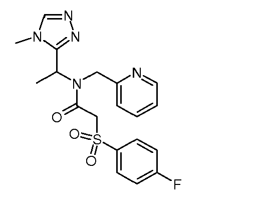 |
| 82 | 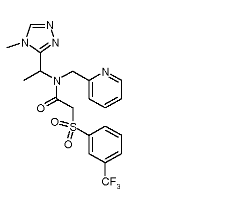 | 83 | 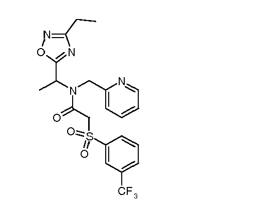 |
| 84 | 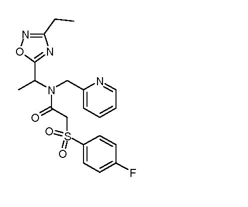 | 85 | 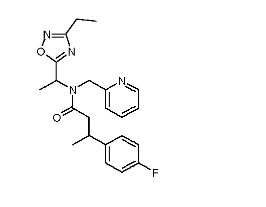 |
| 86 | 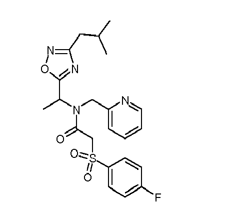 | 87 | 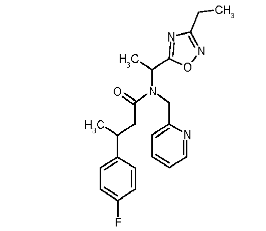 |
| 88 | 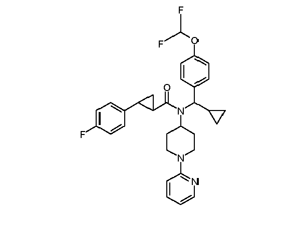 | 89 | 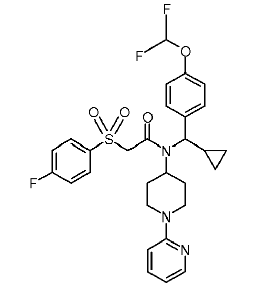 |
| 90 | 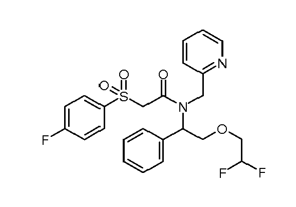 | 91 | 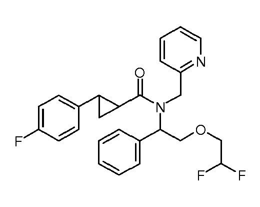 |
| 92 | 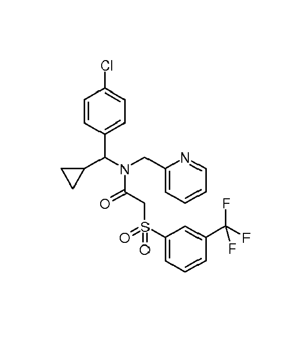 | 93 | 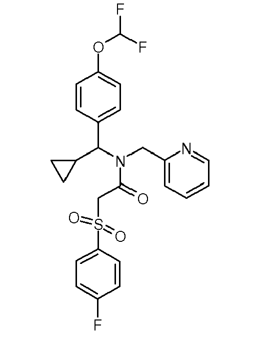 |
| 94 | 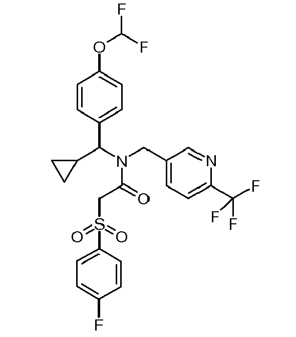 | 95 | 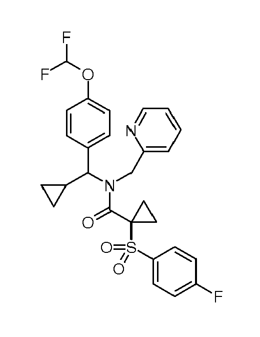 |
| 96 | 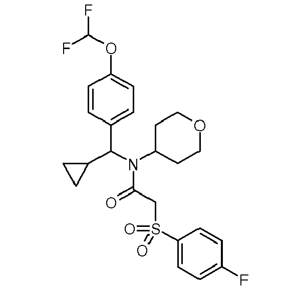 | 97 | 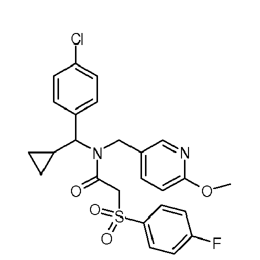 |
| 98 | 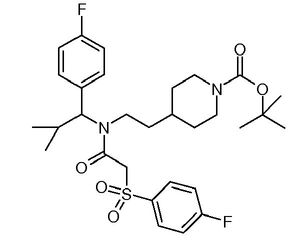 | 99 | 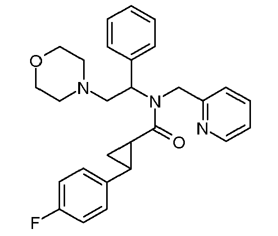 |
| 100 | 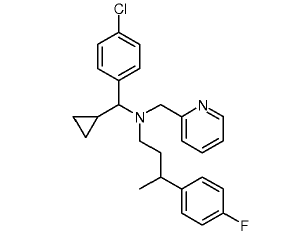 | 101 | 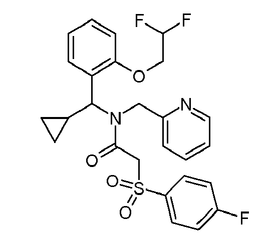 |

7. A compound of Formula (I) according to claim 1 for use as a medicament.

8. A compound of Formula (I) according to claims 1 to 6 for use in the treatment or prophylaxis of a condition selected from demyelinated diseases, Multiple sclerosis, Rheumatoid arthritis, Psoriasis, Type 1 Diabetes, Systemic lupus nephritis, cancer, Glomerulonephritis, Sjögrens's syndrome, Transplant rejection, Graft versus host disease, Allergic contact dermatitis, Neointimal hyperplasia/restenosis, Periodontal disease, Leprosy, and Obesity.

9. A pharmaceutical composition comprising at least one compound of Formula (I) according to claims 1 to 6, and a pharmaceutical carrier, excipient or diluent.

10. A kit or a set comprising at least one compound of Formula (I), consisting of separate packs of:
(a) an effective amount of a compound of the formula (I) and/or pharmaceutically usable derivatives, solvates, salts, hydrates and stereoisomers thereof, including mixtures thereof in all ratios, and
(b) an effective amount of a further medicament active ingredient.

11. A process of making compounds of Formula (I) as defined in claim 1 comprising the steps of reacting a compound of Formula 5, wherein R¹, R², R^{2'}, R³, R⁴, G¹, X, Q, T and U are as defined in claim 1, with a compound of Formula 8, wherein G², Y, W, and V are as defined in claim 1 and wherein LG is a suitable leaving group, 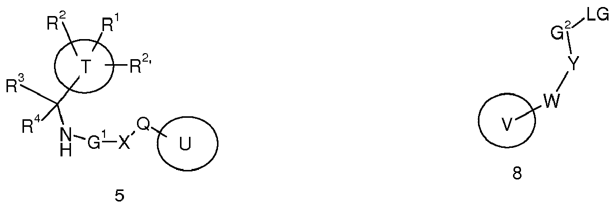 Or reacting a compound of Formula 7, wherein R¹, R², R^{2'}, R³, R⁴, G², Y, W, T and V are as defined in claim 1, with a compound of Formula 9, wherein G¹, X, Q and U are as defined in claim 1 and wherein LG denotes a suitable leaving group.
